(19) Europäisches Patentamt
European Patent Office
Office européen des brevets

(11) Publication number : **0 524 004 A1**

# (12) EUROPEAN PATENT APPLICATION

(21) Application number : **92306553.6**

(22) Date of filing : **16.07.92**

(51) Int. Cl.$^5$ : **C07D 471/14,** C07D 491/22, A61K 31/47, // (C07D471/14, 221:00, 221:00, 209:00), (C07D471/14, 221:00, 221:00, 221:00), (C07D491/22, 317:00, 221:00, 221:00, 209:00)

(30) Priority : **17.07.91 US 731691**

(43) Date of publication of application : **20.01.93 Bulletin 93/03**

(84) Designated Contracting States : **AT BE CH DE DK ES FR GB GR IT LI LU NL PT SE**

(71) Applicant : **SYNTEX (U.S.A.) INC.**
**3401 Hillview Avenue**
**Palo Alto California 94304 (US)**

(72) Inventor : **Clark, Robin D.**
**2025 Columbia Street**
**Palo Alto, California 94306 (US)**
Inventor : **Spedding, Michael**
**28 Foulis Crescent, Juniper Green**
**Edinburgh, EH14 5BN (GB)**
Inventor : **Redfern, William Salisbury**
**5 East Claremont Street**
**Edinburgh, EH7 4HT (GB)**

(74) Representative : **Nicholls, Kathryn Margaret et al**
**Mewburn Ellis, 2 Cursitor Street**
**London EC4A 1BQ (GB)**

## (54) Decahydro-8H-isoquino[2,1-g][1,6]naphthyridine and decahydrobenzo[a]pyrrolo[2,3-e]quinolizine derivatives.

(57) Compounds of the formula

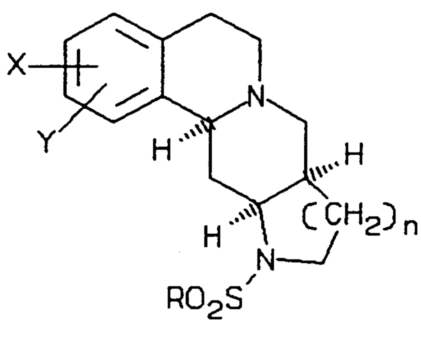

(1)

wherein :

X and Y are independently hydrogen ; hydroxy ; lower alkyl of 1-6 carbon atoms ; lower alkoxy of 1-6 carbon atoms ; or halo ; or X and Y when adjacent and taken together are methylenedioxy or ethylene-1,2-dioxy ;

R is lower alkyl of 1-6 carbon atoms ; cycloalkyl of 3-8 carbon atoms ; phenyl or phenyl lower alkyl in which any phenyl group may be optionally substituted by one or two substituents chosen from the group consisting of halo, lower alkyl of 1-4 carbon atoms and lower alkoxy of 1-4 carbon atoms ; or $-ANHSO_2R^1$ ; wherein A is lower alkylene of 1-6 carbon atoms ; and $R^1$ is lower alkyl of 1-6 carbon atoms or $-NR^2R^3$ ; wherein

$R^2$ and $R^3$ are independently hydrogen or lower alkyl of 1-6 carbon atoms, or $R^2$ and $R^3$ taken together are cycloalkyl of 3-8 carbon atoms ; and

EP 0 524 004 A1

n is 1 or 2 ;

and the pharmaceutically acceptable salts thereof, are useful as $\alpha_2$-adrenoceptor antagonists, in particular as peripherally selective $\alpha_2$ -adrenoceptor antagonists.

The invention relates to various decahydro-8H-isoquino [2,1-g][1,6]naphthyridine and decahydrobenzo [a]pyrrolo[2,3-e]quinolizine derivatives, as single enantiomers or racemic or non-racemic mixtures thereof, which exhibit selective $\alpha_2$-adrenoceptor blockade in mammals, and which, therefore, are useful as medicaments for the treatment of physiological conditions affected by such blockade. Such treatment includes, for example, lowering of blood pressure in hypertensive mammals, treatment of irritable bowel syndrome, inhibition of platelet aggregation, treatment of hypoglycemia, alleviation of male impotence, palliation of diabetes, and lowering of intraocular pressure.

In addition, the compounds of formula (1) in normotensive mammals unexpectedly induce selective vasodilation in certain peripheral blood vessels without lowering blood pressure. As a consequence the compounds of formula (1) are particularly useful for the treatment of peripheral vascular disease, including diabetes and its sequelae such as diabetic retinopathy, nephropathy, neuropathy and associated circulatory disturbances, healing of skin lesions and necrotic ulcers, and treatment of intermittent claudication and Raynaud's disease.

The novel compounds of this invention are various decahydro-8H-isoquino[2,1-g][1,6]naphthyridine and decahydrobenzo[a]pyrrolo[2,3-e]quinolizine derivatives, as single enantiomers or racemic or non-racemic mixtures thereof, useful as $\alpha_2$-adrenoceptor antagonists and for the treatment of peripheral vascular disease. Compounds somewhat structurally related are described in Patent Nos. U.S. 4,454,139, U.S. 4,550,114, U.S. 4,791,108, U.S. 4,886,798, and in Nouveau J. Chim. 4(3), 199-202 (1980).

One aspect of the invention concerns novel compounds represented by the formula:

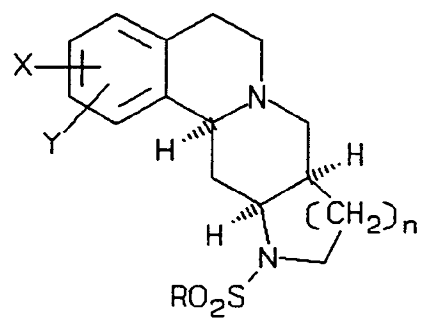

(1)

wherein:

X and Y are independently hydrogen; hydroxy; lower alkyl of 1-6 carbon atoms; lower alkoxy of 1-6 carbon atoms; or halo; or X and Y when adjacent and taken together are methylenedioxy or ethylene-1,2-dioxy;

R is lower alkyl of 1-6 carbon atoms; cycloalkyl of 3-8 carbon atoms; phenyl or phenyl lower alkyl in which any phenyl group may be optionally substituted by one or two substituents chosen from the group consisting of halo, lower alkyl of 1-4 carbon atoms and lower alkoxy of 1-4 carbon atoms; or $-ANHSO_2R^1$; wherein A is lower alkylene of 1-6 carbon atoms; and $R^1$ is lower alkyl of 1-6 carbon atoms or $-NR^2R^3$; wherein

$R^2$ and $R^3$ are independently hydrogen or lower alkyl of 1-6 carbon atoms, or $R^2$ and $R^3$ taken together are cycloalkyl of 3-8 carbon atoms; and

n is 1 or 2;

and the pharmaceutically acceptable salts thereof, are useful as selective $\alpha_2$-adrenoceptor antagonists, and in addition are particular useful for the treatment of peripheral vascular disease.

Other aspects of the invention relate to the novel intermediates of formula (XI), to pharmaceutical compositions containing compounds of formula (1) in admixture with one or more pharmaceutically acceptable, non-toxic carriers, and to methods pertaining to the use of compounds of formula (1).

## Definitions

As used herein:

"Alkyl" means a branched or unbranched saturated monovalent hydrocarbon radical containing 1 to 8 carbon atoms, such as methyl, ethyl, propyl, tert-butyl, n-hexyl, n-octyl and the like.

"Lower alkyl" means a branched or unbranched saturated monovalent hydrocarbon radical containing 1 to 6 carbon atoms, such as methyl, ethyl, propyl, isopropyl, tert-butyl, butyl, n-hexyl and the like, unless otherwise indicated.

"Lower alkoxy" means the group -O-(lower alkyl) wherein lower alkyl is as herein defined.

"Cycloalkyl" as used herein means a saturated monovalent monocyclic hydrocarbon radical containing 3-8 carbon atoms, such as cyclopropyl, cyclobutyl, cyclopentyl, cyclohexyl, cycloheptyl and cyclooctyl.

"Lower alkylene" as used herein means a branched or unbranched saturated divalent hydrocarbon radical containing 1 to 6 carbon atoms, such as methylene, ethylene, propylene, 2-methylpropylene, 1,2-dimethylpropylene, hexylene, and the like.

As used herein, the terms "inert organic solvent" or "inert solvent" mean a solvent inert under the conditions of the reaction being described in conjunction therewith [including, for example, benzene, toluene, acetonitrile, tetrahydrofuran ("THF"), dimethylformamide ("DMF"), chloroform ("$CHCl_3$"), methylene chloride (or dichloromethane or "$CH_2Cl_2$"), diethyl ether, ethyl acetate, acetone, methylethyl ketone, methanol, ethanol, propanol, isopropanol, tert-butanol, dioxane, pyridine, and the like]. Unless specified to the contrary, the solvents used in the reactions of the present invention are inert solvents.

"Halo" as used herein denotes fluoro, chloro, bromo, or iodo.

"Phenyl" as used herein encompasses all possible isomeric phenyl radicals optionally monosubstituted or disubstituted with a substituent selected from the group consisting of lower alkyl, lower alkoxy, and halo.

"Phenyl lower alkyl" as used herein denotes phenyl as defined above attached to a lower alkyl group as defined above.

"Pharmaceutically acceptable acid addition salt" refers to those salts which retain the biological effectiveness and properties of the free bases and which are not biologically or otherwise undesirable, formed with inorganic acids such as hydrochloric acid, hydrobromic acid, sulfuric acid, nitric acid, phosphoric acid and the like, and organic acids such as acetic acid, propionic acid, glycolic acid, pyruvic acid, oxalic acid, malic acid, malonic acid, succinic acid, maleic acid, fumaric acid, tartaric acid, citric acid, benzoic acid, cinnamic acid, mandelic acid, menthanesulfonic acid, ethanesulfonic acid, p-toluenesulfonic acid, salicylic acid and the like.

"Optional" or "optionally" means that the subsequently described event or circumstance may or may not occur, and that the description includes instances where said event or circumstance occurs and instances in which it does not. For example, "optionally substituted phenyl" means that the phenyl may or may not be substituted and that the description includes both unsubstituted phenyl and substituted phenyl;
"optionally followed by converting the free base to the acid addition salt" means that said conversion may or may not be carried out in order for the process described to fall within the invention, and the invention includes those processes wherein the free base is converted to the acid addition salt and those processes in which it is not.

The terms "α and β" indicate the specific stereochemical configuration of a substituent at an asymmetric carbon atom in a chemical structure as drawn. Thus "α", denoted by a broken line, indicates that the group at the position in question is below the general plane of the molecule as drawn, and "β", denoted by a bold line, indicates that the group at the position in question is above the general plane of the molecule as drawn.

It should be understood that formula (1) as drawn is intended to represent the racemic form of compounds of formula (1) as well as the individual enantiomers and non-racemic mixtures thereof, although for the sake of clarity only one enantiomer is shown. For the purpose of illustration the two enantiomers of compounds of formula (1) are represented below as (1A) and (1B):

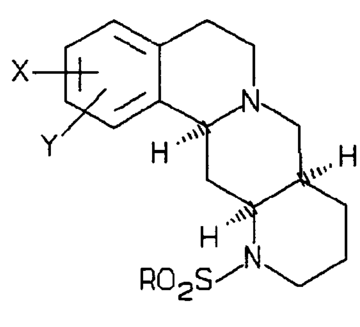

(1A)

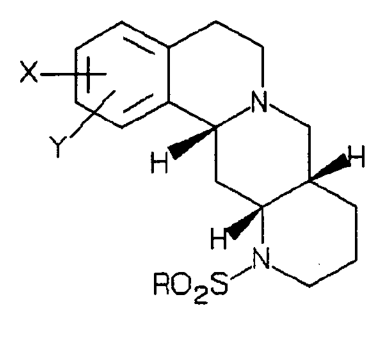

(1B)

The scope of the invention as described and claimed encompasses the racemic forms of the compounds of formula (1) as well as the individual enantiomers of formula (1A) and (1B), and non-racemic mixtures thereof.

The symbol "(±)" is used to designate a racemic mixture of individual (+) and (-) isomers. When the compound of formula (1) is a pure enantiomer, the stereochemistry at each chiral carbon atom is specified by either R or S according to the Cahn-Ingold-Prelog R-S system. In this manner relative stereochemistry is conveyed

unambiguously.

"Isomers" are different compounds that have the same molecular formula.

"Stereoisomers" are isomers that differ only in the way the atoms are arranged in space.

"Enantiomers" are a pair of stereoisomers that are non-superimposable mirror images of each other. A 1:1 mixture of a pair of enantiomers is a "racemic" mixture.

"Diastereoisomers" are stereoisomers which are not mirror-images of each other.

"Epimers" are diastereoisomers which differ only in the configuration of one asymmetric center.

The term "treatment" as used herein covers any treatment of a disease in a mammal, particularly a human, and includes:

(i) preventing the disease from occurring in a subject which may be predisposed to the disease but has not yet been diagnosed as having it;

(ii) inhibiting the disease, i.e. arresting its development; or

(iii) relieving the disease, i.e. causing regression of the disease.

The term "disease state which is alleviated by treatment with an $\alpha_2$-adrenoceptor antagonist" as used herein is intended to cover all disease states which are generally acknowledged in the art to be usefully treated with $\alpha_2$-adrenoceptor antagonists in general, and those disease states which have been found to be usefully treated by the specific $\alpha_2$-adrenoceptor antagonists of our invention, the compounds of formula (1). Such disease states include, but are not limited to, elevated blood pressure in hypertensive mammals, irritable bowel syndrome, platelet aggregation, hyperglycemia, male impotence, elevated intraocular pressure, peripheral vascular disease including diabetes and its sequelae such as diabetic retinopathy, nephropathy, neuropathy and associated circulatory disturbances, healing of skin lesions and necrotic ulcers, and treatment of intermittent claudication and Raynaud's disease.

The term "therapeutically effective amount" refers to that amount which is sufficient to effect treatment, as defined above, when administered to a mammal in need of such treatment. The therapeutically effective amount will vary depending on the subject and disease state being treated, the severity of the affliction and the manner of administration, and may be determined routinely by one of ordinary skill in the art.

The compounds of the invention include those compounds where n is 1 and those where n is 2. The compounds of formula (1) where n is 2 are illustrated below in order to demonstrate the numbering system used for naming such compounds:

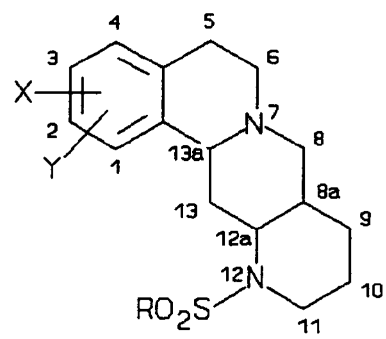

Following are examples of how representative compounds of formula (1) where n is 2 are named:

A racemic compound of formula (1) wherein n is 2, X is 3-methoxy, Y is hydrogen and R is $-ANHSO_2R^1$, in which A is propylene and $R^1$ is methyl is named:

(±)-3-methoxy-12-[3-(methanesulfonylamino)propanesulfonyl]-5,6,8aα,9,10,11,12,12aα,13,13aα-decahydro-8H-isoquino [2,1-g][1,6]naphthyridine.

The (+)-isomer of a compound of formula (1) wherein n is 2, X and Y taken together are 2,3-methylenedioxy and R is $-ANHSO_2R^1$, in which A is propylene and $R^1$ is $-NR^2R^3$, where $R^2$ and $R^3$ are both methyl, is named:

(8aR,12aS,13aS)-2,3-methylenedioxy-12-[3-(N,N-dimethylaminosulfonylamino)propanesulfonyl]-5,6,8a,9,10,11,12,12a,13,13a-decahydro-8H-isoquino[2,1-g][1,6]naphthyridine.

The compounds of formula (1) where n is 1 are illustrated below in order to demonstrate the numbering system used in naming such compounds:

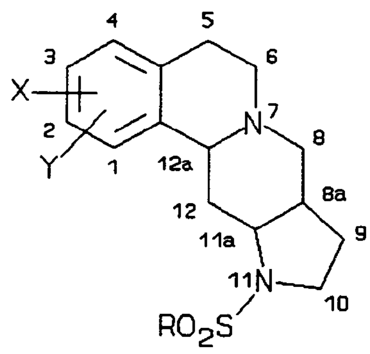

Following are examples of how representative compounds of formula (1) where n is 1 are named:

A racemic compound of formula (1) wherein n is 1, X is 3-methoxy, Y is hydrogen and R is -ANHSO$_2$R$^1$, in which A is propylene and R$^1$ is methyl is named:

(±)-(8aα,11aα,12aα)-3-methoxy-11-[3-(methanesulfonylamino)propanesulfonyl]-5,6,8,8a,9,10,11,11a,12,12a-decahydrobenzo[a]pyrrolo[2,3-e]quinolizine.

The (+)-isomer of a compound of formula (1) wherein n is 1, X and Y taken together are 2,3-methylenedioxy and R is -ANHSO$_2$R$^1$, in which A is propylene and R$^1$ is is -NR$^2$R$^3$, where R$^2$ and R$^3$ are both methyl, is named:

(8aR,11aS,12aS)-2,3-methylenedioxy-11-[3-(N,N-dimethylaminosulfonylamino)propanesulfonyl]-5,6,8,8a,9,10,11,11a,12,12a-decahydrobenzo[a]pyrrolo[2,3-e]quinolizine.

Among the family of compounds of the present invention, a preferred group includes the compounds in which n is 1. Within this group a preferred subgroup includes the compounds wherein R is lower alkyl. One preferred class within this subgroup includes compounds in which X and Y are independently hydrogen or lower alkoxy, or X and Y taken together is methylenedioxy. A second preferred subgroup within this group includes compounds in which R is -ANHSO$_2$R$^1$. One preferred class within this subgroup includes compounds in which A is alkylene of 2-4 carbon atoms and R$^1$ is lower alkyl, in particular where A is propylene, especially where X and Y are independently hydrogen or lower alkoxy, or X and Y taken together is methylenedioxy. A second preferred class within this subgroup includes compounds in which A is alkylene of 2-4 carbon atoms and R$^1$ is NR$^2$R$^3$, in particular where A is propylene and R$^2$ and R$^3$ are both methyl, especially where X and Y are independently hydrogen or lower alkoxy, or X and Y taken together is methylenedioxy.

A second preferred group includes the compounds in which n is 2 and R is -ANHSO$_2$R$^1$. One preferred class within this group includes compounds in which A is alkylene of 2-4 carbon atoms and R$^1$ is lower alkyl, in particular where A is propylene, especially where X and Y are independently hydrogen or lower alkoxy, or X and Y taken together is methylenedioxy. A second preferred class within this group includes compounds in which A is alkylene of 2-4 carbon atoms and R$^1$ is NR$^2$R$^3$, in particular where A is propylene and R$^2$ and R$^3$ are both methyl, especially where X and Y are independently hydrogen or lower alkoxy, or X and Y taken together is methylenedioxy.

At present, the preferred compounds are:

(±)-(8aα,11aα,12aα)-3-methoxy-11-methanesulfonyl-5,6,8,8a,9,10,11,11a,12,12a-decahydrobenzo[a]pyrrolo[2,3-e]quinolizine;

(±)-(8aα,11aα,12aα)-2,3-methylenedioxy-11-methanesulfonyl-5,6,8,8a,9,10,11,11a,12,12a-decahydrobenzo[a]pyrrolo[2,3-e]quinolizine;

(±)-(8aα,11aα,12aα)-3-methoxy-11-[3-(methanesulfonylamino)propanesulfonyl]-5,6,8,8a,9,10,11,11a,12,12a-decahydrobenzo[a]pyrrolo[2,3-e]quinolizine;

(±)-(8aα,11aα,12aα)-3-methoxy-11-[3-(aminosulfonylamino)propanesulfonyl]-5,6,8,8a,9,10,11,11a,12,12a-decahydrobenzo[a]pyrrolo[2,3-e]quinolizine;

(±)-(8aα,11aα,12aα)-3-methoxy-11-[3-(N,N-dimethylaminosulfonylamino)propanesulfonyl]-5,6,8,8a,9,10,11,11a,12,12a-decahydrobenzo[a]pyrrolo[2,3-e]quinolizine;

(8aR,12aS,13aS)-3-methoxy-12-[3-(methanesulfonylamino)propanesulfonyl]-5,6,8a,9,10,11,12,12a,13,13a-decahydro-8H-isoquino[2,1-g][1,6]naphthyridine;

(8aR,12aS,13aS)-3-methoxy-12-[3-(N,N-dimethylaminosulfonylamino)propanesulfonyl]-5,6,8a,9,10,11,12,12a,13,13a-decahydro-8H-isoquino[2,1-g][1,6]naphthyridine; and

(8aR,12aS,13aS)-3-methoxy-12-[3-(aminosulfonylamino)propanesulfonyl]-5,6,8a,9,10,11,12,12a,13,13a-decahydro-8H-isoquino[2,1-g][1,6]naphthyridine.

METHODS OF PREPARATION

Preparation of Compounds of Formula (VII)

The racemic compounds of formula (1) where n is 2 are prepared from the intermediates of formula (VII), the preparation of which is illustrated below in Reaction Scheme I.

It should be understood that the structures unless otherwise indicated in the text are intended to represent racemic mixtures, although for the sake of clarity only one enantiomer is shown.

**REACTION SCHEME I**

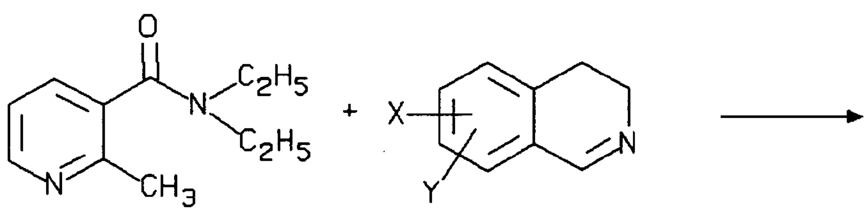

(II) (III)

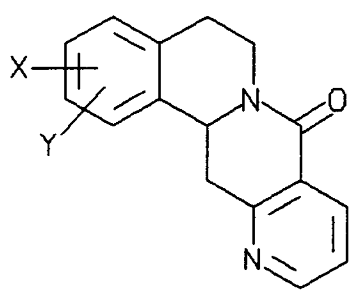

(IV)

(IV)

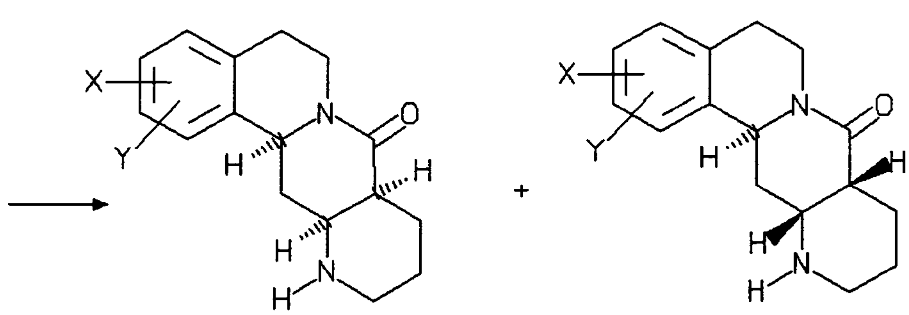

(V) (VI)

(V)

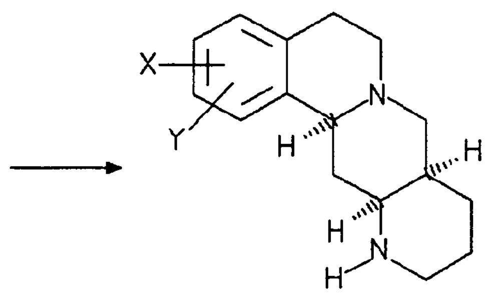

(VII)

Preparation of Compounds of Formula (IV)

The intermediate of formula (II), 2-methylnicotinic acid diethylamide, is prepared according to the method disclosed in Ber., 72B, 563 (1939). The intermediates of formula (III), optionally substituted dihydroisoquinolines, are prepared according to the method of Bischler-Napieralski, disclosed in Organic Reactions, Vol. VI, p 74 (1951), by the cyclization of formamides of commercially available optionally substituted phenylethylamines. To prepare a compound of formula (IV), a compound of formula (II) and a compound of formula (III) are reacted together in the presence of a strong base, for example potassium t-butoxide, sodamide, sodium triphenylmethane, lithium diethylamide or preferably lithium diisopropylamide. The reaction is preferably carried out in an ethereal solvent, for example diethyl ether, dimethoxyethane, dioxane or tetrahydrofuran, at a temperature of about 0°C to -50°C, preferably at about -10°C to -40°C, for about 30 minutes to 4 hours. For example, diisopropylamine is dissolved in an ethereal solvent, preferably tetrahydrofuran, and cooled to a temperature of about -20° to -80°C, preferably about -65°C. To this solution is added about 1 molar equivalent of an alkyl lithium, preferably 1.6M n-butyllithium. To this cooled solution is added a mixture of about 1 molar equivalent of the compound of formula (II) and about 1 molar equivalent of the compound of formula (III) in an ethereal solvent, preferably tetrahydrofuran. The reaction mixture is allowed to warm to about -10° to -40°C, preferably about -20°C, over a period of about 1 hour, and the reaction then quenched with an acid, preferably hydrochloric acid. The product of formula (IV), a (±)-5,6,13,13a-tetrahydroisoquino-[2,1-g] [1,6]naphthyridine-8-one hydrochloride, is isolated and purified by conventional means, preferably recrystallization.

Preparation of Compounds of Formula (V) and (VI)

A compound of formula (IV) as an acid salt, preferably the hydrochloride, is then hydrogenated in an inert solvent with a suitable heterogeneous catalyst, for example palladium on carbon, platinum oxide or preferably rhodium on alumina, to give the corresponding mixture of diastereoisomers of formula (V) and (VI). For example, for every gram of the hydrochloride of the compound of formula (IV) in a solution of acetic acid is added from 0.1 to 0.6 g, preferably about 0.25 g, of 5% rhodium on alumina catalyst and the mixture hydrogenated at a pressure of about 25-80 psi, preferably about 50 psi. The reaction is conducted at a temperature of about 0° to 50°C, preferably about 25°C, for about 24 to 72 hours, preferably about 42 hours. When the reaction is substantially complete, the mixture of compounds of formula (IV) and (V) is isolated by conventional means and the mixture chromatographed on silica gel, eluting with a suitable solvent mixture, for example 5-20% methanol in methylene chloride. The first component eluted is a (±)-5,6,8aβ,9,10,11,12,12aβ,13,13aα-decahydroisoquino[2,1-g][1,6]naphthyridin-8-one derivative, a compound of formula (VI), and the second component eluted is a (±)-5,6,8aα,9,10,11,12,12aα,13,13a-decahydroisoquino[2,1-g][1,6]naphthyridin-8-one derivative, a compound of formula (V).

Preparation of Compounds of Formula (VII)

A compound of formula (V) is then reduced to the corresponding compound of formula (VII) with a suitable reducing agent, for example borane, triethyloxonium fluoroborate followed by sodium borohydride, sodium borohydride in the presence of a carboxylic acid, or preferably lithium aluminum hydride. For example, a solution of a compound of formula (V) in an ethereal solvent, preferably tetrahydrofuran, is slowly added to a solution of about 1 to 4 molar equivalents, preferably about 1.5 to 2 molar equivalents, of lithium aluminum hydride in the same ethereal solvent at about 25°C. The mixture is then refluxed for about 1-10 hours, preferably about 3 hours. When the reaction is substantially complete, the (±)-5,6,8aα,9,10, 11,12,12aα,13,13aα-decahydro-8H-isoquino[2,1-g][1,6] naphthyridine derivative, a compound of formula (VII), is separated and purified by conventional means, for example recrystallization of an acid salt.

Separation of Optical Isomers of (V), (VI) or (VII)

Methods of obtaining the optical isomers of the compounds of formulas (V), (VI) and (VII) and related compounds is disclosed in U.S. Patent No. 4,886,798, the complete disclosure of which is hereby incorporated by reference. For example, a racemic compound of formula (V) is separable into two enantiomers, which are represented below as compounds of formulae (VA) and (VB):

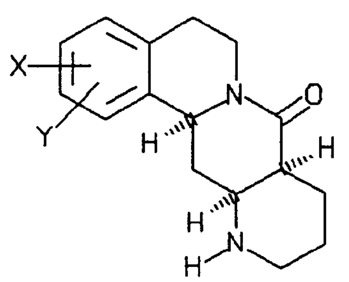

(VA)

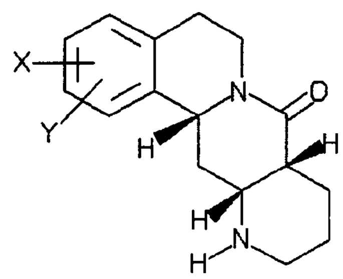

(VB)

In addition, a preferred procedure for the preparation of the enantiomers of formula (VA) is set forth in a U.S. Application, Serial No. 07/673,693, Case No. 27230, the complete disclosure of which is hereby incorporated by reference. The subject matter and the claimed invention of both the present application and the above identified application are subject to an obligation of assignment to the same Research Organization.

An enantiomer of formulae (VA) or (VB) is then reduced [in the same manner as shown above for the reduction of racemic (V)] to the appropriate enantiomer of a compound of formula (VII), which are illustrated below as (VIIA) and (VIIB).

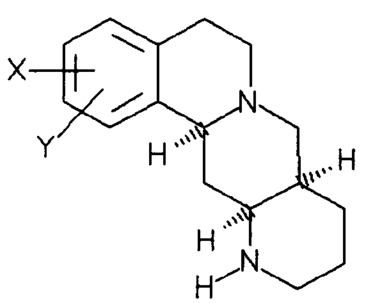

(VIIA)

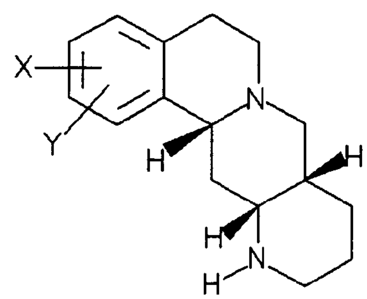

(VIIB)

Preparation of Compounds of Formula (XI)

The compounds of formula (1) where n is 1 are prepared from the intermediates of formula (XI), the preparation of which is illustrated below in Reaction Scheme II.

It should be understood that the structures unless otherwise indicated in the text are intended to represent racemic mixtures, although for the sake of clarity only one enantiomer is shown.

## REACTION SCHEME II

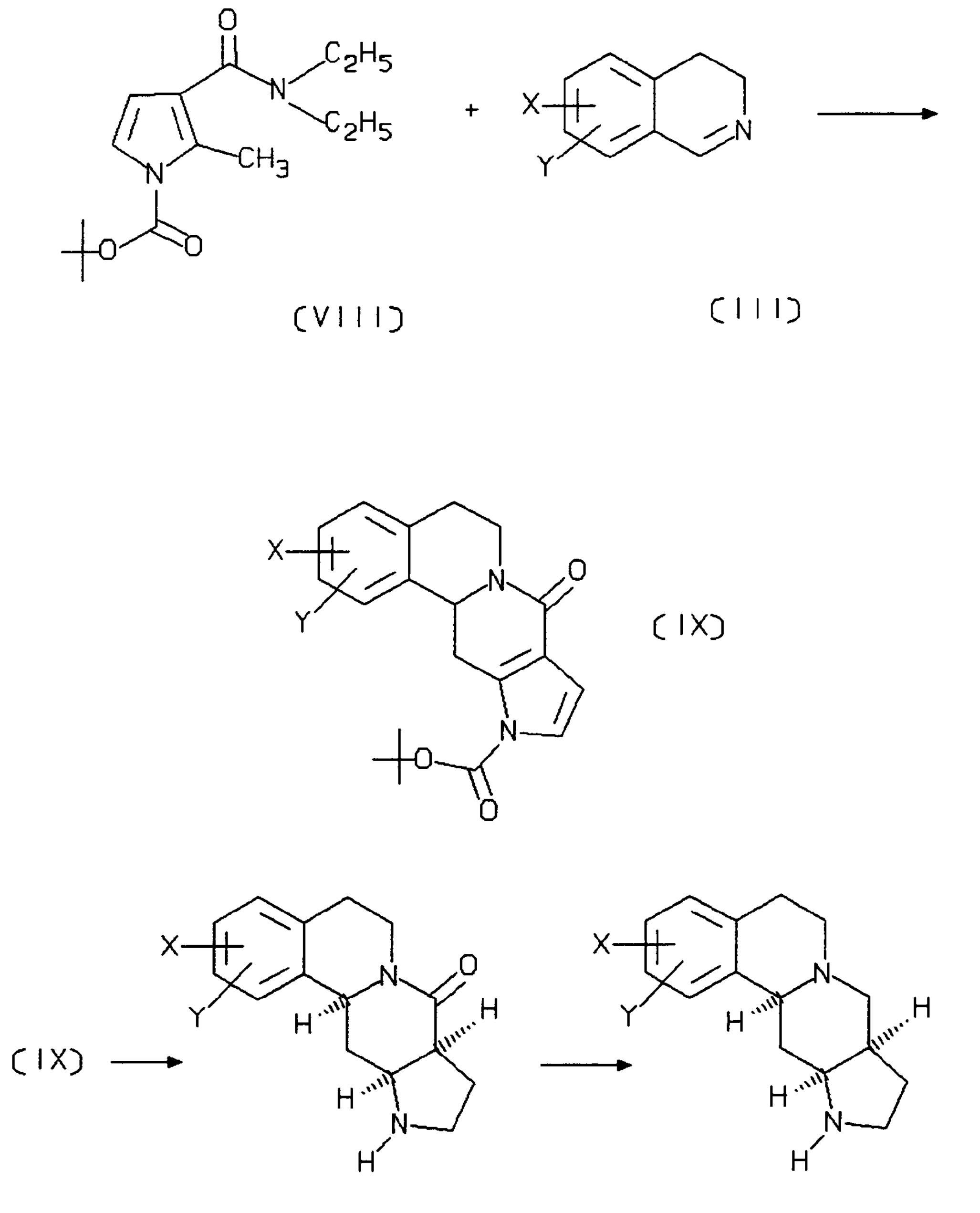

(VIII)

(III)

(IX)

(IX)

(X)

(XI)

Preparation of the Intermediate of Formula (VIII)

The intermediate of formula (VIII), 1-tert-butoxycarbonyl-2-methylpyrrole-3-carboxylic acid diethylamide, is prepared from 3-(ethoxycarbonyl)-2-methylpyrrole, as shown in Reaction Scheme III below:

## REACTION SCHEME III

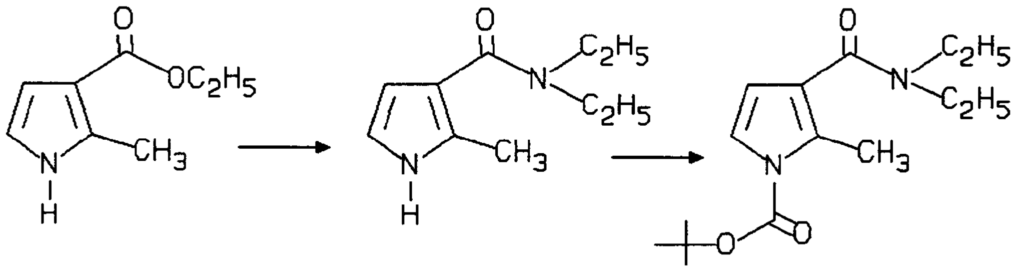

(a) (b) (VIII)

The starting 3-(ethoxycarbonyl)-2-methylpyrrole of formula (a) is prepared by the method disclosed in J. Org. Chem., Vol. 49, pp 3327-3336 (1984). This material is converted to 2-methylpyrrole-3-carboxylic acid diethylamide of formula (b), according to the method disclosed in Tetrahedron Letters, No. 48, pp 4171-4174 (1977), which is converted to 1-tert-butoxycarbonyl-2-methylpyrrole-3-carboxylic acid diethylamide of formula (VIII) by the procedure disclosed in J. Org Chem., Vol. 46, pp 157-164 (1981).

Preparation of Compounds of Formula (IX)

To prepare a compound of formula (IX), the anion of a compound of formula (VIII) is first formed by reaction with a strong base, for example potassium t-butoxide, sodamide, sodium triphenylmethane, lithium diethylamide or preferably lithium diisopropylamide. The reaction is preferably carried out in an ethereal solvent, for example diethyl ether, dimethoxyethane, dioxane, most preferably tetrahydrofuran, at a temperature of about -100°C to -50°C, preferably at about -70°C, for about 5 minutes to 1 hour, preferably about 30 minutes. For example, diisopropylamine is dissolved in an ethereal solvent, preferably tetrahydrofuran, and cooled to a temperature of about -60° to -80°C, preferably about -70°C. To ths solution is added about 1 molar equivalent of an alkyl lithium, preferably 1.6M n-butyllithium. To this cooled solution is added a mixture of about 1 molar equivalent of a compound of formula (VIII) in an ethereal solvent, preferably tetrahydrofuran, followed by about 1 molar equivalent of a compound of formula (III) in the same solvent. After about 5 minutes to 1 hour, preferably about 30 minutes, the reaction mixture is allowed to warm to about -20° to -40°C, preferably about -30°C, over a period of about 30 minutes, and quenched with water. The (±)-11-(tert-butoxy-carbonyl)benzo[a]pyrrole[2,3-e]quinolizin-8-one derivative, a compound of formula (IX), is isolated and purified by conventional means, preferably chromatography followed by recrystallization.

Preparation of Compounds of Formula (X)

A compound of formula (IX) is then hydrogenated with a suitable heterogeneous catalyst, for example palladium on carbon, platinum oxide or preferably rhodium on alumina, to give the corresponding compound of formula (X). For example, for every gram of a compound of formula (IX) dissolved in a suitable solvent, for example acetic acid or preferably ethanol, is added from 0.1 to 0.6 g, preferably about 0.25 g, of 5% rhodium on alumina catalyst and the mixture hydrogenated at a pressure of about 25-80 psi, preferably about 40 psi. The reaction is conducted at a temperature of about 0° to 50°C, preferably about 25°C, until uptake of hydrogen ceases, usually about 2-4 hours. When the reaction is substantially complete, the product is separated conventionally and dissolved in an inert organic solvent, preferably dichloromethane. An excess of a strong acid, preferably trifluoroacetic acid, is then added in order to remove the t-butoxycarbonyl protecting group. The reaction is conducted at a temperature of about 0° to 50°C, preferably about 25°C, for about 5-48 hours, preferably about 18 hours. When the reaction is complete a (±)-(8aα,11aα,12aα)-5,6,8,8a,9,10,11,11a,12,12a-decahydrobenzo[a]pyrrolo[2,3-e]quinolizin-8-one derivative, a compound of formula (X), is separated and purified by conventional means, preferably chromatography on silica gel.

Preparation of Compounds of Formula (XI)

A compound of formula (X) is then reduced to the compound of formula (XI) with a suitable reducing agent, for example borane, triethyloxonium fluoroborate followed by sodium borohydride, sodium borohydride in the presence of a carboxylic acid, or preferably lithium aluminum hydride. For example, a solution of a compound

of formula (X) in an ethereal solvent, preferably tetrahydrofuran, is slowly added to a solution of about 1 to 4 molar equivalents, preferably about 1.5 to 2 molar equivalents, of lithium aluminum hydride in the same ethereal solvent at about 25°C. The mixture is then refluxed for about 1-10 hours, preferably about 3 hours. When the reaction is substantially complete, a (±)-(8aα,11aα,12aα)-5,6,8,8a,9,10,11,11a,12,12a-decahydrobenzo[a]pyrrolo[2,3-e]quinolizine derivative, a compound of formula (XI), is separated and purified by conventional means, or alternatively may be used in the next reaction with no further purification.

Separation of Optical Isomers of (X) or (XI)

The optical isomers of the compounds of formula (X) or (XI) may be obtained by conventional resolution means; for example by separation (e.g. fractional crystallization) of the diastereomeric salts formed by the reaction of a compound of formula (X) or (XI) with an optically active acid, at temperatures between 0°C and the reflux temperature of the solvent employed for fractional crystallization. Exemplary of such optically active acids are the optically active forms of camphor-10-sulfonic acid, 2-bromo-camphor-10-sulfonic acid, camphoric acid, menthoxyacetic acid, tartaric acid, malic acid, diacetyltartaric acid, pyrrolidine-5-carboxylic acid and the like. The separated pure diastereomeric salts may then be cleaved by standard means, such as treatment with a base, to afford the respective optical isomers of the compounds of formula (X) or (XI).

Alternatively, similar methods to those discussed above for the preparation of the optical isomers of the compounds of formula (VII) and related compounds may be employed.

Preparation of Compounds of Formula (1)

Compounds of formula (1) where n is 2 are prepared from the compounds of formula (VII), and compounds of formula (1) where n is 1 are prepared from the compounds of formula (XI).

Preparation of Compounds of Formula (1) where R is lower alkyl, cycloalkyl, or optionally substituted phenyl

The compounds of formula (1) where R is lower alkyl, cycloalkyl, or optionally substituted phenyl are prepared as depicted in Reaction Scheme IV below.

REACTION SCHEME IV

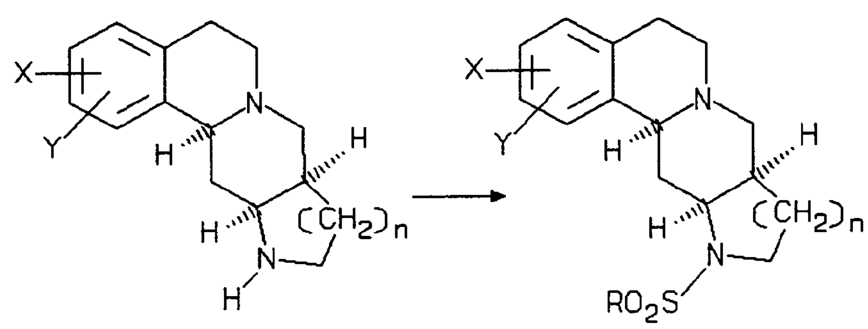

(VII) or (XI) (1)

A compound of formula (1) where n is 1 or 2 and R is lower alkyl, cycloalkyl, or optionally substituted phenyl are prepared by reacting the corresponding individual compound of formula (VII) or (XI) with a substituted sulfonyl halide of the formula $ZSO_2R$, where Z is chlorine or bromine and R is lower alkyl, cycloalkyl, or optionally substituted phenyl. The sulfonyl halides of formula $ZSO_2R$ are either commercially available from, inter alia, Aldrich Chemical Co., or may be prepared according to the method of Zeigler and Sprague, disclosed in J. Org. Chem., Vol 16, p 621 (1951).

For example, a compound of formula (VII) or (XI) is dissolved in an inert organic solvent, such as benzene, toluene, ethyl acetate, tetrahydrofuran, diethyl ether, chloroform or preferably dichloromethane, containing from 1-10 molar equivalents, preferably about 2 molar equivalents, of an inorganic base such as sodium carbonate, potassium bicarbonate or the like, or preferably a tertiary organic base, such as pyridine, N-methylpiperidine

and the like, preferably triethylamine. The mixture is cooled to about -10° to 10°C, preferably about 0°C, and about 1-4 molar equivalents, preferably about 1.25 molar equivalents, of the appropriately substituted sulfonyl halide of formula $ZSO_2R$ added and the mixture stirred for about 30 minutes to 4 hours, preferably about 1 hour at a temperature of about 10° to 40°C, preferably about 25°C. An inert solvent, preferably diethyl ether, is then added, and a compound of formula (1) where R is lower alkyl, cycloalkyl, or optionally substituted phenyl, (an (8aα,11aα,12aα)-11-(substituted-sulfonyl)-5,6,8,8a,9,10,11,11a,12,12a-decahydrobenzo [a]-pyrrolo[2,3-e]quinolizine derivative, or an (8aα,12aα,13aα)-12-(substituted-sulfonyl)-5,6,8a,9,10,11,12,12a,13,13a-decahydro-8H-isoquino[2,1-g][1,6]naphthyridine derivative), is separated and purified by conventional means, for example recrystallization of an acid salt.

Alternative Preparation of Compounds of Formula (1) where n is 2

Other synthetic methods for the preparation of compounds of formula (1) where n is 2 are described in U.S. Patent No. 4,791,108, the complete disclosure of which is hereby incorporated by reference.

Preferred Preparation of Compounds of Formula (1)

A preferred procedure for the conversion of a compound of formulae (V), (VA) or (VB), or (X) or an enantiomer of (X), to the corresponding compound of formula (1) where n is 1 or 2 consists of first reacting (V) or (VA), or (X) or an enantiomer of (X), with an appropriately substituted sulfonyl halide of formula $ZSO_2R$, and then reducing the lactam thus formed to give the compound of formula (1). This reaction sequence is set forth in more detail in U.S. Application Serial No. 07/673,693, Case No. 27230, and in U.S. Patent No. 4,960,891, the complete disclosures of which are hereby incorporated by reference.

Preparation of Compounds of Formula (1) where R is $-ANHSO_2R^1$

A compound of formula (1) in which R is $-ANHSO_2R^1$, where Z is chlorine or bromine and A, $R^1$, X, Y, and n are as defined above, is prepared as depicted in Reaction Scheme V below.

## REACTION SCHEME V

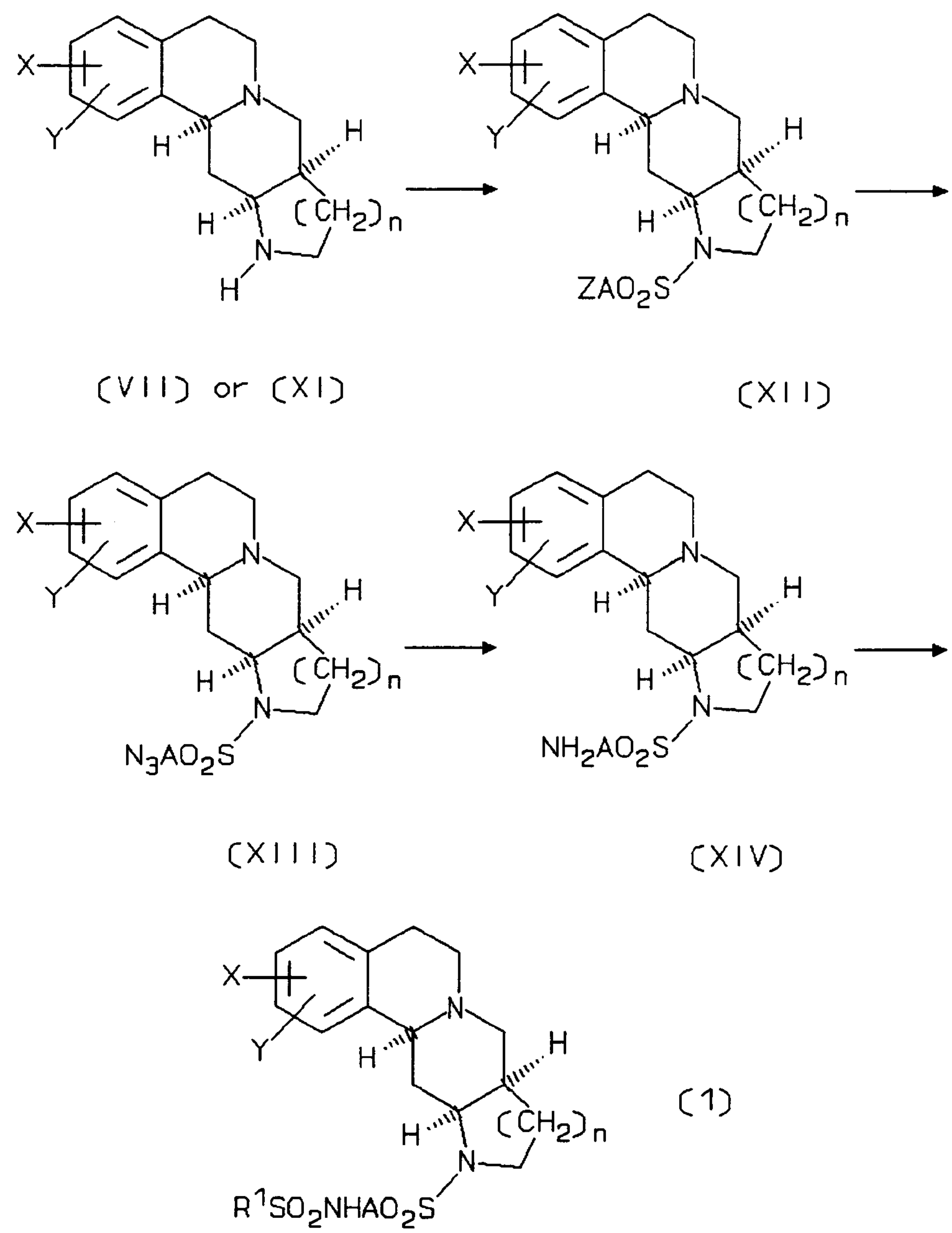

(VII) or (XI)

(XII)

(XIII)

(XIV)

(1)

### Preparation of Compounds of Formula (XII)

In the first step, a compound of formulae (VII) or (XI) is reacted with a compound of the formula $ZASO_2Z$, where A and Z are as defined above. The reaction is carried out in an inert organic solvent as defined above, preferably dichloromethane, in the presence of about 1-10 molar equivalents, preferably about 2 molar equivalents, of an inorganic base or tertiary organic base as defined above, preferably triethylamine. The mixture is cooled to a temperature of about -10°C to 30°C, preferably about 0°C, and about 1-4 molar equivalents, preferably about 1.1 molar equivalents, of the appropriately substituted haloalkylsulfonyl halide of formula $ZASO_2Z$ is added. The mixture is stirred for about 5 minutes to 1 hour, preferably about 30 minutes. When the reaction is substantially complete the corresponding product of formula (XII), (an (8aα,11aα,12aα)-11-(ω-haloalkylsulfonyl)-5,6,8,8a,9,10,11,-11a,12,12a-decahydrobenzo [a]pyrrolo[2,3-e]quinolizine derivative, or an (8aα, 12aα,13aα)-12-(ω-haloalkylsulfonyl)-5,6,8a,9,10,11,12, 12a,13,13a-decahydro-8H-isoquino[2,1-g][1,6]naphthyridine derivative), is isolated and purified by conventional means, preferably chromatography followed by recrystallization of an acid salt, preferably a hydrochloride salt.

Preparation of Compounds of Formula (XIII)

A compound of formula (XII) as the free base is then reacted with an alkali metal azide to give the corresponding compound of formula (XIII). In general a compound of formula (XII) is reacted with about 1-5 molar equivalents, preferably about 2 molar equivalents, of an alkali metal azide, preferably sodium azide, in the presence of a catalytic amount of an alkali metal iodide, preferably sodium iodide. The reaction is carried out in an aprotic polar solvent, preferably dimethylformamide, at a temperature of about 50°C to 120°C, preferably about 80°C, for about 5-48 hours, preferably about 18 hours. When the reaction is substantially complete a product of formula (XIII), (an (8aα,11aα,12aα)-11-(ω-azidoalkylsulfonyl)5,6,8,8a,9,10,11,11a,12,12a-decahydrobenzo[a]pyrrolo[2,3-e]quinolizine derivative, or an (8aα,12aα,13aα)-12- (ω-azidoalkylsulfonyl)-5,6,8a,9,10,11,12,12a,13,13a-decahydro -8H-isoquino[2,1-g][1,6]naphthyridine derivative), is isolated by conventional means, and preferably used in the next reaction with no further purification.

Preparation of Compounds of Formula (XIV)

A compound of formula (XIII) is then reduced with a suitable reducing agent, for example borane, sodium borohydride in the presence of a carboxylic acid, or lithium aluminum hydride, or preferably hydrogenated under acid conditions using a suitable heterogeneous catalyst, for example rhodium on alumina, platinum oxide or preferably palladium hydroxide on carbon, to give the corresponding compound of formula (XIV). For example, for every gram of the compound of formula (XIII) in a solution of ethanol/ hydrochloric acid is added from 0.1 to 0.6 g, preferably about 0.4 g, of palladium hydroxide on carbon catalyst and the mixture hydrogenated at a pressure of about 25-80 psi, preferably about 50 psi. The reaction is conducted at a temperature of about 0° to 50°C, preferably about 25°C, for about 1-12 hours, preferably about 3 hours. When the reaction is substantially complete, a compound of formula (XIV), (an (8aα,11aα,12aα)-11-(ω-aminoalkyl-sulfonyl)-5,6,8,8a,9,10,11,11a,12,12a-decahydrobenzo-[a]pyrrolo-[2,3-e]quinolizine derivative, or an (8aα,12aα,13aα)-12- (ω-aminoalkylsulfonyl)-5,6,8a,9,-10,11,12,12a,13,13a-decahydro -8H-isoquino[2,1-g][1,6]naphthyridine derivative), is isolated and purified by conventional means, preferably chromatography followed by recrystallization of an acid salt, preferably the hydrochloride salt.

Preparation of Compounds of Formula (1)

A compound of formula (XIV) is then reacted with a compound of the formula $ZSO_2R^1$, where Z and $R^1$ are as defined above. The reaction is carried out in an inert organic solvent as defined above, preferably dichloromethane, in the presence of about 1-10 molar equivalents, preferably about 2 molar equivalents, of an inorganic base or tertiary organic base as defined above, preferably triethylamine. The mixture is maintained at a temperature of about 0°C to 30°C, preferably about 0°C where $R^1$ is alkyl, and preferably about 25°C where $R^1$ is $-NR^2R^3$, and about 1-4 molar equivalents, preferably about 1.1 molar equivalents, of the appropriately substituted compound of the formula $ZSO_2R^1$ is added. In the case where $R^1$ is alkyl, the mixture is stirred for about 5 minutes to 1 hour, preferably about 30 minutes. In the case where $R^1$ is $-NR^2R^3$, the mixture is stirred for about 12 hours to 72 hours, preferably about 18 hours. When the reaction is substantially complete the corresponding product of formula (1), (an (8aα,11aα,12aα)-11-(ω-substituted-sulfonylamino-alkylsulfonyl)-5,6,8,8a,9,10,11,11a,12,12a-decahydrobenzo[a]pyrrolo[2,3-e]quinolizine derivative, or an (8aα,12aα, 13aα)-12-(ω-substituted-sulfonylamino-alkylsulfonyl)-5,6,8a,9,10,11,12,12a,13,13a-decahydro-8H-isoquino[2,1-g][1,6]naphthyridine derivative), is isolated and purified by conventional means, preferably chromatography followed by recrystallization of an acid salt, for example a maleic acid salt.

Preparation of Compounds of Formula (1) where $R^1$ is $-NH_2$

To prepare a compound of formula (1) where $R^1$ is $-NH_2$, a compound of formula (XIV) is first reacted with a compound of the formula $ZSO_2NHR^2$, where Z is as defined above and $R^2$ is tert-butyl. Similarly, to prepare a compound of formula (1) where $R^1$ is $-NHR^3$, a compound of formula (XIV) is first reacted with a compound of the formula $ZSO_2NR^2R^3$, where Z and $R^3$ are as defined above and $R^2$ is tert-butyl. The reaction is carried out in the same manner as described above for the case where $R^1$ is $-NR^2R^3$. When the reaction is substantially complete the product is treated with trifluoroacetic acid at a temperature of about 0° to 50°C, preferably about 25°C, for about 6-48 hours, preferably about 18 hours. When the reaction is substantially complete, the product of formula (1) where $R^1$ is $-NH_2$ or $-NHR^3$, (an (8aα,11aα,12aα)-11-(ω-aminosulfonylaminoalkylsulfonyl)-5,6,8,8a,9,10,11,11a,12,12a-decahydrobenzo[a]pyrrolo[2,3-e]quinolizine derivative, or an (8aα,12aα,13aα)-12-(ω-aminosulfonylaminoalkylsulfonyl)-5,6,8a,9,10,11,12,12a,13,13a-decahydro-8H-isoquino[2,1-g][1,6]naphthyridine derivative), is isolated and purified by conventional means, preferably chromatography followed by recrystallization.

Isolation and Purification of the Compounds

Isolation and purification of the compounds and intermediates described herein can be effected, if desired, by any suitable separation or purification procedure such as, for example, filtration, extraction, crystallization, column chromatography, thin-layer chromatography, thick-layer chromatography, preparative low or high-pressure liquid chromatography or a combination of these procedures. Specific illustrations of suitable separation and isolation procedures can be had by reference to the Examples hereinbelow. However, other equivalent separation or isolation procedures could, of course, also be used.

Separation of the Enantiomers of Compounds of Formula (1)

In addition to the procedures discussed above for the preparation of the individual enantiomers of compounds of formula (1), the enantiomers of compounds of formula (1) may also be obtained by conventional resolution means, for example by separation (e.g. fractional crystallization) of the diastereomeric salts formed by the reaction of a racemic compound of formula (1) with an optically active acid, as discussed above for the separation of the optical isomers of compounds of formula (X) or (XI).

Salts of Compounds of Formula (1)

The compounds of formula (1) may be converted to a corresponding acid addition salt by virtue of the presence of the tertiary nitrogen atoms.

The conversion is accomplished by treatment with at least a stoichiometric amount of an appropriate acid, such as hydrochloric acid, hydrobromic acid, sulfuric acid, nitric acid, phosphoric acid and the like, and organic acids such as acetic acid, propionic acid, glycolic acid, pyruvic acid, oxalic acid, malic acid, malonic acid, succinic acid, maleic acid, fumaric acid, tartaric acid, citric acid, benzoic acid, cinnamic acid, mandelic acid, menthanesulfonic acid, ethanesulfonic acid, p-toluenesulfonic acid, salicylic acid and the like. Typically, the free base is dissolved in an inert organic solvent such as diethyl ether, ethyl acetate, chloroform, ethanol or methanol and the like, and the acid added in a similar solvent. The temperature is maintained at 0°-50°C. The resulting salt precipitates spontaneously or may be brought out of solution with a less polar solvent.

The acid addition salts of the compounds of formula (1) may be converted to the corresponding free bases by treatment with at least a stoichiometric amount of a suitable base such as sodium or potassium hydroxide, potassium carbonate, sodium bicarbonate, ammonia, and the like.

Preferred Processes

In summary, the compounds of the present invention are made according to the following last steps.

1. A process for preparing compounds of the formula (1), wherein:

X and Y are independently hydrogen; hydroxy; lower alkyl of 1-6 carbon atoms; lower alkoxy of 1-6 carbon atoms; or halo; or X and Y when adjacent and taken together are methylenedioxy or ethylene-1,2-dioxy;

R is lower alkyl of 1-6 carbon atoms; cycloalkyl of 3-8 carbon atoms; phenyl or phenyl lower alkyl in which any phenyl group may be optionally substituted by one or two substituents chosen from the group consisting of halo, lower alkyl of 1-4 carbon atoms and lower alkoxy of 1-4 carbon atoms; or $-ANHSO_2R^1$; wherein A is lower alkylene of 1-6 carbon atoms; and $R^1$ is lower alkyl of 1-6 carbon atoms or $-NR^2R^3$; wherein

$R^2$ and $R^3$ are independently hydrogen or lower alkyl of 1-6 carbon atoms, or $R^2$ and $R^3$ taken together are cycloalkyl of 3-8 carbon atoms; and

n is 1 or 2;

constitutes:

(a) reacting a compound of the formula:

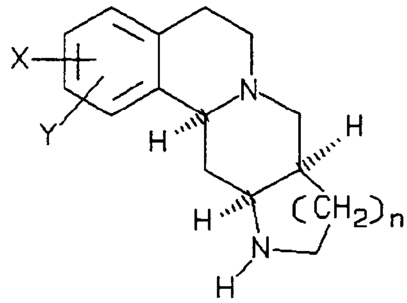

(VII) or (XI)

with a compound of the formula $ZSO_2R$, where R is lower alkyl, cycloalkyl, or optionally substituted phenyl and Z is chlorine or bromine; or

(b) reacting the free base of the compound of formula (1) with an acid to give a pharmaceutically acceptable acid addition salt; or

(c) reacting an acid addition salt of the compound of formula (1) with a base to give the corresponding free base; or

(d) converting an acid addition salt of the compound of formula (1) to another pharmaceutically acceptable acid addition salt of formula (1).

2. Alternatively, a process for preparing compounds of the formula (1) where R is $-ANHSO_2R^1$, in which;

A is lower alkylene of 1-6 carbon atoms; and

$R^1$ is lower alkyl of 1-6 carbon atoms or $-NR^2R^3$; where

$R^2$ and $R^3$ are independently hydrogen or lower alkyl of 1-6 carbon atoms, or

$R^2$ and $R^3$ taken together are cycloalkyl of 3-8 carbon atoms;

constitutes reacting a compound of the formula:

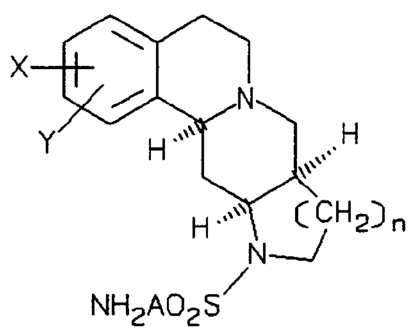

(XIV)

where A, X, Y and n are as defined above, with a compound of the formula $ZSO_2R^1$, where $R^1$ and Z are as defined above.

3. Alternatively, a process for preparing compounds of the formula (1) constitutes reacting a compound of the formula:

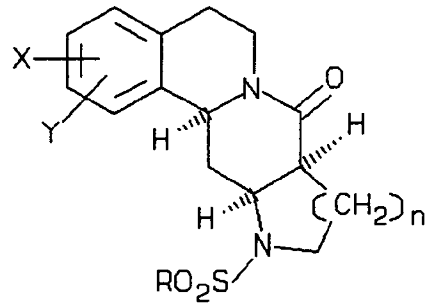

where n, R, X and Y are as defined above, with a suitable reducing agent, preferably sodium borohydride in the presence of boron trifluoride etherate.

4. Alternatively, a process for preparing compounds of the formula (1) constitutes reacting a compound of the formula:

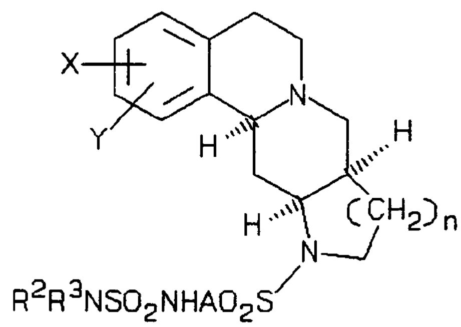

where $R^2$ is tertiary butyl and n, A, $R^3$, X and Y are as defined above, with trifluoroacetic acid.

Utility and Administration:

General Utility

The compounds of formula (1) and the pharmaceutically acceptable acid addition salts thereof have been found to possess valuable pharmacological properties and, in particular, have been shown to selectively block $\alpha_2$-adrenoceptors in standard laboratory tests. Accordingly these compounds and pharmaceutically acceptable compositions containing them are useful in the regulation of physiological phenomena related to $\alpha_2$-receptors, including lowering of blood pressure in hypertensive mammals, treatment of irritable bowel syndrome, inhibition of platelet aggregation, treatment of hyperglycemia, alleviation of male impotence, palliation of diabetes, and lowering of intraocular pressure. In addition, the compounds of formula (1) are particularly useful for the treatment of peripheral vascular disease, including diabetes and its sequelae such as diabetic retinopathy, nephropathy, neuropathy and associated circulatory disturbances, and intermittent claudication and Raynaud's disease. Necrotic skin lesions and related skin disorders are also amenable to such treatment.

Testing

Potential for $\alpha_2$-adrenoceptor antagonism is determined in vitro by the method, described in Example 14, of Arunlakshana and Schild, Br. J. Pharmacol. Chemother., Vol. 14, 48-58 (1959). Alternatively, such activity may be established by the procedure according to Caroon, J.M. et al., J. Med. Chem., 1982, Vol. 25, 666.

Platelet aggregation inhibition is determined in vitro by the turbidimetric method of Born, J. Physiol., 162, p67 (1962).

Lowering of intraocular pressure is shown in vivo by the method of Moses, R. A., Tr. Am. Acad. Opth. and Otol., Jan-Feb 1962: 88-95.

Alleviation of male impotence is shown in vivo as described by P. Sodersten, D. A. Dammassa and E. R. Smith, Hormones and Behavior, Vol. 8, pp 320-334 (1977).

Alleviation of irritable-bowel syndrome is shown by a test that is a modification of the method of Macht and Barba-Gose (Macht, D. T. and Barba-Gose, J. (1931): J. Amer. Pharm. Ass. 20, 558).

The antihypertensive activity of the compounds may be determined in conscious spontaneous hypertensive rats prepared with indwelling arterial catheter by the in vivo assay described in Popovic V. and Popovic P., J. Applied. Physiol., Vol. 15, pp. 727-728 (1960), or a modification thereof.

Selective vasodilating activity is shown in vivo by an unpublished method developed by W. S. Redfern et al.

Peripheral selectivity activity is shown by the methods described in Naunyn-Schniedeberg's Arch. Pharmac., Vol. 307, p 45-50 (Koss and Christensen 1979); Br. J. Pharmac., Vol. 78, p 507-515 (Beridge et al., 1983); and Br. J. Pharmac., Vol. 83, p 707-712 (Gadie et al., 1984)

## General Administration

In applying the compounds of this invention to treatment of the above conditions, administration of the active compounds and salts described herein can be via any of the accepted modes of administration for $\alpha_2$-adrenoceptor antagonists including oral, parenteral and otherwise systemic route of administration. Any pharmaceutically acceptable mode of administration can be used, including solid, semi-solid or liquid dosage forms, such as, for example, tablets, suppositories, pills, capsules, powders, liquids, suspensions, or the like, preferably in unit dosage forms suitable for single administration of precise dosages, or in sustained or controlled release dosage forms for the prolonged administration of the compound at a predetermined rate. The compositions will typically include a conventional pharmaceutical carrier or excipient and an active compound of formula (1) or the pharmaceutically acceptable salts thereof and, in addition, may include other medicinal agents, pharmaceutical agents, carriers, adjuvants, etc.

The amount of active compound administered will of course, be dependent on the subject being treated, the severity of the affliction, the manner of administration and the judgment of the prescribing physician. However, an effective dosage is in the range of 0.001-20 mg/kg/day, preferably 0.005-10 mg/kg/day. For an average 70 kg human, this would amount to 0.070-1400 mg per day, or preferably 0.35-700 mg/day.

For solid compositions, conventional non-toxic solid carriers include, for example, pharmaceutical grades of mannitol, lactose, starch, magnesium stearate, sodium saccharin, talcum, cellulose, sodium crosscarmellose, glucose, sucrose, magnesium carbonate, and the like may be used. The active compound as defined above may be formulated as suppositories using, for example, polyalkylene glycols, for example, propylene glycol, as the carrier. Liquid pharmaceutically administrable compositions can, for example, be prepared by dissolving, dispersing, etc. an active compound as defined above and optional pharmaceutical adjuvants in a carrier, such as, for example, water, saline, aqueous dextrose, glycerol, ethanol, and the like, to thereby form a solution or suspension. If desired, the pharmaceutical composition to be administered may also contain minor amounts of nontoxic auxiliary substances such as wetting or emulsifying agents, pH buffering agents and the like, for example, sodium acetate, sorbitan monolaurate, triethanolamine sodium acetate, sorbitan monolaurate, triethanolamine oleate, etc. Actual methods of preparing such dosage forms are known, or will be apparent, to those skilled in this art; for example, see Remington's Pharmaceutical Sciences, Mack Publishing Company, Easton, Pennsylvania, 16th Edition, 1980. The composition or formulation to be administered will, in any event, contain a quantity of the active compound(s) in an amount effective to alleviate the symptoms of the subject being treated.

Dosage forms or compositions containing an active ingredient (compounds of formula (1) or its salts) in the range of 0.025 to 95% with the balance made up from non-toxic carrier may be prepared.

For oral administration, a pharmaceutically acceptable non-toxic composition is formed by the incorporation of any of the normally employed excipients, such as, for example pharmaceutical grades of mannitol, lactose, starch, magnesium stearate, sodium saccharin, talcum, cellulose, sodium crosscarmellose, glucose, sucrose, magnesium, carbonate, and the like. Such compositions take the form of solutions, suspensions, tablets, capsules, powders, sustained release formulations and the like. Such compositions may contain 0.1%-95% active ingredient, preferably 0.5-80%.

Parenteral administration is generally characterized by injection, either subcutaneously, intramuscularly or intravenously. Injectables can be prepared in conventional forms, either as liquid solutions or suspensions, solid forms suitable for solution or suspension in liquid prior to injection, or as emulsions. Suitable excipients are, for example, water, saline, dextrose, glycerol, ethanol or the like. In addition, if desired, the pharmaceutical compositions to be administered may also contain minor amounts of non-toxic auxiliary substances such as wetting or emulsifying agents, pH buffering agents and the like, such as for example, sodium acetate, sorbitan monolaurate, triethanolamine oleate, etc.

A more recently devised approach for parenteral administration employs the implantation of a slow-release or sustained-release system, such that a constant level of dosage is maintained. See, e.g., U.S. Patent No. 3,710,795, which is hereby incorporated by reference.

The percentage of active compound contained in such parental compositions is highly dependent on the specific nature thereof, as well as the activity of the compound and the needs of the subject. However, percentages of active ingredient of 0.01% to 10% in solution are employable, and will be higher if the composition is a solid which will be subsequently diluted to the above percentages. Preferably the composition will comprise 0.02-8% of the active agent in solution.

For systemic administration via suppository, traditional binders and carriers include, e.g. polyalkylene glycols or triglycerides. Such suppositories may be formed from mixtures containing active ingredient in the range of 0.05%-10%, preferably 0.1-2%.

In applying the compounds of the invention to treatment of diseases or disorders of the eye which are associated with an abnormally high intraocular pressure, administration may be achieved by any pharmaceutically acceptable mode of administration which provides adequate local concentrations to provide the desired response. These include direct administration to the eye via drops and controlled release inserts or implants, as well as systemic administration as previously described.

Drops and solutions applied directly to the eye are typically sterilized aqueous solutions containing 0.001% to 10%, most preferably 0.005% to 1% of the active ingredient, along with suitable buffer, stabilizer, and preservative. The total concentration of solutes should be such that, if possible, the resulting solution is isotonic with the lacrimal fluid (though this is not absolutely necessary) and has an equivalent pH in the range of pH 6-8. Typical sterilants are phenyl mercuric acetate, thimerosal, chlorobutanol, and benzalconium chloride. Typical buffer systems and salts are based on, for example, citrate, borate or phosphate; suitable stabilizers include glycerin and polysorbate 80. The aqueous solutions are formulated simply by dissolving the solutes in a suitable quantity of water, adjusting the pH to about 6.8-8.0, making a final volume adjustment with additional water, and sterilizing the preparation using methods known to those in the art.

The dosage level of the resulting composition will, of course, depend on the concentration of the drops, the condition of the subject and the individual magnitude of responses to treatment. However, a typical ocular composition could be administered at the rate of about 2-10 drops per day per eye of a 0.1% solution of active ingredient.

The compositions of the present invention may also be formulated for administration in any convenient way by analogy with other topical compositions adapted for use in mammals. These compositions may be presented for use in any conventional manner with the aid of any of a wide variety of pharmaceutical carriers or vehicles. For such topical administration, a pharmaceutically acceptable non-toxic formulation can take the form of semisolid, liquid, or solid, such as, for example, gels, creams, lotions, solutions, suspensions, ointments, powders, or the like. As an example, the active components may be formulated into a gel using ethanol, propylene glycol, propylene carbonate, polyethylene glycols, diisopropyl adipate, glycerol, water, etc., with appropriate gelling agents, such as Carbomers, Klucels, etc. If desired, the formulation may also contain minor amounts of non-toxic auxiliary substances such as preservatives, antioxidants, pH buffering agents, surface active agents, and the like. Actual methods of preparing such dosage forms are known, or will be apparent, to those skilled in the art; for example, see Remington's Pharmaceutical Sciences, Mack Publishing Company, Easton, Pennsylvania, 16th Edition, 1980.

The following preparation and examples illustrate the invention but are not intended to limit its scope.

## PREPARATION 1

### Preparation of (±)-5,6,13,13a-Tetrahydroisoquino-[2,1-g][1,6]naphthyridin-8-one hydrochloride and Related Compounds of Formula (IV)

A. Preparation of Formula (IV) where X and Y are hydrogen

Diisopropylamine (28 ml) and 150 ml of tetrahydrofuran were cooled to -65°C and 125 mL of 1.6 M n-butyllithium was added. To the resulting solution was added a solution of 16.2 g of 3,4-dihydroisoquinoline and 38.4 g of 2-methylnicotinic acid diethylamide in tetrahydrofuran. The mixture was allowed to warm to -20°C and 600 ml of 3N hydrochloric acid was then added followed by 200 ml of water. The mixture was basified with $NH_4OH$ and extracted twice with ether. The ether extracts were combined, dried over anhydrous magnesium sulfate and evaporated to a residue, which was dissolved in methanol and acidified with anhydrous HCl in ether. Acetone (50 ml) was added and the mixture was allowed to stand overnight. The crystalline product was collected by filtration, yielding 34 g of (±)-5,6,13,13a-tetrahydroisoquino[2,1-g][1,6]naphthyridine-8-one hydrochloride, m.p. 220-222°C.

An additional 7.5 g of the title compound as the free base was obtained by evaporation of the mother liquor followed by partitioning between ether and aqueous $NH_4OH$ and silica gel chromatography of the residue obtained from evaporation of the ether, eluting with ethyl acetate, giving the free base, m.p. 72-73°C.

B. Preparation of Formula (IV) varying X and Y

Similarly, replacing 3,4-dihydroisoquinoline with the following compounds of formula (III):
6-methoxy-3,4-dihydroisoquinoline;
6,7-dimethoxy-3,4-dihydroisoquinoline;
5,8-dimethoxy-3,4-dihydroisoquinoline;
6,7-methylenedioxy-3,4-dihydroisoquinoline; and
6,7-(ethylene-1,2-dioxy)-3,4-dihydroisoquinoline;
and following the procedure of Preparation 1A above, the following compounds of formula (IV) were prepared:
(±)-3-methoxy-5,6,13,13a-tetrahydroisoquino[2,1-g][1,6]naphthyridine-8-one hydrochloride, m.p. 244-246°C;
(±)-3-methoxy-5,6,13,13a-tetrahydroisoquino-[2,1-g][1,6]naphthyridine-8-one, m.p. 115-116°C;
(±)-2,3-dimethoxy-5,6,13,13a-tetrahydroisoquino[2,1-g][1,6]naphthyridine-8-one hydrochloride, m.p. 238-240°C;
(±)-1,4-dimethoxy-5,6,13,13a-tetrahydroisoquino[2,1-g][1,6]naphthyridine-8-one hydrochloride;
(±)-2,3-methylenedioxy-5,6,13,13a-tetrahydroisoquino[2,1-g][1,6]naphthyridine-8-one, m.p. 177-179°C; and
(±)-2,3-(ethylene-1,2-dioxy)-5,6,13,13a-tetrahydroisoquino[2,1-g][1,6]naphthyridine-8-one.

C. Preparation of Formula (IV) varying X and Y

Similarly, replacing 3,4-dihydroisoquinoline with the following compounds of formula (III):
8-methyl-3,4-dihydroisoquinoline;
7-methyl-3,4-dihydroisoquinoline;
6-methyl-3,4-dihydroisoquinoline;
6,7-dimethyl-3,4-dihydroisoquinoline;
6-ethyl-3,4-dihydroisoquinoline;
6-isobutyl-3,4-dihydroisoquinoline;
6-n-hexyl-3,4-dihydroisoquinoline;
8-methoxy-3,4-dihydroisoquinoline;
7-methoxy-3,4-dihydroisoquinoline;
5-methoxy-3,4-dihydroisoquinoline;
6-ethoxy-3,4-dihydroisoquinoline;
6-isobutoxy-3,4-dihydroisoquinoline;
6-n-hexyloxy-3,4-dihydroisoquinoline;
6-hydroxy-3,4-dihydroisoquinoline;
6,7-dihydroxy-3,4-dihydroisoquinoline;
7,8-dimethoxy-3,4-dihydroisoquinoline;
5,8-dimethoxy-3,4-dihydroisoquinoline;
5,6-dimethoxy-3,4-dihydroisoquinoline;
6,7-diethoxy-3,4-dihydroisoquinoline;
6,7-di-n-butoxy-3,4-dihydroisoquinoline;
7,8-methylenedioxy-3,4-dihydroisoquinoline;
5,6-methylenedioxy-3,4-dihydroisoquinoline;
8-chloro-3,4-dihydroisoquinoline;
7-chloro-3,4-dihydroisoquinoline;
6-chloro-3,4-dihydroisoquinoline;
5-chloro-3,4-dihydroisoquinoline;
6-bromo-3,4-dihydroisoquinoline;
6-fluoro-3,4-dihydroisoquinoline;
7-fluoro-3,4-dihydroisoquinoline;
and following the procedure of Preparation 1A above, the following exemplary compounds of formula (IV) are prepared:
(±)-1-methyl-5,6,13,13a-tetrahydroisoquino[2,1-g][1,6]naphthyridine-8-one hydrochloride;
(±)-2-methyl-5,6,13,13a-tetrahydroisoquino[2,1-g][1,6]naphthyridine-8-one hydrochloride;
(±)-3-methyl-5,6,13,13a-tetrahydroisoquino[2,1-g][1,6]naphthyridine-8-one hydrochloride;

(±)-2,3-dimethyl-5,6,13,13a-tetrahydroisoquino[2,1-g][1,6]naphthyridine-8-one hydrochloride;
(±)-3-ethyl-5,6,13,13a-tetrahydroisoquino[2,1-g][1,6]naphthyridine-8-one hydrochloride;
(±)-3-isobutyl-5,6,13,13a-tetrahydroisoquino[2,1-g][1,6]naphthyridine-8-one hydrochloride;
(±)-3-n-hexyl-5,6,13,13a-tetrahydroisoquino[2,1-g][1,6]naphthyridine-8-one hydrochloride;
(±)-1-methoxy-5,6,13,13a-tetrahydroisoquino[2,1-g][1,6]naphthyridine-8-one hydrochloride;
(±)-2-methoxy-5,6,13,13a-tetrahydroisoquino[2,1-g][1,6]naphthyridine-8-one hydrochloride;
(±)-4-methoxy-5,6,13,13a-tetrahydroisoquino[2,1-g][1,6]naphthyridine-8-one hydrochloride;
(±)-3-ethoxy-5,6,13,13a-tetrahydroisoquino[2,1-g][1,6]naphthyridine-8-one hydrochloride;
(±)-3-isobutoxy-5,6,13,13a-tetrahydroisoquino[2,1-g][1,6]naphthyridine-8-one hydrochloride;
(±)-3-n-hexyloxy-5,6,13,13a-tetrahydroisoquino[2,1-g][1,6]naphthyridine-8-one hydrochloride;
(±)-3-hydroxy-5,6,13,13a-tetrahydroisoquino[2,1-g][1,6]naphthyridine-8-one hydrochloride;
(±)-2,3-dihydroxy-5,6,13,13a-tetrahydroisoquino[2,1-g][1,6]naphthyridine-8-one hydrochloride;
(±)-1,2-dimethoxy-5,6,13,13a-tetrahydroisoquino[2,1-g][1,6]naphthyridine-8-one hydrochloride;
(±)-1,4-dimethoxy-5,6,13,13a-tetrahydroisoquino[2,1-g][1,6]naphthyridine-8-one hydrochloride;
(±)-3,4-dimethoxy-5,6,13,13a-tetrahydroisoquino[2,1-g][1,6]naphthyridine-8-one hydrochloride;
(±)-2,3-diethoxy-5,6,13,13a-tetrahydroisoquino[2,1-g][1,6]naphthyridine-8-one hydrochloride;
(±)-2,3-di-n-butoxy-5,6,13,13a-tetrahydroisoquino[2,1-g][1,6]naphthyridine-8-one hydrochloride;
(±)-1,2-methylenedioxy-5,6,13,13a-tetrahydroisoquino[2,1-g][1,6]naphthyridine-8-one hydrochloride;
(±)-3,4-methylenedioxy-5,6,13,13a-tetrahydroisoquino[2,1-g][1,6]naphthyridine-8-one hydrochloride;
(±)-1-chloro-5,6,13,13a-tetrahydroisoquino[2,1-g][1,6]naphthyridine-8-one hydrochloride;
(±)-2-chloro-5,6,13,13a-tetrahydroisoquino[2,1-g][1,6]naphthyridine-8-one hydrochloride;
(±)-3-chloro-5,6,13,13a-tetrahydroisoquino[2,1-g][1,6]naphthyridine-8-one hydrochloride;
(±)-4-chloro-5,6,13,13a-tetrahydroisoquino[2,1-g][1,6]naphthyridine-8-one hydrochloride;
(±)-3-bromo-5,6,13,13a-tetrahydroisoquino[2,1-g][1,6]naphthyridine-8-one hydrochloride;
(±)-3-fluoro-5,6,13,13a-tetrahydroisoquino[2,1-g][1,6]naphthyridine-8-one hydrochloride; and
(±)-2-fluoro-5,6,13,13a-tetrahydroisoquino[2,1-g][1,6]naphthyridine-8-one hydrochloride.

## PREPARATION 2

### Preparation of (±)-5,6,8aα,9,10,11,12,12aα,13,13aαdecahydroisoquino[2,1-g][1,6]naphthyridin-8-one (V) and (±)-5,6,8aβ,9,10,11,12,12aβ,13,13aα-decahydroisoquino[2,1-g][1,6]naphthyridin-8-one (VI) and Related Compounds of Formulae (V) and (VI)

A. Preparation of Formulae (V) and (VI) where X and Y are hydrogen

A mixture of 30 g of (±)-5,6,13,13a-tetrahydroisoquino[2,1-g][1,6]naphthyridine-8-one hydrochloride, prepared as shown in Preparation 1 above, and 7.5 g of 5% Rh-$Al_2O_3$ in 300 ml of acetic acid was hydrogenated at 50 psi for 42 hours. The catalyst was removed by filtration and the filtrate was concentrated under reduced pressure. The residue was partitioned between methylene chloride and aqueous $NH_4OH$ and the methylene chloride layer was separated and the solvent removed under reduced pressure. The residue was purified by silica gel chromatography, eluting with from 5-20% methanol in methylene chloride. The first component eluted was (±)-5,6,8aβ,9,10,11,12,12aβ,13,13aα-decahydroisoquino[2,1-g][1,6]naphthyridin-8-one (9.7 g), (VI), m.p. 105-106°C. The second component eluted was (±)-5,6,8aα,9, 10,11,12,12aα,13,13a-decahydroisoquino[2,1-g][1,6]naphthyridin-8-one (11.0 g), (V), m.p. 91-92°C.

B. Preparation of Formulae (V) and (VI) where X and Y are chosen from hydrogen, methoxy, methylenedioxy and ethylenedioxy

Similarly, replacing (±)-5,6,13,13a-tetrahydroisoquino[2,1-g][1,6]naphthyridine-8-one hydrochloride with other compounds of formula (IV), obtained, for example, as described in Preparation 1B, and following the procedure of Preparation 2A above, the following corresponding compounds of formulae (V) and (VI) were prepared:

(±)-3-methoxy-5,6,8aβ,9,10,11,12,12aβ,13,13aα-decahydroisoquino[2,1-g][1,6]naphthyridin-8-one; and
(±)-3-methoxy-5,6,8aα,9,10,11,12,12aα,13,13aα-decahydroisoquino[2,1-g][1,6]naphthyridin-8-one, m.p. 118-119°C;
(±)-2,3-dimethoxy-5,6,8aβ,9,10,11,12,12aβ,13, 13aα-decahydroisoquino[2,1-g][1,6]naphthyridin-8-one; and
(±)-2,3-dimethoxy-5,6,8aα,9,10,11,12,12aα,13, 13aα-decahydroisoquino[2,1-g][1,6]naphthyridin-8-one;

(±)-1,4-dimethoxy-5,6,8aβ,9,10,11,12,12aβ,13, 13aα-decahydroisoquino[2,1-g][1,6]naphthyridin-8-one; and

(±)-1,4-dimethoxy-5,6,8aα,9,10,11,12,12aα,13, 13aα-decahydroisoquino[2,1-g][1,6]naphthyridin-8-one;

(±)-2,3-methylenedioxy-5,6,8aβ,9,10,11,12,12aβ, 13,13aα-decahydroisoquino[2,1-g][1,6]naphthyridin-8-one; and

(±)-2,3-methylenedioxy-5,6,8aα,9,10,11,12,12aα, 13,13aα-decahydroisoquino[2,1-g][1,6]naphthyridin-8-one;

(±)-2,3-(ethylene-1,2-dioxy)-5,6,8aß,9,10,11,12, 12aß,13,13aα-decahydroisoquino[2,1-g][1,6]naphthyridin-8 -one; and

(±)-2,3-(ethylene-1,2-dioxy)-5,6,8aβ,9,10,11,12, 12aα,13,13aα-decahydroisoquino[2,1-g][1,6]naphthyridin-8-one.

C. Preparation of Formulae (V) and (VI), varying X and Y

Similarly, replacing (±)-5,6,13,13a-tetrahydroisoquino[2,1-g][1,6]naphthyridine-8-one hydrochloride with other compounds of formula (IV), obtained, for example, as described in Preparation 1C, and following the procedure of Preparation 2A above, the following exemplary compounds of formulae (V) and (VI) are prepared:

(±)-1-methyl-5,6,8aβ,9,10,11,12,12aβ,13,13aα-decahydroisoquino[2,1-g][1,6]naphthyridin-8-one; and
(±)-1-methyl-5,6,8aα,9,10,11,12,12aα,13,13aα-decahydroisoquino[2,1-g][1,6]naphthyridin-8-one;
(±)-2-methyl-5,6,8aβ,9,10,11,12,12aβ,13,13aα-decahydroisoquino[2,1-g][1,6]naphthyridin-8-one; and
(±)-2-methyl-5,6,8aα,9,10,11,12,12aα,13,13aα-decahydroisoquino[2,1-g][1,6]naphthyridin-8-one;
(±)-3-methyl-5,6,8aβ,9,10,11,12,12aβ,13,13aα-decahydroisoquino[2,1-g][1,6]naphthyridin-8-one; and
(±)-3-methyl-5,6,8aα,9,10,11,12,12aα,13,13aα-decahydroisoquino[2,1-g][1,6]naphthyridin-8-one;
(±)-2,3-dimethyl-5,6,8aβ,9,10,11,12,12aβ,13,13aα-decahydroisoquino[2,1-g][1,6]naphthyridin-8-one; and
(±)-2,3-dimethyl-5,6,8aα,9,10,11,12,12aα,13,13aα-decahydroisoquino[2,1-g][1,6]naphthyridin-8-one;
(±)-3-ethyl-5,6,8aβ,9,10,11,12,12aβ,13,13aα-decahydroisoquino[2,1-g][1,6]naphthyridin-8-one; and
(±)-3-ethyl-5,6,8aα,9,10,11,12,12aα,13,13aα-decahydroisoquino[2,1-g][1,6]naphthyridin-8-one;
(±)-3-isobutyl-5,6,8aβ,9,10,11,12,12aβ,13,13aα-decahydroisoquino[2,1-g][1,6]naphthyridin-8-one; and
(±)-3-isobutyl-5,6,8aα,9,10,11,12,12aα,13,13aα-decahydroisoquino[2,1-g][1,6]naphthyridin-8-one;
(±)-3-n-hexyl-5,6,8aβ,9,10,11,12,12aβ,13,13aα-decahydroisoquino[2,1-g][1,6]naphthyridin-8-one; and
(±)-3-n-hexyl-5,6,8aα,9,10,11,12,12aα,13,13aα-decahydroisoquino[2,1-g][1,6]naphthyridin-8-one;
(±)-1-methoxy-5,6,8aβ,9,10,11,12,12aβ,13,13aα-decahydroisoquino[2,1-g][1,6]naphthyridin-8-one; and
(±)-1-methoxy-5,6,8aα,9,10,11,12,12aα,13,13aα-decahydroisoquino[2,1-g][1,6]naphthyridin-8-one;
(±)-2-methoxy-5,6,8aβ,9,10,11,12,12aβ,13,13aα-decahydroisoquino[2,1-g][1,6]naphthyridin-8-one; and
(±)-2-methoxy-5,6,8aα,9,10,11,12,12aα,13,13aα-decahydroisoquino[2,1-g][1,6]naphthyridin-8-one;
(±)-4-methoxy-5,6,8aβ,9,10,11,12,12aβ,13,13aα-decahydroisoquino[2,1-g][1,6]naphthyridin-8-one; and
(±)-4-methoxy-5,6,8aα,9,10,11,12,12aα,13,13aα-decahydroisoquino[2,1-g][1,6]naphthyridin-8-one;
(±)-3-ethoxy-5,6,8aβ,9,10,11,12,12aβ,13,13aα-decahydroisoquino[2,1-g][1,6]naphthyridin-8-one; and
(±)-3-ethoxy-5,6,8aα,9,10,11,12,12aα,13,13aα-decahydroisoquino[2,1-g][1,6]naphthyridin-8-one;
(±)-3-isobutoxy-5,6,8aβ,9,10,11,12,12aβ,13,13aα-decahydroisoquino[2,1-g][1,6]naphthyridin-8-one; and
(±)-3-isobutoxy-5,6,8aα,9,10,11,12,12aα,13,13aα-decahydroisoquino[2,1-g][1,6]naphthyridin-8-one;
(±)-3-n-hexyloxy-5,6,8aβ,9,10,11,12,12aβ,13,13aα-decahydroisoquino[2,1-g][1,6]naphthyridin-8-one; and
(±)-3-n-hexyloxy-5,6,8aα,9,10,11,12,12aα,13,13aα-decahydroisoquino[2,1-g][1,6]naphthyridin-8-one;
(±)-3-hydroxy-5,6,8aβ,9,10,11,12,12aβ,13,13aα-decahydroisoquino[2,1-g][1,6]naphthyridin-8-one; and
(±)-3-hydroxy-5,6,8aα,9,10,11,12,12aα,13,13aα-decahydroisoquino[2,1-g][1,6]naphthyridin-8-one;
(±)-2,3-dihydroxy-5,6,8aβ,9,10,11,12,12aβ,13,13aα-decahydroisoquino[2,1-g][1,6]naphthyridin-8-one; and
(±)-2,3-dihydroxy-5,6,8aα,9,10,11,12,12aα,13,13aα-decahydroisoquino[2,1-g][1,6]naphthyridin-8-one;
(±)-1,2-dimethoxy-5,6,8aβ,9,10,11,12,12aβ,13,13aα-decahydroisoquino[2,1-g][1,6]naphthyridin-8-one; and
(±)-1,2-dimethoxy-5,6,8aα,9,10,11,12,12aα,13,13aα-decahydroisoquino[2,1-g][1,6]naphthyridin-8-one;
(±)-1,4-dimethoxy-5,6,8aβ,9,10,11,12,12aβ,13,13aα-decahydroisoquino[2,1-g][1,6]naphthyridin-8-one; and
(±)-1,4-dimethoxy-5,6,8aα,9,10,11,12,12aα,13,13aα-decahydroisoquino[2,1-g][1,6]naphthyridin-8-one;
(±)-3,4-dimethoxy-5,6,8aβ,9,10,11,12,12aβ,13,13aα-decahydroisoquino[2,1-g][1,6]naphthyridin-8-one; and
(±)-3,4-dimethoxy-5,6,8aα,9,10,11,12,12aα,13,13aα-decahydroisoquino[2,1-g][1,6]naphthyridin-8-one;
(±)-2,3-diethoxy-5,6,8aβ,9,10,11,12,12aβ,13,13aα-decahydroisoquino[2,1-g][1,6]naphthyridin-8-one; and
(±)-2,3-diethoxy-5,6,8aα,9,10,11,12,12aα,13,13aα-decahydroisoquino[2,1-g][1,6]naphthyridin-8-one;
(±)-2,3-di-n-butoxy-5,6,8aβ,9,10,11,12,12aβ,13,13aα-decahydroisoquino[2,1-g][1,6]naphthyridin-8-one;

and

(±)-1,2-methylenedioxy-5,6,8aβ,9,10,11,12,12aβ,13,13aα-decahydroisoquino[2,1-g][1,6]naphthyridin-8-one;

and

(±)-1,2-methylenedioxy-5,6,8aα,9,10,11,12,12aα,13,13aα-decahydroisoquino[2,1-g][1,6]naphthyridin-8-one;
(±)-2-chloro-5,6,8aβ,9,10,11,12,12aβ,13,13aα-decahydroisoquino[2,1-g][1,6]naphthyridin-8-one; and
(±)-2-chloro-5,6,8aα,9,10,11,12,12aα,13,13aα-decahydroisoquino[2,1-g][1,6]naphthyridin-8-one;
(±)-3-chloro-5,6,8aβ,9,10,11,12,12aβ,13,13aα-decahydroisoquino[2,1-g][1,6]naphthyridin-8-one; and
(±)-3-chloro-5,6,8aα,9,10,11,12,12aα,13,13aα-decahydroisoquino[2,1-g][1,6]naphthyridin-8-one;
(±)-4-chloro-5,6,8aβ,9,10,11,12,12aβ,13,13aα-decahydroisoquino[2,1-g][1,6]naphthyridin-8-one; and
(±)-4-chloro-5,6,8aα,9,10,11,12,12aα,13,13aα-decahydroisoquino[2,1-g][1,6]naphthyridin-8-one;
(±)-3-bromo-5,6,8aβ,9,10,11,12,12aβ,13,13aα-decahydroisoquino[2,1-g][1,6]naphthyridin-8-one; and
(±)-3-bromo-5,6,8aα,9,10,11,12,12aα,13,13aα-decahydroisoquino[2,1-g][1,6]naphthyridin-8-one;
(±)-3-fluoro-5,6,8aβ,9,10,11,12,12aβ,13,13aα-decahydroisoquino[2,1-g][1,6]naphthyridin-8-one; and
(±)-3-fluoro-5,6,8aα,9,10,11,12,12aα,13,13aα-decahydroisoquino[2,1-g][1,6]naphthyridin-8-one;
(±)-2-fluoro-5,6,8aβ,9,10,11,12,12aβ,13,13aα-decahydroisoquino[2,1-g][1,6]naphthyridin-8-one; and
(±)-2-fluoro-5,6,8aα,9,10,11,12,12aα,13,13aα-decahydroisoquino[2,1-g][1,6]naphthyridin-8-one.

## PREPARATION 3

### Preparation of (±)-5,6,8aα,9,10,11,12,12aα,13, 13aα-Decahydro-8H-isoquino[2,1g][1,6]naphthyridine and Related Compounds of formula (VII)

A. Preparation of Formula (VII) where X and Y are hydrogen

A solution of 9.6 g of (±)-5,6,8aα,9,10,11,12,12aα, 13,13aα-decahydroisoquino[2,1-g][1,6]naphthyridin-8-one (V), prepared as shown in Preparation 2, in 50 ml of tetrahydrofuran was added slowly to a solution of 2.5 g of lithium aluminum hydride in 75 ml of tetrahydrofuran. The resulting mixture was stirred at reflux for 3 hours, cooled, and treated sequentially with 2.5 ml of water, 2.5 ml of 15% sodium hydroxide, and 7.5 ml of water. The mixture was filtered and the filtrate was evaporated to afford 8.8 g of (±)-5,6,8aα,9,10,11,12,12aα,13,13aα-decahydro-8H-isoquino[2,1-g][1,6]naphthyridine (VII) as a thick oil. The oil was dissolved in ethanol and acidified with anhydrous HCl in ether, from which a dihydrochloride salt was crystallized, m.p. 290-295°C.

B. Preparation of Formula (VII) where X and Y are chosen from hydrogen, methoxy, methylenedioxy and ethylenedioxy

Similarly, replacing (±)-5,6,8aα,9,10,11,12,12aα, 13,13aα-decahydroisoquino[2,1-g][1,6]naphthyridin-8-one with compounds of formula (V), obtained, for example, as described in Preparation 2B, and following the procedure of Preparation 3A above, the following corresponding compounds of formula (VII) were prepared:

(±)-3-methoxy-5,6,8aα,9,10,11,12,12aα,13,13aα-decahydro-8H-isoquino[2,1-g][1,6]naphthyridine;
(±)-2,3-dimethoxy-5,6,8aα,9,10,11,12,12aα,13,13aα-decahydro-8H-isoquino[2,1-g][1,6]naphthyridine;
(±)-1,4-dimethoxy-5,6,8aα,9,10,11,12,12aα,13,13aα-decahydro-8H-isoquino[2,1-g][1,6]naphthyridine;
(±)-2,3-methylenedioxy-5,6,8aα,9,10,11,12,12aα,13,13aα-decahydro-8H-isoquino[2,1-g][1,6]naphthyridine; and
(±)-2,3-(ethylene-1,2-dioxy)-5,6,8aα,9,10,11,12,12aα,13,13aα-decahydro-8H-isoquino[2,1-g][1,6]naphthyridine.

C. Preparation of Formula (VII), varying X and Y

Similarly, replacing (±)-5,6,8aα,9,10,11,12,12aα, 13,13aα-decahydroisoquino[2,1-g][1,6]naphthyridin-8-one with other compounds of formula (V), obtained, for example, as described in Preparation 2C, and following the procedure of Preparation 3A above, the following exemplary compounds of formula (VII) are prepared:

(±)-1-methyl-5,6,8aα,9,10,11,12,12aα,13,13aα-decahydro-8H-isoquino[2,1-g][1,6]naphthyridine;
(±)-2-methyl-5,6,8aα,9,10,11,12,12aα,13,13aα-decahydro-8H-isoquino[2,1-g][1,6]naphthyridine;
(±)-3-methyl-5,6,8aα,9,10,11,12,12aα,13,13aα-decahydro-8H-isoquino[2,1-g][1,6]naphthyridine;
(±)-2,3-dimethyl-5,6,8aα,9,10,11,12,12aα,13,13aα-decahydro-8H-isoquino[2,1-g][1,6]naphthyridine;
(±)-3-ethyl-5,6,8aα,9,10,11,12,12aα,13,13aα-decahydro-8H-isoquino[2,1-g][1,6]naphthyridine;
(±)-3-isobutyl-5,6,8aα,9,10,11,12,12aα,13,13aα-decahydro-8H-isoquino[2,1-g][1,6]naphthyridine;
(±)-3-n-hexyl-5,6,8aα,9,10,11,12,12aα,13,13aα-decahydro-8H-isoquino[2,1-g][1,6]naphthyridine;

(±)-1-methoxy-5,6,8aα,9,10,11,12,12aα,13,13aα-decahydro-8H-isoquino[2,1-g][1,6]naphthyridine;
(±)-2-methoxy-5,6,8aα,9,10,11,12,12aα,13,13aα-decahydro-8H-isoquino[2,1-g][1,6]naphthyridine;
(±)-4-methoxy-5,6,8aα,9,10,11,12,12aα,13,13aα-decahydro-8H-isoquino[2,1-g][1,6]naphthyridine;
(±)-3-ethoxy-5,6,8aα,9,10,11,12,12aα,13,13aα-decahydro-8H-isoquino[2,1-g][1,6]naphthyridine;
(±)-3-isobutoxy-5,6,8aα,9,10,11,12,12aα,13,13aα-decahydro-8H-isoquino[2,1-g][1,6]naphthyridine;
(±)-3-n-hexyloxy-5,6,8aα,9,10,11,12,12aα,13,13aα-decahydro-8H-isoquino[2,1-g][1,6]naphthyridine;
(±)-3-hydroxy-5,6,8aα,9,10,11,12,12aα,13,13aα-decahydro-8H-isoquino[2,1-g][1,6]naphthyridine;
(±)-2,3-dihydroxy-5,6,8aα,9,10,11,12,12aα,13,13aα-decahydro-8H-isoquino[2,1-g][1,6]naphthyridine;
(±)-1,2-dimethoxy-5,6,8aα,9,10,11,12,12aα,13,13aα-decahydro-8H-isoquino[2,1-g][1,6]naphthyridine;
(±)-1,4-dimethoxy-5,6,8aα,9,10,11,12,12aα,13,13aα-decahydro-8H-isoquino[2,1-g][1,6]naphthyridine;
(±)-3,4-dimethoxy-5,6,8aα,9,10,11,12,12aα,13,13aα-decahydro-8H-isoquino[2,1-g][1,6]naphthyridine;
(±)-2,3-diethoxy-5,6,8aα,9,10,11,12,12aα,13,13aα-decahydro-8H-isoquino[2,1-g][1,6]naphthyridine;
(±)-2,3-di-n-butoxy-5,6,8aα,9,10,11,12,12aα,13,13aα-decahydro-8H-isoquino[2,1-g][1,6]naphthyridine;
(±)-1,2-methylenedioxy-5,6,8aα,9,10,11,12,12aα,13,13aα-decahydro-8H-isoquino[2,1-g][1,6]naphthyridine;
(±)-2-chloro-5,6,8aα,9,10,11,12,12aα,13,13aα-decahydro-8H-isoquino[2,1-g][1,6]naphthyridine;
(±)-3-chloro-5,6,8aα,9,10,11,12,12aα,13,13aα-decahydro-8H-isoquino[2,1-g][1,6]naphthyridine;
(±)-4-chloro-5,6,8aα,9,10,11,12,12aα,13,13aα-decahydro-8H-isoquino[2,1-g][1,6]naphthyridine;
(±)-3-bromo-5,6,8aα,9,10,11,12,12aα,13,13aα-decahydro-8H-isoquino[2,1-g][1,6]naphthyridine;
(±)-3-fluoro-5,6,8aα,9,10,11,12,12aα,13,13aα-decahydro-8H-isoquino[2,1-g][1,6]naphthyridine; and
(±)-2-fluoro-5,6,8aα,9,10,11,12,12aα,13,13aα-decahydro-8H-isoquino[2,1-g][1,6]naphthyridine.

## PREPARATION 4

### Preparation of (8aR,12aS,13aS)-3-methoxy-5,6,8a,9,10,11,12,12a,13,13a-decahydro-8H-isoquino[2,1-g][1,6]naphthyridine and (8aS,12aR,13aR)-3-methoxy-5,6,8a,9,10,11,12,12a,13,13a-decahydro-8H-isoquino[2,1-g][1,6]naphthyridine and Related Compounds of Formula (VIIA) and (VIIB)

### Preparation of Formulae (VIIA) and (VIIB) where X is 3-methoxy and Y is hydrogen

A. A solution of 1.95 g of (±)-3-methoxy-5,6,8aα,9,10,11, 12,12aα,13,13aα-decahydroisoquino[2,1-g][1,6]naphthyridin-8-one, a compound of formula (V), and 1.0 g of (R)-(+)-α-methylbenzyl isocyanate in 50 ml of methylene chloride was stirred at room temperature for 30 minutes. Solvent was then removed under reduced pressure, and the residue chromatographed on silica gel, using multiple medium pressure chromatography and eluting with 5% methanol in ethyl acetate.The first compound eluted was (8aS,12aS,13aS)-3-methoxy-12-(1-R-phenethylaminocarbonyl)-5,6,8a,9,10,11,12, 12a,13,13a-decahydroisoquino[2,1-g][1,6]naphthyridin-8-one, mp 198-199°C, $[\alpha]_D^{25}$= +36.5 ($CHCl_3$)

followed by (8aR,12aR,13aR)-3-methoxy-12-(1-R-phenethylaminocarbonyl)-5,6,8a,9,10,11,12,12a,13,13a-decahydroisoquino[2,1-g][1,6]naphthyridin-8-one,
mp 220-221°C, $[\alpha]_D^{25}$= -11.4 ($CHCl_3$).

B. A solution of 11.5 g of (8aS,12aS,13aS)-3-methoxy-12-(1-R-phenethylaminocarbonyl)-5,6,8a,9,10,11,12, 12a,13,13a-decahydroisoquino[2,1-g][1,6]naphthyridin-8-one, in 50 ml of tetrahydrofuran was added slowly to a solution of 2.0 g of lithium aluminum hydride in 75 ml of tetrahydrofuran. The resulting mixture was stirred at reflux for 2 hours, cooled, and treated sequentially with 2.5 ml of water, 2.5 ml of 15% sodium hydroxide, and 7.5 ml of water. The mixture was filtered and the filtrate was evaporated to afford 8.8 g of (8aR,12aS,13aS)-3-methoxy-12-(1-R-phenethylaminocarbonyl)-5,6,8a,9,10,11,12,12a,13,13a-decahydro-8H-isoquino[2,1-g][1,6]naphthyridine, as a foam. The foam was used as such in the next reaction with no further purification.

C. A solution of 10.5 g of (8aR,12aS,13aS)-3-methoxy-12-(1-R-phenethylaminocarbonyl)-5,6,8a,9,10,11,12, 12a,13, 13a-decahydro-8H-isoquino[2,1-g][1,6]naphthyridine in 125 ml of 2N sodium n-butoxide in n-butanol was refluxed for 4 hours. After cooling, water was added and the solution acidified with 2N hydrochloric acid. The solution was then washed with ethyl acetate, the aqueous portion basified with aqueous ammonium hydroxide and extracted further with methylene chloride. Solvent was then removed from the extract under reduced pressure and the residue chromatographed on silica gel, eluting with 10-20% methanol in methylene chloride, to give (8aR,12aS,13aS)-3-methoxy-5,6,8a,9,10,11,12,12a,13,13a-decahydro-8H-isoquino [2,1-g][1,6]naphthyridine, a compound of formula (VIIA), mp 125-127°C, $[\alpha]_D^{25}$= -150.7 ($CHCl_3$)

D. Preparation of Formulae (VIIA) and (VIIB) where X and Y are chosen from hydrogen, methoxy and methylenedioxy

Similarly, replacing (±)-3-methoxy-5,6,8aα,9,10,11, 12,12aα,13,13aα-decahydroisoquino[2,1-g][1,6] naphthyridin-8 -one with compounds of formula (V), prepared, for example, as shown in Preparation 2B, and following the procedures of Preparation 4A, 4B and 4C above, the following compounds of formula (VIIA) and (VIIB) were prepared:

(8aS,12aR,13aR)-3-methoxy-5,6,8a,9,10,11,12,12a,13, 13a-decahydro-8H-isoquino[2,1-g][1,6]naphthyridine, mp 125-127°C, $[\alpha]_D^{25}$= +154.5 ($CHCl_3$).

(8aR,12aS,13aS)-5,6,8a,9,10,11,12,12a,13,13a-decahydro -8H-isoquino[2,1-g][1,6]naphthyridine;

(8aS,12aR,13aR)-5,6,8a,9,10,11,12,12a,13,13a-decahydro -8H-isoquino[2,1-g][1,6]naphthyridine;

(8aR,12aS,13aS)-2,3-methylenedioxy-5,6,8a,9,10,11, 12,12a,13,13a-decahydro-8H-isoquino[2,1-g][1, 6]naphthyridine; and

(8aS,12aR,13aR)-2,3-methylenedioxy-5,6,8a,9,10,11, 12,12a,13,13a-decahydro-8H-isoquino[2,1-g][1, 6]naphthyridine.

E. Preparation of Formulae (VIIA) and (VIIB), varying X and Y

Similarly, replacing (±)-3-methoxy-5,6,8aα,9,10,11,12,12aα,13,13aα-decahydroisoquino[2,1-g][1,6]-naphthyridin-8-one with other compounds of formula (V), prepared, for example, as shown in Preparation 2B, and following the procedures of Preparation 4A, 4B and 4C above, the following exemplary compounds of formula (VIIA) and (VIIB) are prepared:

(8aR,12aS,13aS)-2,3-dimethoxy-5,6,8a,9,10,11,12,12a,13,13a-decahydro-8H-isoquino[2,1-g][1,6]naphthyridine;

(8aS,12aR,13aR)-2,3-dimethoxy-5,6,8a,9,10,11,12,12a,13,13a-decahydro-8H-isoquino[2,1-g][1,6]naphthyridine;

(8aR,12aS,13aS)-1,4-dimethoxy-5,6,8a,9,10,11,12,12a,13,13a-decahydro-8H-isoquino[2,1-g][1,6]naphthyridine;

(8aS,12aR,13aR)-1,4-dimethoxy-5,6,8a,9,10,11,12,12a,13,13a-decahydro-8H-isoquino[2,1-g][1,6]naphthyridine;

(8aR,12aS,13aS)-2,3-(ethylene-1,2-dioxy)-5,6,8a,9,10,11,12,12a,13,13a-decahydro-8H-isoquino[2,1-g][1,6]naphthyridine;

(8aS,12aR,13aR)-2,3-(ethylene-1,2-dioxy)-5,6,8a,9,10,11,12,12a,13,13a-decahydro-8H-isoquino[2,1-g][1,6]naphthyridine;

(8aR,12aS,13aS)-1-methyl-5,6,8a,9,10,11,12,12a,13,13a-decahydro-8H-isoquino[2,1-g][1,6]naphthyridine;

(8aS,12aR,13aR)-1-methyl-5,6,8a,9,10,11,12,12a,13,13a-decahydro-8H-isoquino[2,1-g][1,6]naphthyridine;

(8aR,12aS,13aS)-2-methyl-5,6,8a,9,10,11,12,12a,13,13a-decahydro-8H-isoquino[2,1-g][1,6]naphthyridine;

(8aS,12aR,13aR)-2-methyl-5,6,8a,9,10,11,12,12a,13,13a-decahydro-8H-isoquino[2,1-g][1,6]naphthyridine;

(8aR,12aS,13aS)-3-methyl-5,6,8a,9,10,11,12,12a,13,13a-decahydro-8H-isoquino[2,1-g][1,6]naphthyridine;

(8aS,12aR,13aR)-3-methyl-5,6,8a,9,10,11,12,12a,13,13a-decahydro-8H-isoquino[2,1-g][1,6]naphthyridine;

(8aR,12aS,13aS)-2,3-dimethyl-5,6,8a,9,10,11,12,12a,13,13a-decahydro-8H-isoquino[2,1-g][1,6]naphthyridine;

(8aS,12aR,13aR)-2,3-dimethyl-5,6,8a,9,10,11,12,12a,13,13a-decahydro-8H-isoquino[2,1-g][1,6]naphthyridine;

(8aR,12aS,13aS)-3-ethyl-5,6,8a,9,10,11,12,12a,13,13a-decahydro-8H-isoquino-[2,1-g][1,6]naphthyridine;

(8aS,12aR,13aR)-3-ethyl-5,6,8a,9,10,11,12,12a,13,13a-decahydro-8H-isoquino[2,1-g][1,6]naphthyridine;

(8aR,12aS,13aS)-3-isobutyl-5,6,8a,9,10,11,12,12a,13,13a-decahydro-8H-isoquino[2,1-g][1,6]naphthyridine;

(8aS,12aR,13aR)-3-isobutyl-5,6,8a,9,10,11,12,12a,13,13a-decahydro-8H-isoquino[2,1-g][1,6]naphthyridine;

(8aR,12aS,13aS)-3-n-hexyl-5,6,8a,9,10,11,12,12a,13,13a-decahydro-8H-isoquino[2,1-g][1,6]naphthyridine;

(8aS,12aR,13aR)-3-n-hexyl-5,6,8a,9,10,11,12,12a,13,13a-decahydro-8H-isoquino[2,1-g][1,6]naphthyridine;

(8aR,12aS,13aS)-1-methoxy-5,6,8a,9,10,11,12,12a,13,13a-decahydro-8H-isoquino[2,1-g][1,6]naphthyridine;

(8aS,12aR,13aR)-1-methoxy-5,6,8a,9,10,11,12,12a,13,13a-decahydro-8H-isoquino[2,1-g][1,6]naphthyridine;

(8aR,12aS,13aS)-2-methoxy-5,6,8a,9,10,11,12,12a,13,13a-decahydro-8H-isoquino[2,1-g][1,6]naphthyridine;

(8aS,12aR,13aR)-2-methoxy-5,6,8a,9,10,11,12,12a,13,13a-decahydro-8H-isoquino[2,1-g][1,6]naphthyridine;

(8aR,12aS,13aS)-4-methoxy-5,6,8a,9,10,11,12,12a,13,13a-decahydro-8H-isoquino[2,1-g][1,6]naphthyridine;

(8aS,12aR,13aR)-4-methoxy-5,6,8a,9,10,11,12,12a,13, 13a-decahydro-8H-isoquino[2,1-g][1,6]naphthyridine;

(8aR,12aS,13aS)-3-methoxy-2-methyl-5,6,8a,9,10,11,12,12a,13,13a-decahydro-8H-isoquino[2,1-g][1,6] naphthyridine;

(8aS,12aR,13aR)-3-methoxy-2-methyl-5,6,8a,9,10,11,12,12a,13,13a-decahydro-8H-isoquino[2,1-g][1,6] naphthyridine;

(8aR,12aS,13aS)-3-ethoxy-5,6,8a,9,10,11,12,12a,13,13a-decahydro -8H-isoquino[2,1-g][1,6]naphthyridine;

(8aS,12aR,13aR)-3-ethoxy-5,6,8a,9,10,11,12,12a,13,13a-decahydro-8H-isoquino[2,1-g][1,6]naphthyridine;

(8aR,12aS,13aS)-3-isopropoxy-5,6,8a,9,10,11,12,12a,13,13a-decahydro-8H-isoquino[2,1-g][1,6]naphthyridine;

(8aS,12aR,13aR)-3-isobutoxy-5,6,8a,9,10,11,12,12a,13,13a-decahydro-8H-isoquino[2,1-g][1,6]naphthyridine;

(8aR,12aS,13aS)-3-isopropoxy-5,6,8a,9,10,11,12,12a,13,13a-decahydro-8H-isoquino[2,1-g][1,6]naphthyridine;

(8aS,12aR,13aR)-3-isobutoxy-5,6,8a,9,10,11,12,12a,13,13a-decahydro-8H-isoquino[2,1-g][1,6]naphthyridine;

(8aR,12aS,13aS)-3-n-hexyloxy-5,6,8a,9,10,11,12,12a,13,13a-decahydro-8H-isoquino[2,1-g][1,6]naphthyridine;

(8aS,12aR,13aR)-3-n-hexyloxy-5,6,8a,9,10,11,12,12a,13,13a-decahydro-8H-isoquino[2,1-g][1,6]naphthyridine;

(8aR,12aS,13aS)-3-hydroxy-5,6,8a,9,10,11,12,12a,13,13a-decahydro-8H-isoquino[2,1-g][1,6]naphthyridine;

(8aS,12aR,13aR)-3-hydroxy-5,6,8a,9,10,11,12,12a,13,13a-decahydro-8H-isoquino[2,1-g][1,6]naphthyridine;

(8aR,12aS,13aS)-2,3-dihydroxy-5,6,8a,9,10,11,12,12a,13,13a-decahydro-8H-isoquino[2,1-g][1,6]naphthyridine;

(8aS,12aR,13aR)-2,3-dihydroxy-5,6,8a,9,10,11,12,12a,13,13a-decahydro-8H-isoquino[2,1-g][1,6]naphthyridine;

(8aR,12aS,13aS)-1,2-dimethoxy-5,6,8a,9,10,11,12,12a,13,13a-decahydro-8H-isoquino[2,1-g][1,6]naphthyridine;

(8aS,12aR,13aR)-1,2-dimethoxy-5,6,8a,9,10,11,12,12a,13,13a-decahydro-8H-isoquino[2,1-g][1,6]naphthyridine;

(8aR,12aS,13aS)-1,4-dimethoxy-5,6,8a,9,10,11,12,12a,13,13a-decahydro-8H-isoquino[2,1-g][1,6]naphthyridine;

(8aS,12aR,13aR)-1,4-dimethoxy-5,6,8a,9,10,11,12,12a,13,13a-decahydro-8H-isoquino[2,1-g][1,6]naphthyridine;

(8aR,12aS,13aS)-3,4-dimethoxy-5,6,8a,9,10,11,12,12a,13,13a-decahydro-8H-isoquino[2,1-g][1,6]naphthyridine;

(8aS,12aR,13aR)-3,4-dimethoxy-5,6,8a,9,10,11,12,12a,13,13a-decahydro-8H-isoquino[2,1-g][1,6]naphthyridine;

(8aR,12aS,13aS)-2,3-diethoxy-5,6,8a,9,10,11,12,12a,13,13a-decahydro-8H-isoquino[2,1-g][1,6]naphthyridine;

(8aS,12aR,13aR)-2,3-diethoxy-5,6,8a,9,10,11,12,12a,13,13a-decahydro-8H-isoquino[2,1-g][1,6]naphthyridine;

(8aR,12aS,13aS)-2,3-di-n-butoxy-5,6,8a,9,10,11,12,12a,13,13a-decahydro-8H-isoquino[2,1-g][1,6]naphthyridine;

(8aS,12aR,13aR)-2,3-di-n-butoxy-5,6,8a,9,10,11,12,12a,13,13a-decahydro-8H-isoquino[2,1-g][1,6]naphthyridine;

(8aR,12aS,13aS)-1,2-methylenedioxy-5,6,8a,9,10,11, 12,12a,13,13a-decahydro-8H-isoquino[2,1-g][1,6]naphthyridine;

(8aS,12aR,13aR)-1,2-methylenedioxy-5,6,8a,9,10,11,12,12a,13,13a-decahydro-8H-isoquino[2,1-g][1,6] naphthyridine;

(8aR,12aS,13aS)-2-chloro-5,6,8a,9,10,11,12,12a,13,13a-decahydro-8H-isoquino[2,1-g][1,6]naphthyridine;

(8aS,12aR,13aR)-2-chloro-5,6,8a,9,10,11,12,12a,13,13a-decahydro-8H-isoquino[2,1-g][1,6]naphthyridine;

(8aR,12aS,13aS)-3-chloro-5,6,8a,9,10,11,12,12a,13,13a-decahydro-8H-isoquino[2,1-g][1,6]naphthyridine;

(8aS,12aR,13aR)-3-chloro-5,6,8a,9,10,11,12,12a,13,13a-decahydro-8H-isoquino[2,1-g][1,6]naphthyridine;

(8aR,12aS,13aS)-4-chloro-5,6,8a,9,10,11,12,12a,13,13a-decahydro-8H-isoquino[2,1-g][1,6]naphthyridine;

(8aS,12aR,13aR)-4-chloro-5,6,8a,9,10,11,12,12a,13,13a-decahydro-8H-isoquino[2,1-g][1,6]naphthyridine;

(8aR,12aS,13aS)-3-bromo-5,6,8a,9,10,11,12,12a,13,13a-decahydro-8H-isoquino[2,1-g][1,6]naphthyridine;

(8aS,12aR,13aR)-3-bromo-5,6,8a,9,10,11,12,12a,13,13a-decahydro-8H-isoquino[2,1-g][1,6]naphthyridine;

(8aR,12aS,13aS)-3-fluoro-5,6,8a,9,10,11,12,12a,13,13a-decahydro-8H-isoquino[2,1-g][1,6]naphthyridine;

(8aS,12aR,13aR)-3-fluoro-5,6,8a,9,10,11,12,12a,13,13a-decahydro-8H-isoquino[2,1-g][1,6]naphthyridine;

(8aR,12aS,13aS)-2-fluoro-5,6,8a,9,10,11,12,12a,13,13a-decahydro-8H-isoquino[2,1-g][1,6]naphthyridine;

and

(8aS,12aR,13aR)-2-fluoro-5,6,8a,9,10,11,12,12a,13,13a-decahydro-8H-isoquino[2,1-g][1,6]naphthyridine.

PREPARATION 5

Preparation of 2-methylpyrrole-3-carboxylic acid diethylamide

To a solution of 1.6 ml of diethylamine in 30 ml of toluene was added 7.5 ml of 2M trimethylaluminum, and the mixture stirred for 20 minutes. To this mixture was added 1.53 g of 2-methylpyrrole-3-carboxylic acid ethyl ester, and the reaction mixture refluxed overnight. The mixture was cooled, acidified with aqueous 2N hydrochloric acid and extracted twice with ethyl acetate and three times with methylene chloride. Solvent was removed from the combined extracts under reduced pressure, and the residue was flash chromatographed on silica gel, eluting with 75% ethyl acetate/hexane, giving 1.5 g of 2-methylpyrrole-3-carboxylic acid diethylamide, m.p. 120-121°C.

PREPARATION 6

Preparation of 1-tert-butoxycarbonyl-2-methylpyrrole-3-carboxylic acid diethylamide

To a solution of 3.6 g of 2-methylpyrrole-3-carboxylic acid diethylamide in 25 ml of acetonitrile was added 4.76 g of di-(tert-butyl)carbonate and 0.10 g of 4-dimethylaminopyridine. The mixture was stirred for 1 hour, water was added and the product extracted with ethyl acetate. The organic layer was washed with water and the solvent was removed under reduced pressure. The residue was flash chromatographed on silica gel, eluting with 40% ethyl acetate/hexane, giving 1.5 g of 1-tert-butoxycarbonyl-2-methylpyrrole -3-carboxylic acid diethylamide as an oil.

PREPARATION 7

Preparation of (±)-11-(tert-Butoxycarbonyl)-5,6,8,11,12,12a-hexahydro-3-methoxybenzo[a]pyrrole[2,3-e]quinolizin-8-one and Related Compounds of Formula (IX)

A. Preparation of Formula (IX) where X is 3-methoxy and Y is hydrogen

Diisopropylamine (2.8 ml, 20 mmol)) and 75 ml of tetrahydrofuran were cooled to -70°C and 12.5 ml (20 mmol) of 2.5 M n-butyllithium was added. To the resulting solution was added a solution of 4.2 g (15 mmol) of 1-(tert-butoxycarbonyl)-2-methylpyrrole-3-carboxylic acid diethylamide in 10 ml of tetrahydrofuran and the mixture was stirred for about 3 minutes. To this mixture was added 2.9 g (18 mmol) of 6-methoxy-3,4-dihydroisoquinoline in 10 ml of tetrahydrofuran. The mixture was stirred for about 30 minutes at -70°C, and then allowed to warm to -30°C over a period of about 30 minutes. Water (100 ml) was added, and the mixture extracted twice

with ethyl acetate. The ethyl acetate extracts were combined, dried over anhydrous magnesium sulfate and evaporated to a residue, which was flash-chromatographed, eluting with 25% ethyl acetate/hexane, to give 3 g of an oil. The oil was crystallized from diethyl ether to give (±)-11-(tert-butoxycarbonyl)-5,6,8, 11,12,12a-hexahydro-3-methoxybenzo[a]pyrrole [2,3-e]quinolizin-8-one, m.p. 121-122°C.

B. Preparation of Formula (IX) where X and Y are 2,3-methylenedioxy

Similarly, replacing 6-methoxy-3,4-dihydroisoquinoline with 6,7-methylenedioxy-3,4-dihydroisoquinoline, as described in Preparation 1B, and following the procedure of Preparation 7A above, the following compound of formula (IX) was prepared:

(±)-11-(tert-butoxycarbonyl)-5,6,8,11,12,12a-hexahydro-2,3-methylenedioxybenzo[a]pyrrole[2,3-e]quinolizin-8-one, m.p. 171-172°C.

C. Preparation of Formula (IX), varying X and Y

Similarly, replacing 6-methoxy-3,4-dihydroisoquinoline with other compounds of formula (III) and following the procedure of Preparation 7A above, the following compounds of formula (IX) are prepared:

(±)-11-(tert-butoxycarbonyl)-5,6,8,11,12,12a-hexahydrobenzo[a]pyrrole[2,3-e]quinolizin-8-one;

(±)-11-(tert-butoxycarbonyl)-5,6,8,11,12,12a-hexahydro-1-methoxybenzo[a]pyrrole[2,3-e]quinolizin-8-one;

(±)-11-(tert-butoxycarbonyl)-5,6,8,11,12,12a-hexahydro-2-methoxybenzo[a]pyrrole[2,3-e]quinolizin-8-one;

(±)-11-(tert-butoxycarbonyl)-5,6,8,11,12,12a-hexahydro-4-methoxybenzo[a]pyrrole[2,3-e]quinolizin-8-one;

(±)-11-(tert-butoxycarbonyl)-5,6,8,11,12,12a-hexahydro-3-ethoxybenzo[a]pyrrole[2,3-e]quinolizin-8-one;

(±)-11-(tert-butoxycarbonyl)-5,6,8,11,12,12a-hexahydro-3-isobutoxybenzo[a]pyrrole[2,3-e]quinolizin-8-one;

(±)-11-(tert-butoxycarbonyl)-5,6,8,11,12,12a-hexahydro-3-n-hexyloxybenzo[a]pyrrole[2,3-e]quinolizin-8-one;

(±)-11-(tert-butoxycarbonyl)-5,6,8,11,12,12a-hexahydro-3-hydroxybenzo[a]pyrrole[2,3-e]quinolizin-8-one;

(±)-11-(tert-butoxycarbonyl)-5,6,8,11,12,12a-hexahydro-2,3-dihydroxybenzo[a]pyrrole[2,3-e]quinolizin-8-one;

(±)-11-(tert-butoxycarbonyl)-5,6,8,11,12,12a-hexahydro-2,3-dimethoxybenzo[a]pyrrole[2,3-e]quinolizin-8-one;

(±)-11-(tert-butoxycarbonyl)-5,6,8,11,12,12a-hexahydro-1,4-dimethoxybenzo[a]pyrrole[2,3-e]quinolizin-8-one;

(±)-11-(tert-butoxycarbonyl)-5,6,8,11,12,12a-hexahydro-3,4-dimethoxybenzo[a]pyrrole[2,3-e]quinolizin-8-one;

(±)-11-(tert-butoxycarbonyl)-5,6,8,11,12,12a-hexahydro-2,3-diethoxybenzo[a]pyrrole[2,3-e]quinolizin-8-one;

(±)-11-(tert-butoxycarbonyl)-5,6,8,11,12,12a-hexahydro-2,3-di-n-butoxybenzo[a]pyrrole[2,3-e]quinolizin-8-one;

(±)-11-(tert-butoxycarbonyl)-5,6,8,11,12,12a-hexahydro-1,2-methylenedioxybenzo[a]pyrrole[2,3-e]-quinolizin-8-one;

(±)-11-(tert-butoxycarbonyl)-5,6,8,11,12,12a-hexahydro-3,4-methylenedioxybenzo[a]pyrrole[2,3-e]quinolizin-8-one;

(±)-11-(tert-butoxycarbonyl)-5,6,8,11,12,12a-hexahydro-2,3-(ethylene-1,2-dioxy)benzo[a]pyrrole[2,3-e] quinolizin-8-one;

(±)-11-(tert-butoxycarbonyl)-5,6,8,11,12,12a-hexahydro-1-methylbenzo[a]pyrrole[2,3-e]quinolizin-8-one;

(±)-11-(tert-butoxycarbonyl)-5,6,8,11,12,12a-hexahydro-2-methylbenzo[a]pyrrole[2,3-e]quinolizin-8-one;

(±)-11-(tert-butoxycarbonyl)-5,6,8,11,12,12a-hexahydro-3-methylbenzo[a]pyrrole[2,3-e]quinolizin-8-one;

(±)-11-(tert-butoxycarbonyl)-5,6,8,11,12,12a-hexahydro-2,3-dimethylbenzo[a]pyrrole[2,3-e]quinolizin-8-one;

(±)-11-(tert-butoxycarbonyl)-5,6,8,11,12,12a-hexahydro-3-ethylbenzo[a]pyrrole[2,3-e]quinolizin-8-one;

(±)-11-(tert-butoxycarbonyl)-5,6,8,11,12,12a-hexahydro-3-isobutylbenzo[a]pyrrole[2,3-e]quinolizin-8-one;

(±)-11-(tert-butoxycarbonyl)-5,6,8,11,12,12a-hexahydro -3-n-hexylbenzo[a]pyrrole[2,3-e]quinolizin-8-one;

(±)-11-(tert-butoxycarbonyl)-5,6,8,11,12,12a-hexahydro-1-chlorobenzo[a]pyrrole[2,3-e]quinolizin-8-one;

(±)-11-(tert-butoxycarbonyl)-5,6,8,11,12,12a-hexahydro-2-chlorobenzo[a]pyrrole[2,3-e]quinolizin-8-one;

(±)-11-(tert-butoxycarbonyl)-5,6,8,11,12,12a-hexahydro-3-chlorobenzo[a]pyrrole[2,3-e]quinolizin-8-one;

(±)-11-(tert-butoxycarbonyl)-5,6,8,11,12,12a-hexahydro-3-bromobenzo[a]pyrrole[2,3-e]quinolizin-8-one;

(±)-11-(tert-butoxycarbonyl)-5,6,8,11,12,12a-hexahydro-2-fluorobenzo[a]pyrrole[2,3-e]quinolizin-8-one;

and

(±)-11-(tert-butoxycarbonyl)-5,6,8,11,12,12a-hexahydro-3-fluorobenzo[a]pyrrole[2,3-e]quinolizin-8-one.

PREPARATION 8

Preparation of (±)-(8aα,11aα,12aα)-3-methoxy-5,6,8,8a,9,10,11,11a,12,12a-decahydrobenzo[a]pyrrolo[2,3-e]quinolizin-8-one and Related Compounds of Formula (X)

A. Preparation of Formula (X) where X is 3-methoxy and Y is hydrogen

A mixture of 15 g of (±)-11-(tert-butoxycarbonyl)-5,6,8,11,12,12a-hexahydro-3-methoxybenzo[a]pyrrolo-[2,3-e]quinolizin-8-one, prepared as shown in Preparation 7, and 3.5 g of 5% Rh-$Al_2O_3$ in 200 ml of ethanol was hydrogenated at 40 psi for about 4 hours. The catalyst was removed by filtration and the filtrate was concentrated under reduced pressure. The residue was dissolved in 100 ml of methylene chloride and 15 ml of trifluoroacetic acid added. The mixture was stirred for 18 hours at room temperature and was then partitioned between methylene chloride and aqueous $NH_4OH$, the methylene chloride layer separated and the solvent removed under reduced pressure. The residue was purified by silica gel chromatography, eluting with 1% ammonium hydroxide/10% methanol/methylene chloride to give (±)-(8aα,11aα,12aα)-3-methoxy-5,6,8,8a,9,10,11,11a,12,12a-decahydrobenzo[a]pyrrolo[2,3-e]quinolizin-8-one, (X), m.p. 98-99°C.

B. Preparation of Formula (X) where X and Y are 2,3-methylenedioxy

Similarly, replacing (±)-11-(tert-butoxycarbonyl)-5,6,8,11,12,12a-hexahydro-3-methoxybenzo[a]pyrrolo-[2,3-e]quinolizin-8-one with (±)-11-(tert-butoxycarbonyl)-5,6,8,11,12,12a-hexahydro-2,3-methylenedioxybenzo[a]pyrrole-[2,3-e]quinolizin-8-one, prepared, for example, as shown in Preparation 7B, and following the procedure of Preparation 8A above, the following compound of formula (X) was prepared:

(±)-(8aα,11aα,12aα)-2,3-methylenedioxy-5,6,8,8a,9,10,11,11a,12,12a-decahydrobenzo[a]pyrrolo[2,3-e] quinolizin-8-one, m.p. 175-176°C.

C. Preparation of Formula (X), varying X and Y

Similarly, replacing (±)-11-(tert-butoxycarbonyl)5,6,8,11,12,12a-hexahydro-3-methoxybenzo[a]pyrrolo-[2,3-e]quinolizin-8-one with other compounds of formula (IX), prepared, for example, as shown in Preparation 7C, and following the procedure of Preparation 8A above, the following exemplary compounds of formula (X) are prepared:

(±)-(8aα,11aα,12aα)-5,6,8,8a,9,10,11,11a,12,12a-decahydrobenzo[a]pyrrolo[2,3-e]quinolizin-8-one;

(±)-(8aα,11aα,12aα)-1-methoxy-5,6,8,8a,9,10,11,11a,12,12a-decahydrobenzo[a]pyrrolo[2,3-e]quinolizin-8-one;

(±)-(8aα,11aα,12aα)-2-methoxy-5,6,8,8a,9,10,11,11a,12,12a-decahydrobenzo[a]pyrrolo[2,3-e]quinolizin-8-one;

(±)-(8aα,11aα,12aα)-4-methoxy-5,6,8,8a,9,10,11,11a,12,12a-decahydrobenzo[a]pyrrolo[2,3-e]quinolizin-8-one;

(±)-(8aα,11aα,12aα)-3-ethoxy-5,6,8,8a,9,10,11,11a,12,12a-decahydrobenzo[a]pyrrolo[2,3-e]quinolizin-8-one;

(±)-(8aα,11aα,12aα)-3-isobutoxy-5,6,8,8a,9,10,11,11a,12,12a-decahydrobenzo[a]pyrrolo[2,3-e]quinolizin-8-one;

(±)-(8aα,11aα,12aα)-3-n-hexyloxy-5,6,8,8a,9,10,11,11a,12,12a-decahydrobenzo[a]pyrrolo[2,3-e]quinolizin-8-one;

(±)-(8aα,11aα,12aα)-3-hydroxy-5,6,8,8a,9,10,11,11a,12,12a-decahydrobenzo[a]pyrrolo[2,3-e]quinolizin-8-one;

(±)-(8aα,11aα,12aα)-2,3-dihydroxy-5,6,8,8a,9,10,11,11a,12,12a-decahydrobenzo[a]pyrrolo[2,3-e]quinolizin-8-one;

(±)-(8aα,11aα,12aα)-2,3-dimethoxy-5,6,8,8a,9,10,11,11a,12,12a-decahydrobenzo[a]pyrrolo[2,3-e]quinolizin-8-one;

(±)-(8aα,11aα,12aα)-1,4-dimethoxy-5,6,8,8a,9,10,11,11a,12,12a-decahydrobenzo[a]pyrrolo[2,3-e]quinolizin-8-one;

(±)-(8aα,11aα,12aα)-3,4-dimethoxy-5,6,8,8a,9,10,11,11a,12,12a-decahydrobenzo[a]pyrrolo[2,3-e]quinolizin-8-one;

(±)-(8aα,11aα,12aα)-2,3-diethoxy-5,6,8,8a,9,10,11,11a,12,12a-decahydrobenzo[a]pyrrolo[2,3-e]quinolizin-8-one;

(±)-(8aα,11aα,12aα)-2,3-di-n-butoxy-5,6,8,8a,9,10,11,11a,12,12a-decahydrobenzo[a]pyrrolo[2,3-e]quinolizin-8-one;

(±)-(8aα,11aα,12aα)-1,2-methylenedioxy-5,6,8,8a,9,10,11,11a,12,12a-decahydrobenzo[a]pyrrolo[2,3-e]quinolizin-8-one;

(±)-(8aα,11aα,12aα)-3,4-methylenedioxy-5,6,8,8a,9,10,11,11a,12,12a-decahydrobenzo[a]pyrrolo[2,3-e]quinolizin-8-one;

(±)-(8aα,11aα,12aα)-2,3-(ethylene-1,2-dioxy)-5,6,8,8a,9,10,11,11a,12,12a-decahydrobenzo[a]pyrrole[2,3-e]quinolizin-8-one;

(±)-(8aα,11aα,12aα)-1-methyl-5,6,8,8a,9,10,11,11a,12,12a-decahydrobenzo[a]pyrrolo[2,3-e]quinolizin-8-one;

(±)-(8aα,11aα,12aα)-2-methyl-5,6,8,8a,9,10,11,11a,12,12a-decahydrobenzo[a]pyrrolo[2,3-e]quinolizin-8-one;

(±)-(8aα,11aα,12aα)-3-methyl-5,6,8,8a,9,10,11,11a,12,12a-decahydrobenzo[a]pyrrolo[2,3-e]quinolizin-8-one;

(±)-(8aα,11aα,12aα)-2,3-dimethyl-5,6,8,8a,9,10,11,11a,12,12a-decahydrobenzo[a]pyrrolo[2,3-e]quinolizin-8-one;

(±)-(8aα,11aα,12aα)-3-ethyl-5,6,8,8a,9,10,11,11a,12,12a-decahydrobenzo[a]pyrrolo[2,3-e]quinolizin-8-one;

(±)-(8aα,11aα,12aα)-3-isobutyl-5,6,8,8a,9,10,11,11a,12,12a-decahydrobenzo[a]pyrrolo[2,3-e]quinolizin-8-one;

(±)-(8aα,11aα,12aα)-3-n-hexyl-5,6,8,8a,9,10,11,11a,12,12a-decahydrobenzo[a]pyrrolo[2,3-e]quinolizin-8-one;

(±)-(8aα,11aα,12aα)-1-chloro-5,6,8,8a,9,10,11,11a,12,12a-decahydrobenzo[a]pyrrolo[2,3-e]quinolizin-8-one;

(±)-(8aα,11aα,12aα)-2-chloro-5,6,8,8a,9,10,11,11a,12,12a-decahydrobenzo[a]pyrrolo[2,3-e]quinolizin-8-one;

(±)-(8aα,11aα,12aα)-3-chloro-5,6,8,8a,9,10,11,11a,12,12a-decahydrobenzo[a]pyrrolo[2,3-e]quinolizin-8-one;

(±)-(8aα,11aα,12aα)-3-bromo-5,6,8,8a,9,10,11,11a,12,12a-decahydrobenzo[a]pyrrolo[2,3-e]quinolizin-8-one;

(±)-(8aα,11aα,12aα)-2-fluoro-5,6,8,8a,9,10,11,11a,12,12a-decahydrobenzo[a]pyrrolo[2,3-e]quinolizin-8-one; and

(±)-(8aα,11aα,12aα)-3-fluoro-5,6,8,8a,9,10,11,11a,12,12a-decahydrobenzo[a]pyrrolo[2,3-e]quinolizin-8-one.

## PREPARATION 9

### Preparation of (±)-(8aα,11aα,12aα)-3-methoxy-5,6,8,8a,9,10,11,11a,12,12a-decahydrobenzo[a]pyrrolo[2,3-e]quinolizine and Related Compounds of Formula (XI)

### A. Preparation of Formula (XI) where X is 3-methoxy and Y is hydrogen

A solution of 7.6 g of (±)-(8aα,11aα,12aα)-3-methoxy-5,6,8,8a,9,10,11,11a,12,12a-decahydrobenzo[a]pyrrolo-[2,3-e]quinolizin-8-one (X), prepared as shown in Preparation 8, in 125 ml of tetrahydrofuran was added slowly to a solution of 1.0 g of lithium aluminum hydride in 100 ml of tetrahydrofuran. The resulting mixture was stirred at reflux for 3 hours, cooled, and treated sequentially with 2.5 ml of water, 2.5 ml of 15% sodium hydroxide, and 7.5 ml of water. The mixture was filtered and the filtrate was evaporated to afford (±)-(8aα,11aα,12aα)-3-methoxy-5,6,8, 8a,9,10,11,11a,-12,12a-decahydrobenzo[a]pyrrolo[2,3-e]quinolizine as an oil. The oil was used in the next reaction with no further purification.

### B. Preparation of Formula (XI) where X and Y are 2,3-methylenedioxy

Similarly, replacing (±)-(8aα,11aα,12aα)-3-methoxy-5,6,8,8a,9,10,11,11a,12,12a-decahydrobenzo[a]pyrrolo[2,3-e]quinolizin-8-one with (±)-(8aα,11aα,12aα)-2,3-methylenedioxy -5,6,8,8a,9,10,11,11a,12,12a-decahydro-benzo[a]pyrrolo[2,3-e]quinolizin-8-one, prepared, for example, as shown in Preparation 8B, and following the procedure of Preparation 9A above, the following compound of formula (XI) was prepared:

(±)-(8aα,11aα,12aα)-2,3-methylenedioxy-5,6,8,8a,9,10, 11,11a,12,12a-decahydrobenzo[a]pyrrolo[2,3-e]quinolizine.

C. Preparation of Formula (XI), varying X and Y

Similarly, replacing (±)-(8aα,11aα,12aα)-3-methoxy-5,6,8,8a,9,10,11,11a,12,12a-decahydrobenzo[a]pyrrolo[2,3-e]quinolizin-8-one with other compounds of formula (X), prepared, for example, as shown in Preparation 8C, and following the procedure of Preparation 9A above, the following exemplary compounds of formula (XI) are prepared:

(±)-(8aα,11aα,12aα)-5,6,8,8a,9,10,11,11a,12,12a-decahydrobenzo[a]pyrrolo[2,3-e]quinolizine;
(±)-(8aα,11aα,12aα)-1-methoxy-5,6,8,8a,9,10,11,11a,12,12a-decahydrobenzo[a]pyrrolo[2,3-e]quinolizine;
(±)-(8aα,11aα,12aα)-2-methoxy-5,6,8,8a,9,10,11,11a,12,12a-decahydrobenzo[a]pyrrolo[2,3-e]quinolizine;
(±)-(8aα,11aα,12aα)-4-methoxy-5,6,8,8a,9,10,11,11a,12,12a-decahydrobenzo[a]pyrrolo[2,3-e]quinolizine;
(±)-(8aα,11aα,12aα)-3-ethoxy-5,6,8,8a,9,10,11,11a,12,12a-decahydrobenzo[a]pyrrolo[2,3-e]quinolizine;
(±)-(8aα,11aα,12aα)-3-isobutoxy-5,6,8,8a,9,10,11,11a,12,12a-decahydrobenzo[a]pyrrolo[2,3-e]quinolizine;
(±)-(8aα,11aα,12aα)-3-n-hexyloxy-5,6,8,8a,9,10,11,11a,12,12a-decahydrobenzo[a]pyrrolo[2,3-e]quinolizine;
(±)-(8aα,11aα,12aα)-3-hydroxy-5,6,8,8a,9,10,11,11a,12,12a-decahydrobenzo[a]pyrrolo[2,3-e]quinolizine;
(±)-(8aα,11aα,12aα)-2,3-dihydroxy-5,6,8,8a,9,10,11,11a,12,12a-decahydrobenzo[a]pyrrolo[2,3-e]quinolizine;
(±)-(8aα,11aα,12aα)-2,3-dimethoxy-5,6,8,8a,9,10,11,11a,12,12a-decahydrobenzo[a]pyrrolo[2,3-e]quinolizine;
(±)-(8aα,11aα,12aα)-1,4-dimethoxy-5,6,8,8a,9,10,11,11a,12,12a-decahydrobenzo[a]pyrrolo[2,3-e]quinolizine;
(±)-(8aα,11aα,12aα)-3,4-dimethoxy-5,6,8,8a,9,10,11,11a,12,12a-decahydrobenzo[a]pyrrolo[2,3-e]quinolizine;
(±)-(8aα,11aα,12aα)-2,3-diethoxy-5,6,8,8a,9,10,11,11a,12,12a-decahydrobenzo[a]pyrrolo[2,3-e]quinolizine;
(±)-(8aα,11aα,12aα)-2,3-di-n-butoxy-5,6,8,8a,9,10,11,11a,12,12a-decahydrobenzo[a]pyrrolo[2,3-e]quinolizine;
(±)-(8aα,11aα,12aα)-1,2-methylenedioxy-5,6,8,8a,9,10,11,11a,12,12a-decahydrobenzo[a]pyrrolo[2,3-e]quinolizine;
(±)-(8aα,11aα,12aα)-3,4-methylenedioxy-5,6,8,8a,9,10,11,11a,12,12a-decahydrobenzo[a]pyrrolo[2,3-e]quinolizine;
(±)-(8aα,11aα,12aα)-2,3-(ethylene-1,2-dioxy)-5,6,8,8a,9,10,11,11a,12,12a-decahydrobenzo[a]pyrrolo[2,3-e]quinolizine;
(±)-(8aα,11aα,12aα)-1-methyl-5,6,8,8a,9,10,-11,11a,12,12a-decahydrobenzo[a]pyrrolo[2,3-e]quinolizine;
(±)-(8aα,11aα,12aα)-2-methyl-5,6,8,8a,9,10,11,11a,12,12a-decahydrobenzo[a]pyrrolo[2,3-e]quinolizine;
(±)-(8aα,11aα,12aα)-3-methyl-5,6,8,8a,9,10,11,11a,12,12a-decahydrobenzo[a]pyrrolo[2,3-e]quinolizine;
(±)-(8aα,11aα,12aα)-2,3-dimethyl-5,6,8,8a,9,10,11,11a,12,12a-decahydrobenzo[a]pyrrolo[2,3-e]quinolizine;
(±)-(8aα,11aα,12aα)-3-ethyl-5,6,8,8a,9,10,11,11a,12,12a-decahydrobenzo[a]pyrrolo[2,3-e]quinolizine;
(±)-(8aα,11aα,12aα)-3-isobutyl-5,6,8,8a,9,10,11,11a,12,12a-decahydrobenzo[a]pyrrolo[2,3-e]quinolizine;
(±)-(8aα,11aα,12aα)-3-n-hexyl-5,6,8,8a,9,10,11,11a,12,12a-decahydrobenzo[a]pyrrolo[2,3-e]quinolizine;
(±)-(8aα,11aα,12aα)-1-chloro-5,6,8,8a,9,10,11,11a,12,12a-decahydrobenzo[a]pyrrolo[2,3-e]quinolizine;
(±)-(8aα,11aα,12aα)-2-chloro-5,6,8,8a,9,10,11,11a,12,12a-decahydrobenzo[a]pyrrolo[2,3-e]quinolizine;
(±)-(8aα,11aα,12aα)-3-chloro-5,6,8,8a,9,10,11,11a,12,12a-decahydrobenzo[a]pyrrolo[2,3-e]quinolizine;
(±)-(8aα,11aα,12aα)-3-bromo-5,6,8,8a,9,10,11,11a,12,12a-decahydrobenzo[a]pyrrolo[2,3-e]quinolizine;
(±)-(8aα,11aα,12aα)-2-fluoro-5,6,8,8a,9,10,11,11a,12,12a-decahydrobenzo[a]pyrrolo[2,3-e]quinolizine; and
(±)-(8aα,11aα,12aα)-3-fluoro-5,6,8,8a,9,10,11,11a,12,12a-decahydrobenzo[a]pyrrolo[2,3-e]quinolizine.

PREPARATION 10

Preparation of (±)-(8aα,11aα,12aα)-3-methoxy-11-(3-chloropropanesulfonyl)-5,6,8,8a,9,10,11,11a,12,12a-decahydrobenzo[a]pyrrolo[2,3-e]quinolizine and Related Compounds of Formula (XII)

A. Preparation of Formula (XII) where A is propylene, X is 3-methoxy, Y is hydrogen, Z is chloro and n is 1

A solution of 7.2 g of (±)-(8aα,11aα,12aα)-3-methoxy-5,6,8,8a,9,10,11,11a,12,12a-decahydrobenzo[a]pyrrolo[2,3-e]quinolizine (XI), prepared as shown in Preparation 9, and 7 ml of triethylamine in 150 ml of methylene chloride was cooled to 0°C, and 5.5 g of 3-chloropropanesulfonyl chloride in 50 ml of dichloromethane was added. An exothermic reaction was observed. The resulting mixture was stirred at 0°C for 30 minutes, then washed with dilute ammonium hydroxide. The methylene chloride layer was separated and the solvent removed under reduced pressure. The residue was purified by silica gel chromatography, eluting with 50% ethyl acetate/hexane, to afford (±)-(8aα,11aα,12aα)-3-methoxy -11-(3-chloropropane-sulfonyl)-5,6,8,8a,9,10,11,11a, 12,12a-decahydrobenzo[a]pyrrolo[2,3-e]quinolizine, a compound of formula (XII) where n is 1, as an oil.

B. Preparation of Formula (XII) where A is propylene, X is 3-methoxy, Y is hydrogen, Z is chloro and n is 2

Similarly, replacing (±)-(8aα,11aα,12aα)-3-methoxy-5,6,8,8a,9,10,11,11a,12,12a-decahydrobenzo[a]pyrrolo[2,3-e]quinolizine with (8aR,12aS,13aS)-3-methoxy-5,6,8a,9,10,11, 12,12a,13,13a-decahydro-8H-isoquino[2,1-g][1,6]naphthyridine prepared, for example, as shown in Preparation 4, and following the procedure of Preparation 10A above, the following compound of formula (XII) where n is 2 was prepared:

(8aR,12aS,13aS)-3-methoxy-12-(3-chloropropanesulfonyl)-5,6,8a,9,10,11,12,12a,13,13a-decahydro-8 H-isoquino [2,1-g][1,6]naphthyridine as an oil, which was converted to the hydrochloride salt in ethanol/ether mixture by treatment with anhydrous hydrochloric acid, to give (8aR,12aS,13aS)-3-methoxy-12-(3-chloropropanesulfonyl)-5,6,8a,9,10,11,12,12a,13,13a-decahydro-8H-isoquino [2,1-g][1,6]naphthyridine hydrochloride, m.p. 203-205°C, $[\alpha]_D$ = +28.7° (c = 0.55, $CH_3OH$).

C. Preparation of Formula (XII) where n is 1, varying A, X, Y and Z

Similarly, optionally replacing (±)-(8aα,11aα,12aα)3-methoxy-5,6,8,8a,9,10,11,11a,12,12a-decahydrobenzo[a]pyrrolo[2,3-e]quinolizine with racemic compounds of formula (XI), prepared, for example, as shown in Preparation 9; or single enantiomers of a compound of formula (XI), optionally replacing 3-chloropropanesulfonyl chloride with an appropriate haloalkylsulfonyl halide of formula $ZASO_2Z$, where Z is chloro and A is lower alkylene of 1-6 carbon atoms, and following the procedure of Preparation 10A above, the following compounds of formula (XII) where n is 1 are prepared in racemic or optically active form:

(8aα,11aα,12aα)-3-methoxy-11-(chloromethanesulfonyl)-5,6,8,8a,9,10,11,11a,12,12a-decahydrobenzo [a]pyrrolo[2,3-e]quinolizine;

(8aα,11aα,12aα)-3-methoxy-11-(2-chloroethanesulfonyl)-5,6,8,8a,9,10,11,11a,12,12a-decahydrobenzo [a]pyrrolo[2,3-e]quinolizine;

(8aα,11aα,12aα)-3-methoxy-11-(4-chlorobutanesulfonyl)-5,6,8,8a,9,10,11,11a,12,12a-decahydrobenzo [a]pyrrolo[2,3-e]quinolizine;

(8aα,11aα,12aα)-3-methoxy-11-(3-chloro-2-methylpropanesulfonyl)-5,6,8,8a,9,10,11,11a,12,12a-decahydrobenzo[a]pyrrolo[2,3-e]quinolizine;

(8aα,11aα,12aα)-3-methoxy-11-(3-chloro-1,2-dimethylpropanesulfonyl)-5,6,8,8a,9,10,11,11a,12,12a-decahydrobenzo[a]pyrrolo[2,3-e]quinolizine;

(8aα,11aα,12aα)-3-methoxy-11-(6-chlorohexanesulfonyl)-5,6,8,8a,9,10,11,11a,12,12a-decahydrobenzo [a]pyrrolo[2,3-e]quinolizine;

(8aα,11aα,12aα)-11-(3-chloropropanesulfonyl)-5,6,8,8a,9,10,11,11a,12,12a-decahydrobenzo[a]pyrrolo [2,3-e]quinolizine;

(8aα,11aα,12aα)-1-methoxy-11-(3-chloropropanesulfonyl)-5,6,8,8a,9,10,11,11a,12,12a-decahydrobenzo [a]pyrrolo[2,3-e]quinolizine;

(8aα,11aα,12aα)-2-methoxy-11-(3-chloropropanesulfonyl)-5,6,8,8a,9,10,11,11a,12,12a-decahydrobenzo [a]pyrrolo[2,3-e]quinolizine;

(8aα,11aα,12aα)-4-methoxy-11-(3-chloropropanesulfonyl)-5,6,8,8a,9,10,11,11a,12,12a-decahydrobenzo [a]pyrrolo[2,3-e]quinolizine;

(8aα,11aα,12aα)-3-ethoxy-11-(3-chloropropanesulfonyl)-5,6,8,8a,9,10,11,11a,12,12a-decahydrobenzo [a]pyrrolo[2,3-e]quinolizine;

(8aα,11aα,12aα)-3-isobutoxy-11-(3-chloropropanesulfonyl)-5,6,8,8a,9,10,11,11a,12,12a-decahydrobenzo[a]pyrrolo[2,3-e]quinolizine;

(8aα,11aα,12aα)-3-n-hexyloxy-11-(3-chloropropanesulfonyl)-5,6,8,8a,9,10,11,11a,12,12a-decahydrobenzo[a]pyrrolo[2,3-e]quinolizine;

(8aα,11aα,12aα)-3-hydroxy-11-(3-chloropropanesulfonyl)-5,6,8,8a,9,10,11,11a,12,12a-decahydrobenzo [a]pyrrolo[2,3-e]quinolizine;

(8aα,11aα,12aα)-2,3-dihydroxy-11-(3-chloropropanesulfonyl)-5,6,8,8a,9,10,11,11a,12,12a-decahydrobenzo[a]pyrrolo[2,3-e]quinolizine;

(8aα,11aα,12aα)-2,3-dimethoxy-11-(3-chloropropanesulfonyl)-5,6,8,8a,9,10,11,11a,12,12a-decahydrobenzo[a]pyrrolo[2,3-e]quinolizine;

(8aα,11aα,12aα)-1,4-dimethoxy-11-(3-chloropropanesulfonyl)-5,6,8,8a,9,10,11,11a,12,12a-decahydrobenzo[a]pyrrolo[2,3-e]quinolizine;

(8aα,11aα,12aα)-3,4-dimethoxy-11-(3-chloropropanesulfonyl)-5,6,8,8a,9,10,11,11a,12,12a-decahydrobenzo[a]pyrrolo[2,3-e]quinolizine;

(8aα,11aα,12aα)-2,3-diethoxy-11-(3-chloropropanesulfonyl)-5,6,8,8a,9,10,11,11a,12,12a-decahydrobenzo[a]pyrrolo[2,3-e]quinolizine;

(8aα,11aα,12aα)-2,3-di-n-butoxy-11-(3-chloropropanesulfonyl)-5,6,8,8a,9,10,11,11a,12,12a-decahydrobenzo [a]pyrrolo[2,3-e]quinolizine;

(8aα,11aα,12aα)-2,3-dihexyloxy-11-(3-chloropropanesulfonyl)-5,6,8,8a,9,10,11,11a,12,12a-decahydrobenzo [a]pyrrolo[2,3-e]quinolizine;

(8aα,11aα,12aα)-2,3-methylenedioxy-11-(3-chloropropanesulfonyl)-5,6,8,8a,9,10,11,11a,12,12a-decahydrobenzo [a]pyrrolo[2,3-e]quinolizine;

(8aα,11aα,12aα)-2,3-methylenedioxy-11-(chloromethanesulfonyl)-5,6,8,8a,9,10,11,11a,12,12a-decahydrobenzo[a]pyrrolo[2,3-e]quinolizine;

(8aα,11aα,12aα)-2,3-methylenedioxy-11-(2-chloroethanesulfonyl)-5,6,8,8a,9,10,11,11a,12,12a-decahydrobenzo [a]pyrrolo[2,3-e]quinolizine;

(8aα,11aα,12aα)-2,3-methylenedioxy-11-(4-chlorobutanesulfonyl)-5,6,8,8a,9,10,11,11a,12,12a-decahydrobenzo[a]pyrrolo[2,3-e]quinolizine;

(8aα,11aα,12aα)-2,3-methylenedioxy-11-(3-chloro-2-methylpropanesulfonyl)-5,6,8,8a,9,10,11,11a,12,12a-decahydrobenzo[a]pyrrolo[2,3-e]quinolizine;

(8aα,11aα,12aα)-2,3-methylenedioxy-11-(3-chloro-1,2-dimethylpropanesulfonyl)-5,6,8,8a,9,10,11,11a,12,12a-decahydrobenzo [a]pyrrolo[2,3-e]quinolizine;

(8aα,11aα,12aα)-2,3-methylenedioxy-11-(6-chlorohexanesulfonyl)-5,6,8,8a,9,10,11,11a,12,12a-decahydrobenzo[a]pyrrolo[2,3-e]quinolizine;

(8aα,11aα,12aα)-1,2-methylenedioxy-11-(3-chloropropanesulfonyl)-5,6,8,8a,9,10,11,11a,12,12a-decahydrobenzo[a]pyrrolo[2,3-e]quinolizine;

(8aα,11aα,12aα)-3,4-methylenedioxy-11-(3-chloropropanesulfonyl)-5,6,8,8a,9,10,11,11a,12,12a-decahydrobenzo[a]pyrrolo[2,3-e]quinolizine;

(8aα,11aα,12aα)-2,3-(ethylene-1,2-dioxy)-11-(3-chloropropanesulfonyl)-5,6,8,8a,9,10,11,11a,12,12a-decahydrobenzo[a]pyrrole[2,3-e]quinolizine;

(8aα,11aα,12aα)-1-methyl-11-(3-chloropropanesulfonyl)-5,6,8,8a,9,10,11,11a,12,12a-decahydrobenzo [a]pyrrolo[2,3-e]quinolizine;

(8aα,11aα,12aα)-2-methyl-11-(3-chloropropanesulfonyl)-5,6,8,8a,9,10,11,11a,12,12a-decahydrobenzo [a]pyrrolo[2,3-e]quinolizine;

(8aα,11aα,12aα)-3-methyl-11-(3-chloropropanesulfonyl)-5,6,8,8a,9,10,11,11a,12,12a-decahydrobenzo [a]pyrrolo[2,3-e]quinolizine;

(8aα,11aα,12aα)-2,3-dimethyl-11-(3-chloropropanesulfonyl)-5,6,8,8a,9,10,11,11a,12,12a-decahydrobenzo[a]pyrrolo[2,3-e]quinolizine;

(8aα,11aα,12aα)-3-ethyl-11-(3-chloropropanesulfonyl)-5,6,8,8a,9,10,11,11a,12,12a-decahydrobenzo[a] pyrrolo[2,3-e]quinolizine;

(8aα,11aα,12aα)-3-isobutyl-11-(3-chloropropanesulfonyl)-5,6,8,8a,9,10,11,11a,12,12a-decahydrobenzo [a]pyrrolo[2,3-e]quinolizine;

(8aα,11aα,12aα)-3-n-hexyl-11-(3-chloropropanesulfonyl)-5,6,8,8a,9,10,11,11a,12,12a-decahydrobenzo [a]pyrrolo[2,3-e]quinolizine;

(8aα,11aα,12aα)-1-chloro-11-(3-chloropropanesulfonyl)-5,6,8,8a,9,10,11,11a,12,12a-decahydrobenzo [a]pyrrolo[2,3-e]quinolizine;

(8aα,11aα,12aα)-2-chloro-11-(3-chloropropanesulfonyl)-5,6,8,8a,9,10,11,11a,12,12a-decahydrobenzo [a]pyrrolo[2,3-e]quinolizine;

(8aα,11aα,12aα)-3-chloro-11-(3-chloropropanesulfonyl)-5,6,8,8a,9,10,11,11a,12,12a-decahydrobenzo [a]pyrrolo[2,3-e]quinolizine;

(8aα,11aα,12aα)-3-bromo-11-(3-chloropropanesulfonyl)-5,6,8,8a,9,10,11,11a,12,12a-decahydrobenzo [a]pyrrolo[2,3-e]quinolizine;

(8aα,11aα,12aα)-2-fluoro-11-(3-chloropropanesulfonyl)-5,6,8,8a,9,10,11,11a,12,12a-decahydrobenzo [a]pyrrolo[2,3-e]quinolizine; and

(8aα,11aα,12aα)-3-fluoro-11-(3-chloropropanesulfonyl)-5,6,8,8a,9,10,11,11a,12,12a-decahydrobenzo [a]pyrrolo[2,3-e]quinolizine.

D. Preparation of Formula (XII) where n is 2, varying A, X, Y and Z

Similarly, optionally replacing (±)-(8aα,11aα,12aα)-3-methoxy-5,6,8,8a,9,10,11,11a,12,12a-decahydrobenzo[a]pyrrolo[2,3-e]quinolizine with an appropriate racemic compound of formula (VII), prepared, for example, as shown in Preparation 3, or an individual enantiomer of formula (VIIA) or (VIIB), prepared, for example, as shown in Preparation 4, and optionally replacing 3-chloropropanesulfonyl chloride with an appropriate haloalkylsulfonyl halide of formula $ZASO_2Z$, where Z is chloro and A is lower alkylene of 1-6 carbon atoms, and following the procedures of Preparation 10A above, the following compounds of formula (XII) where n is 2 are prepared in racemic or optically active form:

(8aα,12aα,13aα)-3-methoxy-12-(chloromethanesulfonyl)-5,6,8a,9,10,11,12,12a,13,13a-decahydro-8H-isoquino [2,1-g][1,6]naphthyridine;

(8aα,12aα,13aα)-3-methoxy-12-(2-chloroethanesulfonyl)-5,6,8a,9,10,11,12,12a,13,13a-decahydro-8H-isoquino [2,1-g][1,6]naphthyridine;

(8aα,12aα,13aα)-3-methoxy-12-(4-chlorobutanesulfonyl)-5,6,8a,9,10,11,12,12a,13,13a-decahydro-8H-isoquino[2,1-g][1,6]naphthyridine;

(8aα,12aα,13aα)-3-methoxy-12-(3-chloro-2-methylpropanesulfonyl)-5,6,8a,9,10,11,12,12a,13,13a-decahydro-8H-isoquino[2,1-g][1,6]naphthyridine;

(8aα,12aα,13aα)-3-methoxy-12-(3-chloro-1,2-dimethylpropanesulfonyl)-5,6,8a,9,10,11,12,12a,13,13a-decahydro-8H-isoquino[2,1-g][1,6]naphthyridine;

(8aα,12aα,13aα)-3-methoxy-12-(6-chlorohexanesulfonyl)-5,6,8a,9,10,11,12,12a,13,13a-decahydro-8H-isoquino[2,1-g][1,6]naphthyridine;

(8aα,12aα,13aα)-12-(3-chloropropanesulfonyl)-5,6,8a,9,10,11,12,12a,13,13a-decahydro-8H-isoquino [2,1-g][1,6]naphthyridine;

(8aα,12aα,13aα)-2,3-methylenedioxy-12-(3-chloropropanesulfonyl)-5,6,8a,9,10,11,12,12a,13,13a-decahydro-8H-isoquino[2,1-g][1,6]naphthyridine

(8aα,12aα,13aα)-2,3-methylenedioxy-12-(chloromethanesulfonyl)-5,6,8a,9,10,11,12,12a,13,13a-decahydro-8H-isoquino[2,1-g][1,6]naphthyridine;

(8aα,12aα,13aα)-2,3-methylenedioxy-12-(2-chloroethanesulfonyl)-5,6,8a,9,10,11,12,12a,13,13a-decahydro-8H-isoquino[2,1-g][1,6]naphthyridine;

(8aα,12aα,13aα)-2,3-methylenedioxy-12-(4-chlorobutanesulfonyl)-5,6,8a,9,10,11,12,12a,13,13a-decahydro-8H-isoquino[2,1-g][1,6]naphthyridine;

(8aα,12aα,13aα)-2,3-methylenedioxy-12-(3-chloro-2-methylpropanesulfonyl)-5,6,8a,9,10,11,12,12a,13,13a-decahydro-8H-isoquino[2,1-g][1,6]naphthyridine;

(8aα,12aα,13aα)-2,3-methylenedioxy-12-(3-chloro-1,2-dimethylpropanesulfonyl)-5,6,8a,9,10,11,12,12a,13,13a-decahydro-8H-isoquino[2,1-g][1,6]naphthyridine;

(8aα,12aα,13aα)-2,3-methylenedioxy-12-(6-chlorohexanesulfonyl)-5,6,8a,9,10,11,12,12a,13,13a-decahydro-8H-isoquino[2,1-g][1,6]naphthyridine;

(8aα,12aα,13aα)-2,3-dimethoxy-12-(3-chloropropanesulfonyl)-5,6,8a,9,10,11,12,12a,13,13a-decahydro-8H-isoquino[2,1-g][1,6]naphthyridine;

(8aα,12aα,13aα)-1,4-dimethoxy-12-(3-chloropropanesulfonyl)-5,6,8a,9,10,11,12,12a,13,13a-decahydro-8H-isoquino[2,1-g][1,6]naphthyridine;

(8aα,12aα,13aα)-2,3-(ethylene-1,2-dioxy)-12-(3-chloropropanesulfonyl)-5,6,8a,9,10,11,12,12a,13,13a-decahydro-8H-isoquino[2,1-g][1,6]naphthyridine;

(8aα,12aα,13aα)-1-methyl-12-(3-chloropropanesulfonyl)-5,6,8a,9,10,11,12,12a,13,13a-decahydro-8H-isoquino[2,1-g][1,6]naphthyridine;

(8aα,12aα,13aα)-2-methyl-12-(3-chloropropanesulfonyl)-5,6,8a,9,10,11,12,12a,13,13a-decahydro-8H-isoquino[2,1-g][1,6]naphthyridine;

(8aα,12aα,13aα)-3-methyl-12-(3-chloropropanesulfonyl)-5,6,8a,9,10,11,12,12a,13,13a-decahydro-8H-isoquino[2,1-g][1,6]naphthyridine;

(8aα,12aα,13aα)-2,3-dimethyl-12-(3-chloropropanesulfonyl)-5,6,8a,9,10,11,12,12a,13,13a-decahydro-8H-isoquino[2,1-g][1,6]naphthyridine;

(8aα,12aα,13aα)-3-ethyl-12-(3-chloropropanesulfonyl)-5,6,8a,9,10,11,12,12a,13,13a-decahydro-8H-isoquino-[2,1-g][1,6]naphthyridine;

(8aα,12aα,13aα)-3-isobutyl-12-(3-chloropropanesulfonyl)-5,6,8a,9,10,11,12,12a,13,13a-decahydro-8H-isoquino[2,1-g][1,6]naphthyridine;

(8aα,12aα,13aα)-3-n-hexyl-12-(3-chloropropanesulfonyl)-5,6,8a,9,10,11,12,12a,13,13a-decahydro-8H-isoquino[2,1-g][1,6]naphthyridine;

(8aα,12aα,13aα)-1-methoxy-12-(3-chloropropanesulfonyl)-5,6,8a,9,10,11,12,12a,13,13a-decahydro-8H-isoquino[2,1-g][1,6]naphthyridine;

(8aα,12aα,13aα)-2-methoxy-12-(3-chloropropanesulfonyl)-5,6,8a,9,10,11,12,12a,13,13a-decahydro-8H-isoquino[2,1-g][1,6]naphthyridine;

(8aα,12aα,13aα)-4-methoxy-12-(3-chloropropanesulfonyl)-5,6,8a,9,10,11,12,12a,13,13a-decahydro-8H-isoquino[2,1-g][1,6]naphthyridine;

(8aα,12aα,13aα)-3-methoxy-2-methyl-12-(3-chloropropanesulfonyl)-5,6,8a,9,10,11,12,12a,13,13a-decahydro-8H-isoquino[2,1-g][1,6]naphthyridine;

(8aα,12aα,13aα)-3-ethoxy-12-(3-chloropropanesulfonyl)-5,6,8a,9,10,11,12,12a,13,13a-decahydro-8H-isoquino[2,1-g][1,6]naphthyridine;

(8aα,12aα,13aα)-3-isopropoxy-12-(3-chloropropanesulfonyl)-5,6,8a,9,10,11,12,12a,13,13a-decahydro-8H-isoquino[2,1-g][1,6]naphthyridine;

(8aα,12aα,13aα)-3-isopropoxy-12-(3-chloropropanesulfonyl)-5,6,8a,9,10,11,12,12a,13,13a-decahydro-8H-isoquino[2,1-g][1,6]naphthyridine;

(8aα,12aα,13aα)-3-n-hexyloxy-12-(3-chloropropanesulfonyl)-5,6,8a,9,10,11,12,12a,13,13a-decahydro-8H-isoquino[2,1-g][1,6]naphthyridine;

(8aα,12aα,13aα)-3-hydroxy-12-(3-chloropropanesulfonyl)-5,6,8a,9,10,11,12,12a,13,13a-decahydro-8H-isoquino[2,1-g][1,6]naphthyridine;

(8aα,12aα,13aα)-2,3-dihydroxy-12-(3-chloropropanesulfonyl)-5,6,8a,9,10,11,12,12a,13,13a-decahydro-8H-isoquino[2,1-g][1,6]naphthyridine;

(8aα,12aα,13aα)-1,2-dimethoxy-12-(3-chloropropanesulfonyl)-5,6,8a,9,10,11,12,12a,13,13a-decahydro-8H-isoquino[2,1-g][1,6]naphthyridine;

(8aα,12aα,13aα)-1,4-dimethoxy-12-(3-chloropropanesulfonyl)-5,6,8a,9,10,11,12,12a,13,13a-decahydro-8H-isoquino[2,1-g][1,6]naphthyridine;

(8aα,12aα,13aα)-3,4-dimethoxy-12-(3-chloropropanesulfonyl)-5,6,8a,9,10,11,12,12a,13,13a-decahydro-8H-isoquino[2,1-g][1,6]naphthyridine;

(8aα,12aα,13aα)-2,3-diethoxy-12-(3-chloropropanesulfonyl)-5,6,8a,9,10,11,12,12a,13,13a-decahydro-8H-isoquino[2,1-g][1,6]naphthyridine;

(8aα,12aα,13aα)-2,3-di-n-butoxy-12-(3-chloropropanesulfonyl)-5,6,8a,9,10,11,12,12a,13,13a-decahydro-8H-isoquino[2,1-g][1,6]naphthyridine;

(8aα,12aα,13aα)-1,2-methylenedioxy-12-(3-chloropropanesulfonyl)-5,6,8a,9,10,11,12,12a,13,13a-decahydro-8H-isoquino[2,1-g][1,6]naphthyridine;

(8aα,12aα,13aα)-2-chloro-12-(3-chloropropanesulfonyl)-5,6,8a,9,10,11,12,12a,13,13a-decahydro-8H-isoquino[2,1-g][1,6]naphthyridine;

(8aα,12aα,13aα)-3-chloro-12-(3-chloropropanesulfonyl)-5,6,8a,9,10,11,12,12a,13,13a-decahydro-8H-isoquino[2,1-g][1,6]naphthyridine;

(8aα,12aα,13aα)-4-chloro-12-(3-chloropropanesulfonyl)-5,6,8a,9,10,11,12,12a,13,13a-decahydro-8H-isoquino[2,1-g][1,6]naphthyridine;

(8aα,12aα,13aα)-3-bromo-12-(3-chloropropanesulfonyl)-5,6,8a,9,10,11,12,12a,13,13a-decahydro-8H-isoquino[2,1-g][1,6]naphthyridine;

(8aα,12aα,13aα)-3-fluoro-12-(3-chloropropanesulfonyl)-5,6,8a,9,10,11,12,12a,13,13a-decahydro-8H-isoquino[2,1-g][1,6]naphthyridine; and

(8aα,12aα,13aα)-2-fluoro-12-(3-chloropropanesulfonyl)-5,6,8a,9,10,11,12,12a,13,13a-decahydro-8H-isoquino[2,1-g][1,6]naphthyridine.

## PREPARATION 11

### Preparation of (±)-(8aα,11aα,12aα)-3-methoxy-11-(3-aminopropanesulfonyl)-5,6,8,8a,9,10,11,11a,12,12a-decahydrobenzo [a]pyrrolo[2,3-e]quinolizine and Related Compounds of Formula (XIV)

A. Preparation of Formula (XIII) where A is propylene, X is 3-methoxy, Y is hydrogen and n is 1

A mixture of 5 g of (±)-(8aα,11aα,12aα)-3-methoxy-11-(3-chloropropanesulfonyl)-5,6,8,8a,9,10,11,11a,12,12a-decahydrobenzo[a]pyrrolo[2,3-e]quinolizine (XII), prepared as shown in Preparation 10, 1.6 g of sodium azide and 50 mg of sodium iodide in 25 ml of N,N-dimethylformamide was stirred at 80°C overnight. The reaction mixture was poured into 250 ml of water and extracted three times with diethyl ether. The organic layer was washed with water, then brine, and dried over anhydrous sodium sulfate. The solvent was removed, leaving a residue of (±)-(8aα,11aα,12aα)-3-methoxy-11-(3-azidopropanesulfonyl)-5,6,8,8a,9,10,11,11a,12,12a-decahydrobenzo [a]pyrrolo[2,3-e]quinolizine, a compound of formula (XIII) where n is 1.

B. Preparation of Formula (XIV) where A is propylene, X is 3-methoxy, Y is hydrogen and n is 1

The compound of formula (XIII) was then dissolved in 100 ml of ethanol containing excess anhydrous hydrochloric acid, and 1.7 g of 20% palladium hydroxide on carbon added. The mixture was hydrogenated at 50 psi for 18 hours, then filtered and the solvent removed under reduced pressure. The residue was partitioned between methylene chloride and aqueous $NH_4OH$, the methylene chloride layer separated and the solvent removed under reduced pressure to afford (±)-(8aα,11aα,12aα)-3-methoxy-11-(3-aminopropanesulfonyl)-5,6,8,8a,9,10,11,11a,12,12a-decahydrobenzo[a]pyrrolo[2,3-e]quinolizine, a compound of formula (XIV) where n is 1, as an oil.

C. Preparation of Formula (XIV) where A is propylene, X is 3-methoxy, Y is hydrogen and n is 2

Similarly, replacing (±)-(8aα,11aα,12aα)-3-methoxy-5,6,8,8a,9,10,11,11a,12,12a-decahydrobenzo[a]pyrrolo[2,3-e]quinolizine with (8aR,12aS,13aS)-3-methoxy-5,6,8a,9,10,11,12,12a,13,13a-decahydro-8H-isoquino[2,1-g][1,6]naphthyridine, prepared, for example, as shown in Preparation 10B, and following the procedures of Preparations 11A and 11B above, the following compound of formula (XIV) where n is 2 was prepared:

(8aR,12aS,13aS)-3-methoxy-12-(3-aminopropanesulfonyl)5,6,8a,9,10,11,12,12a,13,13a-decahydro-8H-isoquino-[2,1-g] [1,6]naphthyridine as an oil, which was converted to the hydrochloride salt in ethanol/ether mixture by treatment with anhydrous hydrochloric acid, to give (8aR,12aS,13aS)-3-methoxy-12-(3-aminopropanesulfonyl)-5,6,8a,9,10,11,12, 12a,13,13a-decahydro-8H-isoquino-[2,1-g][1,6]-naphthyridine hydrochloride, $[\alpha]_D$ = +26.8° (c = 0.22, $CH_3OH$).

D. Preparation of Formula (XIV) where n is 1, varying A, X and Y

Similarly, replacing (±)-(8aα,11aα,12aα)-3-methoxy-11-(3-chloropropanesulfonyl)-5,6,8,8a,9,10,11,11a,12,12a-decahydrobenzo [a]pyrrolo[2,3-e]quinolizine with compounds of formula (XII) where n is 1, as racemic mixtures or as single enantiomers, and following the procedures of Preparations 11A and 11B above, the following compounds of formula (XIV) where n is 1 are prepared in racemic or optically active form:

(8aα,11aα,12aα)-3-methoxy-11-(aminomethanesulfonyl)-5,6,8,8a,9,10,11,11a,12,12a-decahydrobenzo [a]pyrrolo[2,3-e]quinolizine;

(8aα,11aα,12aα)-3-methoxy-11-(2-aminoethanesulfonyl)-5,6,8,8a,9,10,11,11a,12,12a-decahydrobenzo [a]pyrrolo[2,3-e]quinolizine;

(8aα,11aα,12aα)-3-methoxy-11-(4-aminobutanesulfonyl)-5,6,8,8a,9,10,11,11a,12,12a-decahydrobenzo [a]pyrrolo[2,3-e]quinolizine;

(8aα,11aα,12aα)-3-methoxy-11-(3-amino-2-methylpropanesulfonyl)-5,6,8,8a,9,10,11,11a,12,12a-decahydrobenzo [a]pyrrolo[2,3-e]quinolizine;

(8aα,11aα,12aα)-3-methoxy-11-(3-amino-1,2-dimethylpropanesulfonyl)-5,6,8,8a,9,10,11,11a,12,12a-decahydrobenzo[a]pyrrolo[2,3-e]quinolizine;

(8aα,11aα,12aα)-3-methoxy-11-(6-aminohexanesulfonyl)-5,6,8,8a,9,10,11,11a,12,12a-decahydrobenzo [a]pyrrolo[2,3-e]quinolizine;

(8aα,11aα,12aα)-11-(3-aminopropanesulfonyl)-5,6,8,8a,9,10,11,11a,12,12a-decahydrobenzo[a]pyrrolo-[2,3-e]quinolizine;

(8aα,11aα,12aα)-1-methoxy-11-(3-aminopropanesulfonyl)-5,6,8,8a,9,10,11,11a,12,12a-decahydrobenzo [a]pyrrolo[2,3-e]quinolizine;

(8aα,11aα,12aα)-2-methoxy-11-(3-aminopropanesulfonyl)-5,6,8,8a,9,10,11,11a,12,12a-decahydrobenzo [a]pyrrolo[2,3-e]quinolizine;

(8aα,11aα,12aα)-4-methoxy-11-(3-aminopropanesulfonyl)-5,6,8,8a,9,10,11,11a,12,12a-decahydrobenzo [a]pyrrolo[2,3-e]quinolizine;

(8aα,11aα,12aα)-3-ethoxy-11-(3-aminopropanesulfonyl)-5,6,8,8a,9,10,11,11a,12,12a-decahydrobenzo [a]pyrrolo[2,3-e]quinolizine;

(8aα,11aα,12aα)-3-isobutoxy-11-(3-aminopropanesulfonyl)-5,6,8,8a,9,10,11,11a,12,12a-decahydrobenzo [a]pyrrolo[2,3-e]quinolizine;

(8aα,11aα,12aα)-3-n-hexyloxy-11-(3-aminopropanesulfonyl)-5,6,8,8a,9,10,11,11a,12,12a-decahydrobenzo[a]pyrrolo[2,3-e]quinolizine;

(8aα,11aα,12aα)-3-hydroxy-11-(3-aminopropanesulfonyl)-5,6,8,8a,9,10,11,11a,12,12a-decahydrobenzo [a]pyrrolo[2,3-e]quinolizine;

(8aα,11aα,12aα)-2,3-dihydroxy-11-(3-aminopropanesulfonyl)-5,6,8,8a,9,10,11,11a,12,12a-decahydrobenzo[a]pyrrolo[2,3-e]quinolizine;

(8aα,11aα,12aα)-2,3-dimethoxy-11-(3-aminopropanesulfonyl)-5,6,8,8a,9,10,11,11a,12,12a-decahydrobenzo[a]pyrrolo[2,3-e]quinolizine;

(8aα,11aα,12aα)-1,4-dimethoxy-11-(3-aminopropanesulfonyl)-5,6,8,8a,9,10,11,11a,12,12a-decahydrobenzo[a]pyrrolo[2,3-e]quinolizine;

(8aα,11aα,12aα)-3,4-dimethoxy-11-(3-aminopropanesulfonyl)-5,6,8,8a,9,10,11,11a,12,12a-decahydrobenzo[a]pyrrolo[2,3-e]quinolizine;

(8aα,11aα,12aα)-2,3-diethoxy-11-(3-aminopropanesulfonyl)-5,6,8,8a,9,10,11,11a,12,12a-decahydrobenzo[a]pyrrolo[2,3-e]quinolizine;

(8aα,11aα,12aα)-2,3-di-n-butoxy-11-(3-aminopropanesulfonyl)-5,6,8,8a,9,10,11,11a,12,12a-decahydrobenzo[a]pyrrolo[2,3-e]quinolizine;

(8aα,11aα,12aα)-2,3-dihexyloxy-11-(3-aminopropanesulfonyl)-5,6,8,8a,9,10,11,11a,12,12a-decahydrobenzo[a]pyrrolo[2,3-e]quinolizine;

(8aα,11aα,12aα)-2,3-methylenedioxy-11-(3-aminopropanesulfonyl)-5,6,8,8a,9,10,11,11a,12,12a-decahydrobenzo[a]pyrrolo[2,3-e]quinolizine;

(8aα,11aα,12aα)-2,3-methylenedioxy-11-(aminomethanesulfonyl)-5,6,8,8a,9,10,11,11a,12,12a-decahydrobenzo[a]pyrrolo[2,3-e]quinolizine;

(8aα,11aα,12aα)-2,3-methylenedioxy-11-(2-aminoethanesulfonyl)-5,6,8,8a,9,10,11,11a,12,12a-decahydrobenzo[a]pyrrolo[2,3-e]quinolizine;

(8aα,11aα,12aα)-2,3-methylenedioxy-11-(4-aminobutanesulfonyl)-5,6,8,8a,9,10,11,11a,12,12a-decahydrobenzo[a]pyrrolo[2,3-e]quinolizine;

(8aα,11aα,12aα)-2,3-methylenedioxy-11-(3-amino-2-methylpropanesulfonyl)-5,6,8,8a,9,10,11,11a,12,12a-decahydrobenzo[a]pyrrolo[2,3-e]quinolizine;

(8aα,11aα,12aα)-2,3-methylenedioxy-11-(3-amino-1,2-dimethylpropanesulfonyl)-5,6,8,8a,9,10,11,11a,12,12a-decahydrobenzo[a]pyrrolo[2,3-e]quinolizine;

(8aα,11aα,12aα)-2,3-methylenedioxy-11-(6-aminohexanesulfonyl)-5,6,8,8a,9,10,11,11a,12,12a-decahydrobenzo[a]pyrrolo[2,3-e]quinolizine;

(8aα,11aα,12aα)-1,2-methylenedioxy-11-(3-aminopropanesulfonyl)-5,6,8,8a,9,10,11,11a,12,12a-decahydrobenzo[a]pyrrolo[2,3-e]quinolizine;

(8aα,11aα,12aα)-3,4-methylenedioxy-11-(3-aminopropanesulfonyl)-5,6,8,8a,9,10,11,11a,12,12a-decahydrobenzo[a]pyrrolo[2,3-e]quinolizine;

(8aα,11aα,12aα)-2,3-(ethylene-1,2-dioxy)-11-(3-aminopropanesulfonyl)-5,6,8,8a,9,10,11,11a,12,12a-decahydrobenzo[a]pyrrole[2,3-e]quinolizine;

(8aα,11aα,12aα)-1-methyl-11-(3-aminopropanesulfonyl)-5,6,8,8a,9,10,11,11a,12,12a-decahydrobenzo[a]pyrrolo[2,3-e]quinolizine;

(8aα,11aα,12aα)-2-methyl-11-(3-aminopropanesulfonyl)-5,6,8,8a,9,10,11,11a,12,12a-decahydrobenzo[a]pyrrolo[2,3-e]quinolizine;

(8aα,11aα,12aα)-3-methyl-11-(3-aminopropanesulfonyl)-5,6,8,8a,9,10,11,11a,12,12a-decahydrobenzo[a]pyrrolo[2,3-e]quinolizine;

(8aα,11aα,12aα)-2,3-dimethyl-11-(3-aminopropanesulfonyl)-5,6,8,8a,9,10,11,11a,12,12a-decahydrobenzo[a]pyrrolo[2,3-e]quinolizine;

(8aα,11aα,12aα)-3-ethyl-11-(3-aminopropanesulfonyl)-5,6,8,8a,9,10,11,11a,12,12a-decahydrobenzo[a]pyrrolo[2,3-e]quinolizine;

(8aα,11aα,12aα)-3-isobutyl-11-(3-aminopropanesulfonyl)-5,6,8,8a,9,10,11,11a,12,12a-decahydrobenzo[a]pyrrolo[2,3-e]quinolizine;

(8aα,11aα,12aα)-3-n-hexyl-11-(3-aminopropanesulfonyl)-5,6,8,8a,9,10,11,11a,12,12a-decahydrobenzo[a]pyrrolo[2,3-e]quinolizine;

(8aα,11aα,12aα)-1-chloro-11-(3-aminopropanesulfonyl)-5,6,8,8a,9,10,11,11a,12,12a-decahydrobenzo[a]pyrrolo[2,3-e]quinolizine;

(8aα,11aα,12aα)-2-chloro-11-(3-aminopropanesulfonyl)-5,6,8,8a,9,10,11,11a,12,12a-decahydrobenzo[a]pyrrolo[2,3-e]quinolizine;

(8aα,11aα,12aα)-3-chloro-11-(3-aminopropanesulfonyl)-5,6,8,8a,9,10,11,11a,12,12a-decahydrobenzo[a]pyrrolo[2,3-e]quinolizine;

(8aα,11aα,12aα)-3-bromo-11-(3-aminopropanesulfonyl)-5,6,8,8a,9,10,11,11a,12,12a-decahydrobenzo[a]pyrrolo[2,3-e]quinolizine;

(8aα,11aα,12aα)-2-fluoro-11-(3-aminopropanesulfonyl)-5,6,8,8a,9,10,11,11a,12,12a-decahydrobenzo[a]pyrrolo[2,3-e]quinolizine; and

(8aα,11aα,12aα)-3-fluoro-11-(3-aminopropanesulfonyl)-5,6,8,8a,9,10,11,11a,12,12a-decahydrobenzo[a]pyrrolo[2,3-e]quinolizine.

E. Preparation of Formula (XIV) where n is 2, varying A, X and Y

Similarly, replacing (8aR,12aS,13aS)-3-methoxy-5,6,8a,9,10,11,12,12a,13,13a-decahydro-8H-isoquino[2,1-g][1,6]naphthyridine with compounds of formula (XII) where n is 2, as racemic mixtures or as single enantiomers, and following the procedures of Preparations 11A and 11B above, the following compounds of formula (XIV) where n is 2 are prepared in racemic or optically active form:

(8aα,12aα,13aα)-3-methoxy-12-(aminomethanesulfonyl)-5,6,8a,9,10,11,12,12a,13,13a-decahydro-8H-isoquino[2,1-g][1,6]naphthyridine;

(8aα,12aα,13aα)-3-methoxy-12-(2-aminoethanesulfonyl)-5,6,8a,9,10,11,12,12a,13,13a-decahydro-8H-isoquino[2,1-g][1,6]naphthyridine;

(8aα,12aα,13aα)-3-methoxy-12-(4-aminobutanesulfonyl)-5,6,8a,9,10,11,12,12a,13,13a-decahydro-8H-isoquino[2,1-g][1,6]naphthyridine;

(8aα,12aα,13aα)-3-methoxy-12-(3-amino-2-methylpropanesulfonyl)-5,6,8a,9,10,11,12,12a,13,13a-decahydro-8H-isoquino[2,1-g][1,6]naphthyridine;

(8aα,12aα,13aα)-3-methoxy-12-(3-amino-1,2-dimethylpropanesulfonyl)-5,6,8a,9,10,11,12,12a,13,13a-decahydro-8H-isoquino[2,1-g][1,6]naphthyridine;

(8aα,12aα,13aα)-3-methoxy-12-(6-aminohexanesulfonyl)-5,6,8a,9,10,11,12,12a,13,13a-decahydro-8H-isoquino[2,1-g][1,6]naphthyridine;

(8aα,12aα,13aα)-12-(3-aminopropanesulfonyl)-5,6,8a,9,10,11,12,12a,13,13a-decahydro-8H-isoquino [2,1-g][1,6]naphthyridine;

(8aα,12aα,13aα)-2,3-methylenedioxy-12-(3-aminopropanesulfonyl)-5,6,8a,9,10,11,12,12a,13,13a-decahydro-8H-isoquino[2,1-g][1,6]naphthyridine;

(8aα,12aα,13aα)-2,3-methylenedioxy-12-(aminomethanesulfonyl)-5,6,8a,9,10,11,12,12a,13,13a-decahydro-8H-isoquino[2,1-g][1,6]naphthyridine;

(8aα,12aα,13aα)-2,3-methylenedioxy-12-(2-aminoethanesulfonyl)-5,6,8a,9,10,11,12,12a,13,13a-decahydro-8H-isoquino[2,1-g][1,6]naphthyridine;

(8aα,12aα,13aα)-2,3-methylenedioxy-12-(4-aminobutanesulfonyl)-5,6,8a,9,10,11,12,12a,13,13a-decahydro-8H-isoquino[2,1-g][1,6]naphthyridine;

(8aα,12aα,13aα)-2,3-methylenedioxy-12-(3-amino-2-methylpropanesulfonyl)-5,6,8a,9,10,11,12,12a,13, 13a-decahydro-8H-isoquino[2,1-g][1,6]naphthyridine;

(8aα,12aα,13aα)-2,3-methylenedioxy-12-(3-amino-1,2-dimethylpropanesulfonyl)-5,6,8a,9,10,11,12,12a, 13,13a-decahydro-8H-isoquino[2,1-g][1,6]naphthyridine;

(8aα,12aα,13aα)-2,3-methylenedioxy-12-(6-amino hexanesulfonyl)-5,6,8a,9,10,11,12,12a,13,13a-decahydro-8H-isoquino[2,1-g][1,6]naphthyridine;

(8aα,12aα,13aα)-2,3-dimethoxy-12-(3-aminopropanesulfonyl)-5,6,8a,9,10,11,12,12a,13,13a-decahydro-8H-isoquino[2,1-g][1,6]naphthyridine;

(8aα,12aα,13aα)-1,4-dimethoxy-12-(3-aminopropanesulfonyl)-5,6,8a,9,10,11,12,12a,13,13a-decahydro-8H-isoquino[2,1-g][1,6]naphthyridine;

(8aα,12aα,13aα)-2,3-(ethylene-1,2-dioxy)-12-(3-aminopropanesulfonyl)-5,6,8a,9,10,11,12,12a,13,13a-decahydro-8H-isoquino[2,1-g][1,6]naphthyridine;

(8aα,12aα,13aα)-1-methyl-12-(3-aminopropanesulfonyl)-5,6,8a,9,10,11,12,12a,13,13a-decahydro-8H-isoquino[2,1-g][1,6]naphthyridine;

(8aα,12aα,13aα)-2-methyl-12-(3-aminopropanesulfonyl)-5,6,8a,9,10,11,12,12a,13,13a-decahydro-8H-isoquino[2,1-g][1,6]naphthyridine;

(8aα,12aα,13aα)-3-methyl-12-(3-aminopropanesulfonyl)-5,6,8a,9,10,11,12,12a,13,13a-decahydro-8H-isoquino[2,1-g][1,6]naphthyridine;

(8aα,12aα,13aα)-2,3-dimethyl-12-(3-aminopropanesulfonyl)-5,6,8a,9,10,11,12,12a,13,13a-decahydro-8H-isoquino-[2,1-g][1,6]naphthyridine;

(8aα,12aα,13aα)-3-ethyl-12-(3-aminopropanesulfonyl)-5,6,8a,9,10,11,12,12a,13,13a-decahydro-8H-isoquino-[2,1-g][1,6]naphthyridine;

(8aα,12aα,13aα)-3-isobutyl-12-(3-aminopropanesulfonyl)-5,6,8a,9,10,11,12,12a,13,13a-decahydro-8H-isoquino[2,1-g][1,6]naphthyridine;

(8aα,12aα,13aα)-3-n-hexyl-12-(3-aminopropanesulfonyl)-5,6,8a,9,10,11,12,12a,13,13a-decahydro-8H-isoquino[2,1-g][1,6]naphthyridine;

(8aα,12aα,13aα)-1-methoxy-12-(3-aminopropanesulfonyl)-5,6,8a,9,10,11,12,12a,13,13a-decahydro-8H-isoquino[2,1-g][1,6]naphthyridine;

(8aα,12aα,13aα)-2-methoxy-12-(3-aminopropanesulfonyl)-5,6,8a,9,10,11,12,12a,13,13a-decahydro-8H-isoquino[2,1-g][1,6]naphthyridine;

(8aα,12aα,13aα)-4-methoxy-12-(3-aminopropanesulfonyl)-5,6,8a,9,10,11,12,12a,13,13a-decahydro-8H-isoquino[2,1-g][1,6]naphthyridine;

(8aα,12aα,13aα)-3-methoxy-2-methyl-12-(3-aminopropanesulfonyl)-5,6,8a,9,10,11,12,12a,13,13a-decahydro-8H-isoquino[2,1-g][1,6]naphthyridine;

(8aα,12aα,13aα)-3-ethoxy-12-(3-aminopropanesulfonyl)-5,6,8a,9,10,11,12,12a,13,13a-decahydro-8H-isoquino[2,1-g][1,6]naphthyridine;

(8aα,12aα,13aα)-3-isopropoxy-12-(3-aminopropanesulfonyl)-5,6,8a,9,10,11,12,12a,13,13a-decahydro-8H-isoquino[2,1-g][1,6]naphthyridine;

(8aα,12aα,13aα)-3-isopropoxy-12-(3-aminopropanesulfonyl)-5,6,8a,9,10,11,12,12a,13,13a-decahydro-8H-isoquino[2,1-g][1,6]naphthyridine;

(8aα,12aα,13aα)-3-n-hexyloxy-12-(3-aminopropanesulfonyl)-5,6,8a,9,10,11,12,12a,13,13a-decahydro-8H-isoquino[2,1-g][1,6]naphthyridine;

(8aα,12aα,13aα)-3-hydroxy-12-(3-aminopropanesulfonyl)-5,6,8a,9,10,11,12,12a,13,13a-decahydro-8H-isoquino[2,1-g][1,6]naphthyridine;

(8aα,12aα,13aα)-2,3-dihydroxy-12-(3-aminopropanesulfonyl)-5,6,8a,9,10,11,12,12a,13,13a-decahydro-8H-isoquino[2,1-g][1,6]naphthyridine;

(8aα,12aα,13aα)-1,2-dimethoxy-12-(3-aminopropanesulfonyl)-5,6,8a,9,10,11,12,12a,13,13a-decahydro-8H-isoquino[2,1-g][1,6]naphthyridine;

(8aα,12aα,13aα)-1,4-dimethoxy-12-(3-aminopropanesulfonyl)-5,6,8a,9,10,11,12,12a,13,13a-decahydro-8H-isoquino[2,1-g][1,6]naphthyridine;

(8aα,12aα,13aα)-3,4-dimethoxy-12-(3-aminopropanesulfonyl)-5,6,8a,9,10,11,12,12a,13,13a-decahydro-8H-isoquino[2,1-g][1,6]naphthyridine;

(8aα,12aα,13aα)-2,3-diethoxy-12-(3-aminopropanesulfonyl)-5,6,8a,9,10,11,12,12a,13,13a-decahydro-8H-isoquino[2,1-g][1,6]naphthyridine;

(8aα,12aα,13aα)-2,3-di-n-butoxy-12-(3-aminopropanesulfonyl)-5,6,8a,9,10,11,12,12a,13,13a-decahydro-8H-isoquino[2,1-g][1,6]naphthyridine;

(8aα,12aα,13aα)-1,2-methylenedioxy-12-(3-aminopropanesulfonyl)-5,6,8a,9,10,11,12,12a,13,13a-decahydro-8H-isoquino[2,1-g][1,6]naphthyridine;

(8aα,12aα,13aα)-2-chloro-12-(3-aminopropanesulfonyl)-5,6,8a,9,10,11,12,12a,13,13a-decahydro-8H-isoquino[2,1-g][1,6]naphthyridine;

(8aα,12aα,13aα)-3-chloro-12-(3-aminopropanesulfonyl)-5,6,8a,9,10,11,12,12a,13,13a-decahydro-8H-isoquino[2,1-g][1,6]naphthyridine;

(8aα,12aα,13aα)-4-chloro-12-(3-aminopropanesulfonyl)-5,6,8a,9,10,11,12,12a,13,13a-decahydro-8H-isoquino[2,1-g][1,6]naphthyridine;

(8aα,12aα,13aα)-3-bromo-12-(3-aminopropanesulfonyl)-5,6,8a,9,10,11,12,12a,13,13a-decahydro-8H-isoquino[2,1-g][1,6]naphthyridine;

(8aα,12aα,13aα)-3-fluoro-12-(3-aminopropanesulfonyl)-5,6,8a,9,10,11,12,12a,13,13a-decahydro-8H-isoquino[2,1-g][1,6]naphthyridine; and

(8aα,12aα,13aα)-2-fluoro-12-(3-aminopropanesulfonyl)-5,6,8a,9,10,11,12,12a,13,13a-decahydro-8H-isoquino[2,1-g][1,6]naphthyridine.

## EXAMPLE 1

### Preparation of (±)-(8aα,11aα,12aα)-3-methoxy-11-methanesulfonyl-5,6,8,8a,9,10,11,11a,12,12a-decahydrobenzo[a]pyrrolo[2,3-e]quinolizine and Related Compounds of Formula (1) where R is alkyl, cycloalkyl, phenyl or phenyl lower alkyl and n is 1

A. Preparation of Formula (1) where R is methyl, X is 3-methoxy, Y is hydrogen and n is 1

A mixture of 1.6 g of (±)-(8aα,11aα,12aα)-3-methoxy-5,6,8,8a,9,10,11,11a,12,12a-decahydrobenzo[a]pyrrolo[2,3-e]quinolizine (XI), prepared as shown in preparation 7, and 1 ml of triethylamine in 25 ml of methylene chloride was cooled to 0°C, and 0.4 ml of methanesulfonyl chloride added. The resulting mixture was stirred at 0°C for 30 minutes, then washed with dilute ammonium hydroxide. The methylene chloride layer was separated and the solvent removed under reduced pressure. The residue was purified by silica gel chromatography to afford (±)-(8aα,11aα,12aα)-3-methoxy-11-methanesulfonyl-5,6,8,8a,9,10,11,11a,12,12a-decahydrobenzo[a]pyrrolo[2,3-e]quinolizine, a compound of formula (1) where n is 1 and R is alkyl, as an oil. The oil was dissolved in ethanol and acidified with anhydrous HCl in ether, from which a hydrochloride salt was crystallized, m.p. 253-254°C.

B. Preparation of Formula (1) where R is methyl, X and Y are 2,3-methylenedioxy and n is 1

Similarly, replacing (±)-(8aα,11aα,12aα)-3-methoxy-5,6,8,8a,9,10,11,11a,12,12a-decahydrobenzo[a]pyrrolo[2,3-e]quinolizine with (±)-(8aα,11aα,12aα)-2,3-methylenedioxy-5,6,8,8a,9,10,11,11a,12,12a-decahydrobenzo[a]pyrrolo[2,3-e]quinolizine, prepared, for example, as shown in Preparation 9, and following the procedure in Example 1A above, the following compound of formula (1) where n is 1 was prepared:

(±)-(8aα,11aα,12aα)-2,3-methylenedioxy-11-methanesulfonyl-5,6,8,8a,9,10,11,11a,12,12a-decahydrobenzo[a]pyrrolo[2,3-e]quinolizine hydrochloride, m.p. 258-259°C.

C. Preparation of Formula (1) where n is 1, R is alkyl, cycloalkyl, phenyl or phenyl lower alkyl, varying X and Y

Similarly, optionally replacing (±)-(8aα,11aα,12aα)-3-methoxy-5,6,8,8a,9,10,11,11a,12,12a-decahydrobenzo[a]pyrrolo[2,3-e]quinolizine with compounds of formula (XI), as racemic mixtures or single enantiomers, prepared, for example, as shown in Preparation 9C, and optionally replacing methanesulfonyl chloride with the appropriate compound of formula $ZSO_2R$ where R is lower alkyl, cycloalkyl, optionally substituted phenyl or phenyl lower alkyl and Z is chloro or bromo, and following the procedure in Example 1A above, the following compounds of formula (1) where n is 1 are prepared in racemic or optically active form:

(8aα,11aα,12aα)-3-methoxy-11-(1-ethanesulfonyl)-5,6,8,8a,9,10,11,11a,12,12a-decahydrobenzo[a]pyrrolo-[2,3-e]quinolizine;

(8aα,11aα,12aα)-3-methoxy-11-(1-propanesulfonyl)-5,6,8,8a,9,10,11,11a,12,12a-decahydrobenzo[a]pyrrolo[2,3-e]quinolizine;

(8aα,11aα,12aα)-3-methoxy-11-(2-methylpropanesulfonyl)-5,6,8,8a,9,10,11,11a,12,12a-decahydrobenzo[a]pyrrolo[2,3-e]quinolizine;

(8aα,11aα,12aα)-3-methoxy-11-(1-hexanesulfonyl)-5,6,8,8a,9,10,11,11a,12,12a-decahydrobenzo[a]Pyrrolo[2,3-e]quinolizine;

(8aα,11aα,12aα)-3-methoxy-11-(phenylsulfonyl)-5,6,8,8a,9,10,11,11a,12,12a-decahydrobenzo[a]pyrrolo-[2,3-e]quinolizine;

(8aα,11aα,12aα)-3-methoxy-11-(4-methoxyphenylsulfonyl)-5,6,8,8a,9,10,11,11a,12,12a-decahydrobenzo[a]pyrrolo[2,3-e]quinolizine;

(8aα,11aα,12aα)-3-methoxy-11-(phenylmethylsulfonyl)-5,6,8,8a,9,10,11,11a,12,12a-decahydrobenzo[a] pyrrolo[2,3-e]quinolizine;

(8aα,11aα,12aα)-11-methanesulfonyl-5,6,8,8a,9,10,11,11a,12,12a-decahydrobenzo[a]pyrrolo[2,3-e]quinolizine;

(8aα,11aα,12aα)-2,3-diethoxy-11-methanesulfonyl-5,6,8,8a,9,10,11,11a,12,12a-decahydrobenzo[a]pyrrolo[2,3-e]quinolizine;

(8aα,11aα,12aα)-2,3-di-n-butoxy-11-methanesulfonyl-5,6,8,8a,9,10,11,11a,12,12a-decahydrobenzo[a] pyrrolo[2,3-e]quinolizine;

(8aα,11aα,12aα)-2,3-dihexyloxy-11-methanesulfonyl-5,6,8,8a,9,10,11,11a,12,12a-decahydrobenzo[a] pyrrolo[2,3-e]quinolizine;

(8aα,11aα,12aα)-2,3-methylenedioxy-11-(1-ethanesulfonyl)-5,6,8,8a,9,10,11,11a,12,12a-decahydrobenzo[a]pyrrolo[2,3-e]quinolizine;

(8aα,11aα,12aα)-2.3-methylenedioxy-11-(1-propanesulfonyl)-5,6,8,8a,9,10,11,11a,12,12a-decahydrobenzo[a]pyrrolo[2,3-e]quinolizine;

(8aα,11aα,12aα)-2.3-methylenedioxy-11-(2-methylpropanesulfonyl)-5,6,8,8a,9,10,11,11a,12,12a-decahydrobenzo[a]pyrrolo[2,3-e]quinolizine;

(8aα,11aα,12aα)-2.3-methylenedioxy-11-(1-hexanesulfonyl)-5,6,8,8a,9,10,11,11a,12,12a-decahydrobenzo[a]pyrrolo[2,3-e]quinolizine;

(8aα,11aα,12aα)-2.3-methylenedioxy-11-(phenylsulfonyl)-5,6,8,8a,9,10,11,11a,12,12a-decahydrobenzo [a]pyrrolo[2,3-e]quinolizine;

(8aα,11aα,12aα)-2.3-methylenedioxy-11-(4-methoxyphenylsulfonyl)-5,6,8,8a,9,10,11,11a,12,12a-decahydrobenzo[a]pyrrolo[2,3-e]quinolizine;

(8aα,11aα,12aα)-2.3-methylenedioxy-11-(phenylmethylsulfonyl)-5,6,8,8a,9,10,11,11a,12,12a-decahydrobenzo[a]pyrrolo[2,3-e]quinolizine;

(8aα,11aα,12aα)-1,2-methylenedioxy-11-methanesulfonyl-5,6,8,8a,9,10,11,11a,12,12a-decahydrobenzo [a]pyrrolo[2,3-e]quinolizine;

(8aα,11aα,12aα)-3,4-methylenedioxy-11-methanesulfonyl-5,6,8,8a,9,10,11,11a,12,12a-decahydrobenzo [a]-

(8aα,11aα,12aα)-11-(1-ethanesulfonyl)-5,6,8,8a,9,10,11,11a,12,12a-decahydrobenzo[a]pyrrolo[2,3-e]quinolizine;

(8aα,11aα,12aα)-1-methoxy-11-methanesulfonyl-5,6,8,8a,9,10,11,11a,12,12a-decahydrobenzo[a]pyrrolo[2,3-e]quinolizine;

(8aα,11aα,12aα)-2-methoxy-11-methanesulfonyl-5,6,8,8a,9,10,11,11a,12,12a-decahydrobenzo[a]pyrrolo[2,3-e]quinolizine;

(8aα,11aα,12aα)-4-methoxy-11-methanesulfonyl-5,6,8,8a,9,10,11,11a,12,12a-decahydrobenzo[a]pyrrolo[2,3-e]quinolizine;

(8aα,11aα,12aα)-3-ethoxy-11-methanesulfonyl-5,6,8,8a,9,10,11,11a,12,12a-decahydrobenzo[a]pyrrolo[2,3-e]quinolizine;

(8aα,11aα,12aα)-3-isobutoxy-11-methanesulfonyl-5,6,8,8a,9,10,11,11a,12,12a-decahydrobenzo[a]pyrrolo[2,3-e]quinolizine;

(8aα,11aα,12aα)-3-n-hexyloxy-11-methanesulfonyl-5,6,8,8a,9,10,11,11a,12,12a-decahydrobenzo[a]pyrrolo[2,3-e]quinolizine;

(8aα,11aα,12aα)-3-hydroxy-11-methanesulfonyl-5,6,8,8a,9,10,11,11a,12,12a-decahydrobenzo[a]pyrrolo[2,3-e]quinolizine;

(8aα,11aα,12aα)-2,3-dihydroxy-11-methanesulfonyl-5,6,8,8a,9,10,11,11a,12,12a-decahydrobenzo[a]pyrrolo[2,3-e]quinolizine;

(8aα,11aα,12aα)-2,3-dimethoxy-11-methanesulfonyl-5,6,8,8a,9,10,11,11a,12,12a-decahydrobenzo[a]pyrrolo[2,3-e]quinolizine;

(8aα,11aα,12aα)-1,4-dimethoxy-11-methanesulfonyl-5,6,8,8a,9,10,11,11a,12,12a-decahydrobenzo[a]pyrrolo[2,3-e]quinolizine;

(8aα,11aα,12aα)-3,4-dimethoxy-11-methanesulfonyl-5,6,8,8a,9,10,11,11a,12,12a-decahydrobenzo[a]pyrrolo[2,3-e]quinolizine;

(8aα,11aα,12aα)-2,3-(ethylene-1,2-dioxy)-11- methanesulfonyl- 5,6,8,8a,9,10,11,11a,12,12a- decahydrobenzo[a]pyrrole[2,3-e]quinolizine;

(8aα,11aα,12aα)-1-methyl-11-methanesulfonyl-5,6,8,8a,9,10,11,11a,12,12a-decahydrobenzo[a]pyrrolo[2,3-e]quinolizine;

(8aα,11aα,12aα)-2-methyl-11-methanesulfonyl-5,6,8,8a,9,10,11,11a,12,12a-decahydrobenzo[a]pyrrolo[2,3-e]quinolizine;

(8aα,11aα,12aα)-3-methyl-11-methanesulfonyl-5,6,8,8a,9,10,11,11a,12,12a-decahydrobenzo[a]pyrrolo[2,3-e]quinolizine;

(8aα,11aα,12aα)-2,3-dimethyl-11-methanesulfonyl-5,6,8,8a,9,10,11,11a,12,12a-decahydrobenzo[a]pyrrolo[2,3-e]quinolizine;

(8aα,11aα,12aα)-3-ethyl-11-methanesulfonyl-5,6,8,8a,9,10,11,11a,12,12a-decahydrobenzo[a]pyrrolo[2,3-e]quinolizine;

(8aα,11aα,12aα)-3-isobutyl-11-methanesulfonyl-5,6,8,8a,9,10,11,11a,12,12a-decahydrobenzo[a]pyrrolo[2,3-e]quinolizine;

(8aα,11aα,12aα)-3-n-hexyl-11-methanesulfonyl-5,6,8,8a,9,10,11,11a,12,12a-decahydrobenzo[a]pyrrolo[2,3-e]quinolizine;

(8aα,11aα,12aα)-1-chloro-11-methanesulfonyl-5,6,8,8a,9,10,11,11a,12,12a-decahydrobenzo[a]pyrrolo[2,3-e]quinolizine;

(8aα,11aα,12aα)-2-chloro-11-methanesulfonyl-5,6,8,8a,9,10,11,11a,12,12a-decahydrobenzo[a]pyrrolo[2,3-e]quinolizine;

(8aα,11aα,12aα)-3-chloro-11-methanesulfonyl-5,6,8,8a,9,10,11,11a,12,12a-decahydrobenzo[a]pyrrolo[2,3-e]quinolizine;

(8aα,11aα,12aα)-3-bromo-11-methanesulfonyl-5,6,8,8a,9,10,11,11a,12,12a-decahydrobenzo[a]pyrrolo[2,3-e]quinolizine;

(8aα,11aα,12aα)-2-fluoro-11-methanesulfonyl-5,6,8,8a,9,10,11,11a,12,12a-decahydrobenzo[a]pyrrolo[2,3-e]quinolizine; and

(8aα,11aα,12aα)-3-fluoro-11-methanesulfonyl-5,6,8,8a,9,10,11,11a,12,12a-decahydrobenzo[a]pyrrolo[2,3-e]quinolizine.

EXAMPLE 2

Preparation of (±)-(8aα,11aα,12aα)-3-methoxy-11-[3-(methanesulfonylamino)propanesulfonyl]-5,6,8,8a,9,10,11,11a,12,12a-decahydrobenzo[a]pyrrolo[2,3-e]quinolizine and Related Compounds of Formula (1) where $R^1$is lower alkyl

A. Preparation of Formula (1) where A is propylene, $R^1$is methyl, X is 3-methoxy, Y is hydrogen and n is 1

A mixture of 1.6 g of (±)-(8aα,11aα,12aα)-3-methoxy-11-(3-aminopropanesulfonyl)-5,6,8,8a,9,10,11,11a,12,12a-decahydiobenzo[a]pyrrolo[2,3-e]quinolizine (XIV), prepared as shown in preparation 9, and 1 ml of triethylamine in 25 ml of methylene chloride was cooled to 0°C, and 0.4 ml of methanesulfonyl chloride added. The resulting mixture was stirred at 0°C for 30 minutes, then washed with dilute ammonium hydroxide. The methylene chloride layer was separated and the solvent removed under reduced pressure. The residue was purified by silica gel chromatography to afford (±)-(8aα,11aα,12aα)-3-methoxy-11-[3-(methanesulfonylamino)-propanesulfonyl]-5,6,8,8a,9,10,11,11a,12,12a-decahydrobenzo[a]pyrrolo[2,3-e]quinolizine, a compound of formula (1) where n is 1 and $R^1$ is alkyl, as an oil. The oil was crystallized from a mixture of ethyl acetate and hexane to form a solid, mp 145-146°C.

B. Preparation of Formula (1) where A is propylene, $R^1$is methyl, X is 3-methoxy, Y is hydrogen and n is 2

Similarly, replacing (±)-(8aα,11aα,12aα)-3-methoxy-11-(3-aminopropanesulfonyl)-5,6,8,8a,9,10,11,11a,12,12a-decahydrobenzo [a]pyrrolo[2,3-e]quinolizine with (8aR,12aS,13aS)-3-methoxy-12-(3-aminopropanesulfonyl)-5,6,8a,9,10,11,12,12a,13,13a-decahydro-8H-isoquino[2,1-g][1,6]naphthyridine, prepared, for example, as shown in Preparation 11C, and following the procedure of Example 2A above, the following compound of formula (1) where n is 2 was prepared, and converted to its maleate salt:

(8aR,12aS,13aS)-3-methoxy-12-[3-(methanesulfonylamino)-propanesulfonyl]-5,6,8a,9,10,11,12,12a,13,13a-decahydro-8H-isoquino[2,1-g][1,6]naphthyridine maleate, mp 108-109°C, $[\alpha]_D$ = +9.9° (c = 1.1, $CH_3OH$).

C. Preparation of Formula (1) where $R^1$is lower alkyl, varying A, X, Y and n

Similarly, optionally replacing (±)-(8aα,11aα,12aα)-3-methoxy-11-(3-aminopropanesulfonyl)-5,6,8,8a,9,10,11,11a, 12,12a-decahydrobenzo[a]pyrrolo[2,3-e]quinolizine with compounds of formula (XIV), as racemic mixtures or single enantiomers, where n is 1 or 2, prepared, for example, as shown in Preparation 11D and 11E, and optionally replacing methanesulfonyl chloride with compounds of formula $ZSO_2R^1$ where $R^1$ is lower alkyl and Z is chloro or bromo, and following the procedure in Example 2A above, the following compounds of formula (1) where n is 1 or 2 and $R^1$ is lower alkyl are prepared in racemic or optically active form:

(8aα,11aα,12aα)-3-methoxy-11-[3-(ethanesulfonylamino)-propanesulfonyl]-5,6,8,8a,9,10,11,11a,12,12a-decahydrobenzo[a]pyrrolo[2,3-e]quinolizine;

(8aα,11aα,12aα)-3-methoxy-11-[3-(propanesulfonylamino)-propanesulfonyl]-5,6,8,8a,9,10,11,11a,12,12a-decahydrobenzo[a]pyrrolo[2,3-e]quinolizine;

(8aα,11aα,12aα)-3-methoxy-11-[3-(hexanesulfonylamino)-propanesulfonyl]-5,6,8,8a,9,10,11,11a,12,12a-decahydrobenzo[a]pyrrolo[2,3-e]quinolizine;

(8aα,11aα,12aα)-3-methoxy-11-(methanesulfonylamino-methanesulfonyl)-5,6,8,8a,9,10,11,11a,12,12a-decahydrobenzo[a]pyrrolo[2,3-e]quinolizine;

(8aα,11aα,12aα)-3-methoxy-11-[2-(methanesulfonylamino)-ethanesulfonyl]-5,6,8,8a,9,10,11,11a,12,12a-decahydrobenzo[a]pyrrolo[2,3-e]quinolizine;

(8aα,11aα,12aα)-3-methoxy-11-[4-(methanesulfonylamino)-butanesulfonyl]-5,6,8,8a,9,10,11,11a,12,12a-decahydrobenzo[a]pyrrolo[2,3-e]quinolizine;

(8aα,11aα,12aα)-3-methoxy-11-[3-(methanesulfonylamino)-2-methylpropanesulfonyl]-5,6,8,8a,9,10,11,11a,12,12a-decahydrobenzo[a]pyrrolo[2,3-e]quinolizine;

(8aα,11aα,12aα)-3-methoxy-11-[3-(methanesulfonylamino)-1,2-dimethylpropanesulfonyl]-5,6,8,8a,9,10,11,11a,12,12a-decahydrobenzo[a]pyrrolo[2,3-e]quinolizine;

(8aα,11aα,12aα)-3-methoxy-11-[6-(methanesulfonylamino)-hexanesulfonyl]-5,6,8,8a,9,10,11,11a,12,12a-decahydrobenzo[a]pyrrolo[2,3-e]quinolizine;

(8aα,11aα,12aα)-11-[3-(methanesulfonylamino)-propanesulfonyl)-5,6,8,8a,9,10,11,11a,12,12a-decahydrobenzo[a]pyrrolo[2,3-e]quinolizine;

(8aα,11aα,12aα)-1-methoxy-11-[3-(methanesulfonylamino)-propanesulfonyl]-5,6,8,8a,9,10,11,11a,12,12a-decahydrobenzo[a]pyrrolo[2,3-e]quinolizine;

(8aα,11aα,12aα)-2-methoxy-11-[3-(methanesulfonylamino)-propanesulfonyl]-5,6,8,8a,9,10,11,11a,12,12a-decahydrobenzo[a]pyrrolo[2,3-e]quinolizine;

(8aα,11aα,12aα)-4-methoxy-11-[3-(methanesulfonylamino)-propanesulfonyl]-5,6,8,8a,9,10,11,11a,12,12a-decahydrobenzo[a]pyrrolo[2,3-e]quinolizine;

(8aα,11aα,12aα)-3-ethoxy-11-[3-(methanesulfonylamino)-propanesulfonyl]-5,6,8,8a,9,10,11,11a,12,12a-decahydrobenzo[a]pyrrolo[2,3-e]quinolizine;

(8aα,11aα,12aα)-3-isobutoxy-11-[3-(methanesulfonylamino)-propanesulfonyl]-5,6,8,8a,9,10,11,11a,12,12a-decahydrobenzo[a]pyrrolo[2,3-e]quinolizine;

(8aα,11aα,12aα)-3-n-hexyloxy-11-[3-(methanesulfonylamino)propanesulfonyl]-5,6,8,8a,9,10,11,11a,12,12a-decahydrobenzo-[a]pyrrolo[2,3-e]quinolizine;

(8aα,11aα,12aα)-3-hydroxy-11-[3-(methanesulfonylamino)-propanesulfonyl]-5,6,8,8a,9,10,11,11a,12,12a-decahydrobenzo[a]pyrrolo[2,3-e]quinolizine;

(8aα,11aα,12aα)-2,3-dihydroxy-11-[3-(methanesulfonylamino)propanesulfonyl]-5,6,8,8a,9,10,11,11a,12,12a-decahydrobenzo[a]pyrrolo[2,3-e]quinolizine;

(8aα,11aα,12aα)-2,3-dimethoxy-11-[3-(methanesulfonylamino)propanesulfonyl]-5,6,8,8a,9,10,11,11a,12,12a-decahydrobenzo[a]pyrrolo[2,3-e]quinolizine;

(8aα,11aα,12aα)-1,4-dimethoxy-11-13-(methanesulfonylamino)propanesulfonyl]-5,6,8,8a,9,10,11,11a,12,12a-decahydrobenzo [a]pyrrolo[2,3-e]quinolizine;

(8aα,11aα,12aα)-3,4-dimethoxy-11-[3-(methanesulfonylamino)propanesulfonyl]-5,6,8,8a,9,10,11,11a,12,12a-decahydrobenzo[a]pyrrolo[2,3-e]quinolizine;

(8aα,11aα,12aα)-2,3-diethoxy-11-[3-(methanesulfonylamino)propanesulfonyl]-5,6,8,8a,9,10,11,11a,12,12a-decahydrobenzo[a]pyrrolo[2,3-e]quinolizine;

(8aα,11aα,12aα)-2,3-di-n-butoxy-11-[3-(methanesulfonylamino)propanesulfonyl]-5,6,8,8a,9,10,11,11a,12,12a-decahydrobenzo[a]pyrrolo[2,3-e]quinolizine;

(8aα,11aα,12aα)-2,3-dihexyloxy-11-[3-(methanesulfonylamino)propanesulfonyl]-5,6,8,8a,9,10,11,11a,12,12a-decahydrobenzo[a]pyrrolo[2,3-e]quinolizine;

(8aα,11aα,12aα)-2,3-methylenedioxy-11-[3-(methanesulfonylamino)propanesulfonyl]-5,6,8,8a,9,10,11,11a,12,12a-decahydrobenzo[a]pyrrolo[2,3-e]quinolizine;

(8aα,11aα,12aα)-2,3-methylenedioxy-11-[3-(ethanesulfonylamino)propanesulfonyl]-5,6,8,8a,9,10,11,11a,12,12a-decahydrobenzo[a]pyrrolo[2,3-e]quinolizine;

(8aα,11aα,12aα)-2,3-methylenedioxy-11-[3-(propanesulfonylamino)propanesulfonyl]-5,6,8,8a,9,10,11,11a,12,12a-decahydrobenzo[a]pyrrolo[2,3-e]quinolizine;

(8aα,11aα,12aα)-2,3-methylenedioxy-11-[3-(hexanesulfonylamino)propanesulfonyl]-5,6,8,8a,9,10,11,11a,12,12a-decahydrobenzo[a]pyrrolo[2,3-e]quinolizine;

(8aα,11aα,12aα)-2,3-methylenedioxy-11-[(methanesulfonylamino)methanesulfonyl]-5,6,8,8a,9,10,11,11a,12,12a-decahydrobenzo[a]pyrrolo[2,3-e]quinolizine;

(8aα,11aα,12aα)-2,3-methylenedioxy-11-[2-(methanesulfonylamino)ethanesulfonyl]-5,6,8,8a,9,10,11,11a,12,12a-decahydrobenzo[a]pyrrolo[2,3-e]quinolizine;

(8aα,11aα,12aα)-2,3-methylenedioxy-11-[4-(methanesulfonylamino)butanesulfonyl]-5,6,8,8a,9,10,11,11a,12,12a-decahydrobenzo[a]pyrrolo[2,3-e]quinolizine;

(8aα,11aα,12aα)-2,3-methylenedioxy-11-[3-(methanesulfonylamino)-2-methylpropanesulfonyl]-5,6,8,8a,9,10,11,11a,12,12a-decahydrobenzo[a]pyrrolo[2,3-e]quinolizine;

(8aα,11aα,12aα)-2,3-methylenedioxy-11-[3-(methanesulfonylamino)-1,2-dimethylpropanesulfonyl]-5,6,8,8a,9,10,11,11a,12,12a-decahydrobenzoa]pyrrolo[2,3-e]quinolizine;

(8aα,11aα,12aα)-2,3-methylenedioxy-11-[6-(methanesulfonylamino)hexanesulfonyl]-5,6,8,8a,9,10,11,11a,12,12a-decahydrobenzo[a]pyrrolo[2,3-e]quinolizine;

(8aα,11aα,12aα)-1,2-methylenedioxy-11-[3-(methanesulfonylamino)propanesulfonyl]-5,6,8,8a,9,10,11,11a,12,12a-decahydrobenzo[a]pyrrolo[2,3-e]quinolizine;

(8aα,11aα,12aα)-3,4-methylenedioxy-11-[3-(methanesulfonylamino)propanesulfonyl]-5,6,8,8a,9,10,11,11a,12,12a-decahydrobenzo[a]pyrrolo[2,3-e]quinolizine;

(8aα,11aα,12aα)-2,3-(ethylene-1,2-dioxy)-11-[3-(methanesulfonylamino)propanesulfonyl]-5,6,8,8a,9,10,11,11a,12,12a-decahydrobenzo[a]pyrrole[2,3-e]quinolizine;

(8aα,11aα,12aα)-1-methyl-11-[3-(methanesulfonylamino)propanesulfonyl]-5,6,8,8a,9,10,11,11a,12,12a-decahydrobenzo[a]pyrrolo[2,3-e]quinolizine;

(8aα,11aα,12aα)-2-methyl-11-[3-(methanesulfonylamino)propanesulfonyl]-5,6,8,8a,9,10,11,11a,12,12a-decahydrobenzo[a]pyrrolo[2,3-e]quinolizine;

(8aα,11aα,12aα)-3-methyl-11-[3-(methanesulfonylamino)propanesulfonyl]-5,6,8,8a,9,10,11,11a,12,12a-decahydrobenzo[a]pyrrolo[2,3-e]quinolizine;

(8aα,11aα,12aα)-2,3-dimethyl-11-[3-(methanesulfonylamino)propanesulfonyl]-5,6,8,8a,9,10,11,11a,12,12a-decahydrobenzo[a]pyrrolo[2,3-e]quinolizine;

(8aα,11aα,12aα)-3-ethyl-11-[3-(methanesulfonylamino)propanesulfonyl]-5,6,8,8a,9,10,11,11a,12,12a-decahydrobenzo[a]pyrrolo[2,3-e]quinolizine;

(8aα,11aα,12aα)-3-isobutyl-11-[3-(methanesulfonylamino)-propanesulfonyl]-5,6,8,8a,9,10,11,11a,12,12a-decahydrobenzo[a]pyrrolo[2,3-e]quinolizine;

(8aα,11aα,12aα)-3-n-hexyl-11-[3-(methanesulfonylamino)propanesulfonyl]-5,6,8,8a,9,10,11,11a,12,12a-decahydrobenzo[a]pyrrolo[2,3-e]quinolizine;

(8aα,11aα,12aα)-1-chloro-11-[3-(methanesulfonylamino)propanesulfonyl]-5,6,8,8a,9,10,11,11a,12,12a-decahydrobenzo[a]pyrrolo[2,3-e]quinolizine;

(8aα,11aα,12aα)-2-chloro-11-[3-(methanesulfonylamino)propanesulfonyl]-5,6,8,8a,9,10,11,11a,12,12a-decahydrobenzo[a]pyrrolo[2,3-e]quinolizine;

(8aα,11aα,12aα)-3-chloro-11-[3-(methanesulfonylamino)propanesulfonyl]-5,6,8,8a,9,10,11,11a,12,12a-decahydrobenzo[a]pyrrolo[2,3-e]quinolizine;

(8aα,11aα,12aα)-3-bromo-11-[3-(methanesulfonylamino)propanesulfonyl]-5,6,8,8a,9,10,11,11a,12,12a-decahydrobenzo[a]pyrrolo[2,3-e]quinolizine;

(8aα,11aα,12aα)-2-fluoro-11-[3-(methanesulfonylamino)propanesulfonyl]-5,6,8,8a,9,10,11,11a,12,12a-decahydrobenzo[a]pyrrolo[2,3-e]quinolizine;

(8aα,11aα,12aα)-3-fluoro-11-[3-(methanesulfonylamino)propanesulfonyl]-5,6,8,8a,9,10,11,11a,12,12a-decahydrobenzo[a]pyrrolo[2,3-e]quinolizine;

(8aα,12aα,13aα)-3-methoxy-12-13-(ethanesulfonylamino)propanesulfonyl]-5,6,8a,9,10,11,12,12a,13,13a-decahydro-8H-isoquino[2,1-g][1,6]naphthyridine;

(8aα,12aα,13aα)-3-methoxy-12-[3-(propanesulfonylamino)propanesulfonyl]-5,6,8a,9,10,11,12,12a,13,13a-decahydro-8H-isoquino[2,1-g][1,6]naphthyridine;

(8aα,12aα,13aα)-3-methoxy-12-[3-(hexanesulfonylamino)propanesulfonyl]-5,6,8a,9,10,11,12,12a,13,13a-decahydro-8H-isoquino[2,1-g][1,6]naphthyridine;

(8aα,12aα,13aα)-3-methoxy-12-(methanesulfonylaminomethanesulfonyl)-5,6,8a,9,10,11,12,12a,13,13a-decahydro-8H-isoquino[2,1-g][1,6]naphthyridine;

(8aα,12aα,13aα)-3-methoxy-12-[2-(methanesulfonylamino)ethanesulfonyl]-5,6,8a,9,10,11,12,12a,13,13a-decahydro-8H-isoquino[2,1-g][1,6]naphthyridine;

(8aα,12aα,13aα)-3-methoxy-12-[4-(methanesulfonylamino)butanesulfonyl]-5,6,8a,9,10,11,12,12a,13,13a-decahydro-8H-isoquino[2,1-g][1,6]naphthyridine;

(8aα,12aα,13aα)-3-methoxy-12-[3-(methanesulfonylamino)-2-methylpropanesulfonyl]-5,6,8a,9,10,11,12,12a,13,13a-decahydro-8H-isoquino[2,1-g][1,6]naphthyridine;

(8aα,12aα,13aα)-3-methoxy-12-[3-(methanesulfonylamino)-1,2-dimethylpropanesulfonyl]-5,6,8a,9,10,11,12,12a,13,13a-decahydro-8H-isoquino[2,1-g][1,6]naphthyridine;

(8aα,12aα,13aα)-3-methoxy-12-[6-(methanesulfonylamino)hexanesulfonyl]-5,6,8a,9,10,11,12,12a,13,13a-decahydro-8H-isoquino[2,1-g][1,6]naphthyridine;

(8aα,12aα,13aα)-12-[3-(methanesulfonylamino)propanesulfonyl)-5,6,8a,9,10,11,12,12a,13,13a-decahydro-8H-isoquino[2,1-g][1,6]naphthyridine;

(8aα,12aα,13aα)-2,3-methylenedioxy-12-[3-(methanesulfonylamino)propanesulfonyl]-5,6,8a,9,10,11,12,12a,13,13a-decahydro-8H-isoquino[2,1-g][1,6]naphthyridine;

(8aα,12aα,13aα)-2,3-methylenedioxy-12-[3-(ethanesulfonylamino)propanesulfonyl]-5,6,8a,9,10,11,12,12a,13,13a-decahydro-8H-isoquino[2,1-g][1,6]naphthyridine;

(8aα,12aα,13aα)-2,3-methylenedioxy-12-[3-(propanesulfonylamino)propanesulfonyl]-5,6,8a,9,10,11,12,12a,13,13a-decahydro-8H-isoquino[2,1-g][1,6]naphthyridine;

(8aα,12aα,13aα)-2,3-methylenedioxy-12-[3-(hexanesulfonylamino)propanesulfonyl]-5,6,8a,9,10,11,12,12a,13,13a-decahydro-8H-isoquino[2,1-g][1,6]naphthyridine;

(8aα,12aα,13aα)-2,3-methylenedioxy-12-(methanesulfonylaminomethanesulfonyl)-5,6,8a,9,10,11,12,12a,13,13a-decahydro-8H-isoquino[2,1-g][1,6]naphthyridine;

(8aα,12aα,13aα)-2,3-methylenedioxy-12-[2-(methanesulfonylamino)ethanesulfonyl]-5,6,8a,9,10,11,12,12a,13,13a-decahydro-8H-isoquino[2,1-g][1,6]naphthyridine;

(8aα,12aα,13aα)-2,3-methylenedioxy-12-[4-(methanesulfonylamino)butanesulfonyl]-5,6,8a,9,10,11,12,12a,13,13a-decahydro-8H-isoquino[2,1-g][1,6]naphthyridine;

(8aα,12aα,13aα)-2,3-methylenedioxy-12-[3-(methanesulfonylamino)-2-methylpropanesulfonyl]-5,6,8a,9,10,11,12,12a,13,13a-decahydro-8H-isoquino[2,1-g][1,6]naphthyridine;

(8aα,12aα,13aα)-2,3-methylenedioxy-12-[3-(methanesulfonylamino)-1,2-dimethylpropanesulfonyl]-5,6,8a,9,10,11,12,12a,13,13a-decahydro-8H-isoquino[2,1-g][1,6]naphthyridine;

(8aα,12aα,13aα)-2,3-methylenedioxy-12-[6-(methanesulfonylamino)hexanesulfonyl]-5,6,8a,9,10,11,12,12a,13,13a-decahydro-8H-isoquino[2,1-g][1,6]naphthyridine;

(8aα,12aα,13aα)-2,3-dimethoxy-12-[3-(methanesulfonylamino)propanesulfonyl]-5,6,8a,9,10,11,12,12a,13,13a-decahydro-8H-isoquino[2,1-g][1,6]naphthyridine;

(8aα,12aα,13aα)-1,4-dimethoxy-12-[3-(methanesulfonylamino)propanesulfonyl]-5,6,8a,9,10,11,12,12a,13,13a-decahydro-8H-isoquino[2,1-g][1,6]naphthyridine;

(8aα,12aα,13aα)-2,3-(ethylene-1,2-dioxy)-12-[3-(methane-sulfonylamino)propanesulfonyl]-5,6,8a,9,10,11,12,12a,13,13a-decahydro-8H-isoquino[2,1-g][1,6]naphthyridine;

(8aα,12aα,13aα)-1-methyl-12-[3-(methanesulfonylamino)propanesulfonyl]-5,6,8a,9,10,11,12,12a,13,13a-decahydro-8H-isoquino[2,1-g][1,6]naphthyridine;

(8aα,12aα,13aα)-2-methyl-12-[3-(methanesulfonylamino)propanesulfonyl]-5,6,8a,9,10,11,12,12a,13,13a-decahydro-8H-isoquino[2,1-g][1,6]naphthyridine;

(8aα,12aα,13aα)-3-methyl-12-[3-(methanesulfonylamino)propanesulfonyl]-5,6,8a,9,10,11,12,12a,13,13a-decahydro-8H-isoquino[2,1-g][1,6]naphthyridine;

(8aα,12aα,13aα)-2,3-dimethyl-12-[3-(methanesulfonylamino)propanesulfonyl]-5,6,8a,9,10,11,12,12a,13,13a-decahydro-8H-isoquino-[2,1-g][1,6]naphthyridine;

(8aα,12aα,13aα)-3-ethyl-12-[3-(methanesulfonylamino)propanesulfonyl]-5,6,8a,9,10,11,12,12a,13,13a-decahydro-8H-isoquino[2,1-g][1,6]naphthyridine;

(8aα,12aα,13aα)-3-isobutyl-12-[3-(methanesulfonylamino)-propanesulfonyl]-5,6,8a,9,10,11,12,12a,13,13a-decahydro-8H-isoquino[2,1-g][1,6]naphthyridine;

(8aα,12aα,13aα)-3-n-hexyl-12-[3-(methanesulfonylamino)-propanesulfonyl]-5,6,8a,9,10,11,12,12a,13,13a-decahydro-8H-isoquino[2,1-g][1,6]naphthyridine;

(8aα,12aα,13aα)-1-methoxy-12-[3-(methanesulfonylamino)propanesulfonyl]-5,6,8a,9,10,11,12,12a,13,13a-decahydro-8H-isoquino[2,1-g][1,6]naphthyridine;

(8aα,12aα,13aα)-2-methoxy-12-[3-(methanesulfonylamino)propanesulfonyl]-5,6,8a,9,10,11,12,12a,13,13a-decahydro-8H-isoquino[2,1-g][1,6]naphthyridine;

(8aα,12aα,13aα)-4-methoxy-12-[3-(methanesulfonylamino)propanesulfonyl]-5,6,8a,9,10,11,12,12a,13,13a-decahydro-8H-isoquino[2,1-g][1,6]naphthyridine;

(8aα,12aα,13aα)-3-methoxy-2-methyl-12-[3-(methanesulfonylamino)propanesulfonyl]-5,6,8a,9,10,11,12,12a,13,13a-decahydro-8H-isoquino[2,1-g][1,6]naphthyridine;

(8aα,12aα,13aα)-3-ethoxy-12-[3-(methanesulfonylamino)propanesulfonyl]-5,6,8a,9,10,11,12,12a,13,13a-decahydro-8H-isoquino[2,1-g][1,6]naphthyridine;

(8aα,12aα,13aα)-3-isopropoxy-12-[3-(methanesulfonylamino)propanesulfonyl]-5,6,8a,9,10,11,12,12a,13,13a-decahydro-8H-isoquino[2,1-g][1,6]naphthyridine;

(8aα,12aα,13aα)-3-isopropoxy-12-[3-(methanesulfonylamino)propanesulfonyl]-5,6,8a,9,10,11,12,12a,13,13a-decahydro-8H-isoquino[2,1-g][1,6]naphthyridine;

(8aα,12aα,13aα)-3-n-hexyloxy-12-[3-(methanesulfonylamino)propanesulfonyl]-5,6,8a,9,10,11,12,12a,13,13a-decahydro-8H-isoquino[2,1-g][1,6]naphthyridine;

(8aα,12aα,13aα)-3-hydroxy-12-[3-(methanesulfonylamino)propanesulfonyl]-5,6,8a,9,10,11,12,12a,13,13a-decahydro-8H-isoquino[2,1-g][1,6]naphthyridine;

(8aα,12aα,13aα)-2,3-dihydroxy-12-[3-(methanesulfonylamino)propanesulfonyl]-5,6,8a,9,10,11,12,12a,13,13a-decahydro-8H-isoquino[2,1-g][1,6]naphthyridine;

(8aα,12aα,13aα)-1,2-dimethoxy-12-[3-(methanesulfonylamino)-propanesulfonyl]-5,6,8a,9,10,11,12,12a,13,13a-decahydro-8H-isoquino[2,1-g][1,6]naphthyridine;

(8aα,12aα,13aα)-1,4-dimethoxy-12-[3-(methanesulfonylamino)propanesulfonyl]-5,6,8a,9,10,11,12,12a,13,13a-decahydro-8H-isoquino[2,1-g][1,6]naphthyridine;

(8aα,12aα,13aα)-3,4-dimethoxy-12-[3-(methanesulfonylamino)propanesulfonyl]-5,6,8a,9,10,11,12,12a,13,13a-decahydro-8H-isoquino[2,1-g][1,6]naphthyridine;

(8aα,12aα,13aα)-2,3-diethoxy-12-[3-(methanesulfonylamino)propanesulfonyl]-5,6,8a,9,10,11,12,12a,13,13a-decahydro-8H-isoquino[2,1-g][1,6]naphthyridine;

(8aα,12aα,13aα)-2,3-di-n-butoxy-12-[3-(methanesulfonylamino)propanesulfonyl]-5,6,8a,9,10,11,12,12a,13,13a-decahydro-8H-isoquino[2,1-g][1,6]naphthyridine;

(8aα,12aα,13aα)-1,2-methylenedioxy-12-(3-(methanesulfonylamino)propanesulfonyl]-5,6,8a,9,10,11,12,12a,13,13a-decahydro-8H-isoquino[2,1-g][1,6]naphthyridine;

(8aα,12aα,13aα)-2-chloro-12-[3-(methanesulfonylamino)propanesulfonyl]-5,6,8a,9,10,11,12,12a,13,13a-decahydro-8H-isoquino[2,1-g][1,6]naphthyridine;

(8aα,12aα,13aα)-3-chloro-12-[3-(methanesulfonylamino)propanesulfonyl]-5,6,8a,9,10,11,12,12a,13,13a-decahydro-8H-isoquino[2,1-g][1,6]naphthyridine;

(8aα,12aα,13aα)-4-chloro-12-[3-(methanesulfonylamino)propanesulfonyl]-5,6,8a,9,10,11,12,12a,13,13a-decahydro-8H-isoquino[2,1-g][1,6]naphthyridine;

(8aα,12aα,13aα)-3-bromo-12-[3-(methanesulfonylamino)propanesulfonyl]-5,6,8a,9,10,11,12,12a,13,13a-decahydro-8H-isoquino[2,1-g][1,6]naphthyridine;

(8aα,12aα,13aα)-3-fluoro-12-[3-(methanesulfonylamino)propanesulfonyl]-5,6,8a,9,10,11,12,12a,13,13a-decahydro-8H-isoquino[2,1-g][1,6]naphthyridine; and

(8aα,12aα,13aα)-2-fluoro-12-[3-(methanesulfonylamino)propanesulfonyl]-5,6,8a,9,10,11,12,12a,13,13a-decahydro-8H-isoquino[2,1-g][1,6]naphthyridine.

## EXAMPLE 3

### Preparation of (±)-(8aα,11aα,12aα)-3-methoxy-11-[3-(dimethylaminosulfonylamino)propanesulfonyl]-5,6,8,8a,9,10,11,11a,12,12a-decahydrobenzo[a]pyrrolo[2,3-e]quinolizine and Related Compounds of Formula (1) where $R^1$ is $-NR^2R^3$

A. Preparation of Formula (1) where A is propylene, $R^2$ and $R^3$ are methyl, X is 3-methoxy, Y is hydrogen and n is 1

A mixture of 1.6 g of (±)-(8aα,11aα,12aα)-3-methoxy-11-(3-aminopropanesulfonyl)-5,6,8,8a,9,10,11,11a,12,12a-decahydrobenzo [a]pyrrolo[2,3-e]quinolizine (XIV where n is 1), prepared as shown in Preparation 9, 1 ml of triethylamine and 0.5 ml of dimethylaminosulfonyl chloride in 25 ml of methylene chloride was stirred at 25°C for 18 hours. The methylene chloride layer was washed with aqueous ammonium hydroxide, the organic layer separated and the solvent removed under reduced pressure. The residue was purified by silica gel chromatography to afford (±)-(8aα,11aα,12aα)-3-methoxy-11-[3-(N,N-dimethylaminosulfonylamino)propanesulfonyl]-5,6,8,8a,9,10,11,11a,12,12a-decahydrobenzo[a]pyrrolo[2,3-e]quinolizine, a compound of formula (1) where n is 1 and $R^1$ is $-NR^2R^3$, as an oil. The oil was crystallized from a mixture of ethyl acetate and hexane to form a solid, mp 150-151°C.

B. Preparation of Formula (1) where A is propylene, $R^2$ and $R^3$ are methyl, X is 3-methoxy, Y is hydrogen and n is 2

Similarly, replacing (±)-(8aα,11aα,12aα)-3-methoxy-11(3-aminopropanesulfonyl)-5,6,8,8a,9,10,11,11a,12,12a-decahydrobenzo [a]pyrrolo[2,3-e]quinolizine with (8aR,12aS,13aS)-3-methoxy-12-(3-aminopropanesulfonyl)-5,6,8a,9,10,11,12,12a,13,13a-decahydro-8H-isoquino[2,1-g][1,6]naphthyridine, prepared, for example, as shown in Preparation 11C, and following the procedure in Example 3A above, the following compound of formula (1) where n is 2 was prepared, and converted to its maleate salt:

(8aR,12aS,13aS)-3-methoxy-12-[3-(N,N-dimethylaminosulfonylamino)propanesulfonyl]-5,6,8a,9,10,11,12,12a,13,13a-decahydro-8H-isoquino[2,1-g][1,6]naphthyridine maleate, mp 165-167°C, $[\alpha]_D$ = +17.18° (c = 1.35, $CH_3OH$).

C. Preparation of Formula (1) where $R^1$ is $-NR^2R^3$, varying A, X, Y, and n

Similarly, optionally replacing (±)-(8aα,11aα,12aα)-3-methoxy-11-(3-aminopropanesulfonyl)-5,6,8,8a,9,10,11,11a,12,12a-decahydrobenzo[a]pyrrolo[2,3-e]-quinolizine with compounds of formula (XIV) where n is 1 or 2, as racemic mixtures or single enantiomers, prepared, for example, as shown in Preparation 11D and 11E, and optionally replacing dimethylaminosulfonyl chloride with compounds of formula $ZSO_2R^1$ where $R^1$ is $-NR^2R^3$ and Z is chloro or bromo, and following the procedure in Example 3A above, the following compounds of formula (1) where n is 1 or 2 and $R^1$ is $-NR^2R^3$ are prepared as racemic mixtures or single enantiomers:

(8aα,11aα,12aα)-3-methoxy-11-[3-(N-methyl-N-ethylaminosulfonylamino)propanesulfonyl]-5,6,8,8a,9, 10,11,11a,12,12a-decahydrobenzo[a]pyrrolo[2,3-e]quinolizine;

(8aα,11aα,12aα)-3-methoxy-11-[3-(N,N-diethylaminosulfonylamino)propanesulfonyl]-5,6,8,8a,9,10,11,11a,12,12a-decahydrobenzo[a]pyrrolo[2,3-e]quinolizine;

(8aα,11aα,12aα)-3-methoxy-11-[3-(N,N-dihexylaminosulfonylamino)propanesulfonyl]-5,6,8,8a,9,10,11,11a,12,12a-decahydrobenzo[a]pyrrolo[2,3-e]quinolizine;

(8aα,11aα,12aα)-3-methoxy-11-[3-(cyclohexylaminosulfonylamino)propanesulfonyl]-5,6,8,8a,9,10,11,11a,12,12a-decahydrobenzo[a]pyrrolo[2,3-e]quinolizine;

(8aα,11aα,12aα)-11-[3-(N,N-dimethylaminosulfonylamino)propanesulfonyl)-5,6,8,8a,9,10,11,11a,12,12a-decahydrobenzo[a]pyrrolo[2,3-e]quinolizine;

(8aα,11aα,12aα)-1-methoxy-11-[3-(N,N-dimethylaminosulfonylamino)propanesulfonyl]-5,6,8,8a,9,10,11, 11a,12,12a-decahydrobenzo[a]pyrrolo[2,3-e]quinolizine;

(8aα,11aα,12aα)-2-methoxy-11-[3-(N,N-dimethylaminosulfonylamino)propanesulfonyl]-5,6,8,8a,9,10,11, 11a,12,12a-decahydrobenzo[a]pyrrolo[2,3-e]quinolizine;

(8aα,11aα,12aα)-4-methoxy-11-[3-(N,N-dimethylaminosulfonylamino)propanesulfonyl]-5,6,8,8a,9,10,11, 11a,12,12a-decahydrobenzo[a]pyrrolo[2,3-e]quinolizine;

(8aα,11aα,12aα)-3-ethoxy-11-[3-(N,N-dimethylaminosulfonylamino)propanesulfonyl]-5,6,8,8a,9,10,11,11a, 12,12a-decahydrobenzo[a]pyrrolo[2,3-e]quinolizine;

(8aα,11aα,12aα)-3-isobutoxy-11-[3-(N,N-dimethylaminosulfonylamino)propanesulfonyl]-5,6,8,8a,9,10,11, 11a,12,12a-decahydrobenzo[a]pyrrolo[2,3-e]quinolizine;

(8aα,11aα,12aα)-3-n-hexyloxy-11-[3-(N,N-dimethylaminosulfonylamino)propanesulfonyl]-5,6,8,8a,9,10, 11,11a,12,12a-decahydrobenzo[a]pyrrolo[2,3-e]quinolizine;

(8aα,11aα,12aα)-3-hydroxy-11-[3-(N,N-dimethylaminosulfonylamino)propanesulfonyl]-5,6,8,8a,9,10,11, 11a,12,12a-decahydrobenzo[a]pyrrolo[2,3-e]quinolizine;

(8aα,11aα,12aα)-2,3-dihydroxy-11-[3-(N,N-dimethylaminosulfonylamino)propanesulfonyl]-5,6,8,8a,9,10, 11,11a,12,12a-decahydrobenzo[a]pyrrolo[2,3-e]quinolizine;

(8aα,11aα,12aα)-2,3-dimethoxy-11-[3-(N,N-dimethylaminosulfonylamino)propanesulfonyl]-5,6,8,8a,9,10, 11,11a,12,12a-decahydrobenzo[a]pyrrolo[2,3-e]quinolizine;

(8aα,11aα,12aα)-1,4-dimethoxy-11-[3-(N,N-dimethylaminosulfonylamino) propanesulfonyl]-5,6,8,8a,9, 10,11,11a,12,12a-decahydrobenzo[a]pyrrolo[2,3-e]quinolizine;

(8aα,11aα,12aα)-3,4-dimethoxy-11-[3-(N,N-dimethylaminosulfonylamino)propanesulfonyl]-5,6,8,8a,9,10,11, 11a,12,12a-decahydrobenzo[a]pyrrolo[2,3-e]quinolizine;

(8aα,11aα,12aα)-2,3-diethoxy-11-[3-(N,N-dimethylaminosulfonylamino)propanesulfonyl]-5,6,8,8a,9,10, 11,11a,12,12a-decahydrobenzo[a]pyrrolo[2,3-e]quinolizine;

(8aα,11aα,12aα)-2,3-di-n-butoxy-11-[3-(N,N-dimethylaminosulfonylamino)propanesulfonyl]-5,6,8,8a,9, 10,11,11a,12,12a-decahydrobenzo[a]pyrrolo[2,3-e]quinolizine;

(8aα,11aα,12aα)-2,3-dihexyloxy-11-[3-(N,N-dimethylaminosulfonylamino)propanesulfonyl]-5,6,8,8a,9,10, 11,11a,12,12a-decahydrobenzo[a]pyrrolo[2,3-e]quinolizine;

(8aα,11aα,12aα)-2,3-methylenedioxy-11-[3-(N,N-dimethylaminosulfonylamino)propanesulfonyl]-5,6,8,8a, 9,10,11,11a,12,12a-decahydrobenzo[a]pyrrolo[2,3-e]quinolizine;

(8aα,11aα,12aα)-2,3-methylenedioxy-11-[3-(N-methyl-N-ethylaminosulfonylamino)propanesulfonyl]-5,6, 8,8a,9,10,11,11a,12,12a-decahydrobenzo[a]pyrrolo[2,3-e]quinolizine;

(8aα,11aα,12aα)-2,3-methylenedioxy-11-[3-(N,N-diethylaminosulfonylamino)propanesulfonyl]-5,6,8, 8a,9,10,11,11a,12,12a-decahydrobenzo[a]pyrrolo[2,3-e]quinolizine;

(8aα,11aα,12aα)-2,3-methylenedioxy-11-[3-(N,N-dihexylaminosulfonylamino)propanesulfonyl]-5,6,8,8a, 9,10,11,11a,12,12a-decahydrobenzo[a]pyrrolo[2,3-e]quinolizine;

(8aα,11aα,12aα)-2,3-methylenedioxy-11-[3-(cyclohexylaminosulfonylamino)propanesulfonyl]-5,6,8,8a, 9,10,11,11a,12,12a-decahydrobenzo[a]pyrrolo[2,3-e]quinolizine;

(8aα,11aα,12aα)-1,2-methylenedioxy-11-[3-(N,N-dimethylaminosulfonylamino)propanesulfonyl]-5,6,8,8a, 9,10,11,11a,12,12a-decahydrobenzo[a]pyrrolo[2,3-e]quinolizine;

(8aα,11aα,12aα)-3,4-methylenedioxy-11-[3-(N,N-dimethylaminosulfonylamino)propanesulfonyl]-5,6,8,8a, 9,10,11,11a,12,12a-decahydrobenzo[a]pyrrolo[2,3-e]quinolizine;

(8aα,11aα,12aα)-2,3-(ethylene-1,2-dioxy)-11-[3-(N,N-dimethylaminosulfonylamino)propanesulfonyl]-5,6, 8,8a,9,10,11,11a,12,12a-decahydrobenzo[a]pyrrole[2,3-e]quinolizine;

(8aα,11aα,12aα)-1-methyl-11-[3-(N,N-dimethylaminosulfonylamino)propanesulfonyl]-5,6,8,8a,9,10,11,11a, 12,12a-decahydrobenzo[a]pyrrolo[2,3-e]quinolizine;

(8aα,11aα,12aα)-2-methyl-11-[3-(N,N-dimethylaminosulfonylamino)propanesulfonyl]-5,6,8,8a,9,10,11,11a, 12,12a-decahydrobenzo[a]pyrrolo[2,3-e]quinolizine;

(8aα,11aα,12aα)-3-methyl-11-[3-(N,N-dimethylaminosulfonylamino)propanesulfonyl]-5,6,8,8a,9,10,11,11a, 12,12a-decahydrobenzo[a]pyrrolo[2,3-e]quinolizine;

(8aα,11aα,12aα)-2,3-dimethyl-11-[3-(N,N-dimethylaminosulfonylamino)propanesulfonyl]-5,6,8,8a,9,10, 11,11a,12,12a-decahydrobenzo[a]pyrrolo[2,3-e]quinolizine;

(8aα,11aα,12aα)-3-ethyl-11-[3-(N,N-dimethylaminosulfonylamino)propanesulfonyl]-5,6,8,8a,9,10,11,11a, 12,12a-decahydrobenzo[a]pyrrolo[2,3-e]quinolizine;

(8aα,11aα,12aα)-3-isobutyl-11-[3-(N,N-dimethylaminosulfonylamino)propanesulfonyl]-5,6,8,8a,9,10,11, 11a,12,12a-decahydrobenzo[a]pyrrolo[2,3-e]quinolizine;

(8aα,11aα,12aα)-3-n-hexyl-11-[3-(N,N-dimethylaminosulfonylamino)propanesulfonyl]-5,6,8,8a,9,10,11, 11a,12,12a-decahydrobenzo[a]pyrrolo[2,3-e]quinolizine;

(8aα,11aα,12aα)-1-chloro-11-[3-(N,N-dimethylaminosulfonylamino)propanesulfonyl]-5,6,8,8a,9,10,11,11a,12,12a-decahydrobenzo[a]pyrrolo[2,3-e]quinolizine;

(8aα,11aα,12aα)-2-chloro-11-[3-(N,N-dimethylaminosulfonylamino)propanesulfonyl]-5,6,8,8a,9,10,11,11a,12,12a-decahydrobenzo[a]pyrrolo[2,3-e]quinolizine;

(8aα,11aα,12aα)-3-chloro-11-[3-(N,N-dimethylaminosulfonylamino)propanesulfonyl]-5,6,8,8a,9,10,11,11a,12,12a-decahydrobenzo[a]pyrrolo[2,3-e]quinolizine;

(8aα,11aα,12aα)-3-bromo-11-13-(N,N-dimethylaminosulfonylamino)propanesulfonyl]-5,6,8,8a,9,10,11,11a,12,12a-decahydrobenzo[a]pyrrolo[2,3-e]quinolizine;

(8aα,11aα,12aα)-2-fluoro-11-[3-(N,N-dimethylaminosulfonylamino)propanesulfonyl]-5,6,8,8a,9,10,11,11a,12,12a-decahydrobenzo[a]pyrrolo[2,3-e]quinolizine;

(8aα,11aα,12aα)-3-fluoro-11-[3-(N,N-dimethylaminosulfonylamino)propanesulfonyl]-5,6,8,8a,9,10,11,11a,12,12a-decahydrobenzo[a]pyrrolo[2,3-e]quinolizine;

(8aα,12aα,13aα)-3-methoxy-12-[3-(N-methyl-N-ethylaminosulfonylamino)propanesulfonyl]-5,6,8a,9,10,11,12,12a,13,13a-decahydro-8H-isoquino[2,1-g][1,6]naphthyridine;

(8aα,12aα,13aα)-3-methoxy-12-[3-(N,N-diethylaminosulfonylamino)propanesulfonyl]-5,6,8a,9,10,11,12,12a,13,13a-decahydro-8H-isoquino[2,1-g][1,6]naphthyridine;

(8aα,12aα,13aα)-3-methoxy-12-[3-(N,N-dihexylaminosulfonylamino)propanesulfonyl]-5,6,8a,9,10,11,12,12a,13,13a-decahydro-8H-isoquino[2,1-g][1,6]naphthyridine;

(8aα,12aα,13aα)-3-methoxy-12-[3-(cyclohexylaminosulfonylamino)propanesulfonyl]-5,6,8a,9,10,11,12,12a,13,13a-decahydro-8H-isoquino[2,1-g][1,6]naphthyridine.;

(8aα,12aα,13aα)-12-[3-(N,N-dimethylaminosulfonylamino)propanesulfonyl)-5,6,8a,9,10,11,12,12a,13,13a-decahydro-8H-isoquino[2,1-g][1,6]naphthyridine;

(8aα,12aα,13aα)-2,3-methylenedioxy-12-[3-(N,N-dimethylaminosulfonylamino)propanesulfonyl]-5,6,8a,9,10,11,12,12a,13,13a-decahydro-8H-isoquino[2,1-g][1,6]naphthyridine;

(8aα,12aα,13aα)-2,3-methylenedioxy-12-[3-(N-methyl-N-ethylaminosulfonylamino)propanesulfonyl]-5,6,8a,9,10,11,12,12a,13,13a-decahydro-8H-isoquino[2,1-g][1,6]naphthyridine;

(8aα,12aα,13aα)-2,3-methylenedioxy-12-[3-(N,N-diethylaminosulfonylamino)propanesulfonyl]-5,6,8a,9,10,11,12,12a,13,13a-decahydro-8H-isoquino[2,1-g][1,6]naphthyridine;

(8aα,12aα,13aα)-2,3-methylenedioxy-12-[3-(N,N-dihexylaminosulfonylamino)propanesulfonyl]-5,6,8a,9,10,11,12,12a,13,13a-decahydro-8H-isoquino[2,1-g][1,6]naphthyridine;

(8aα,12aα,13aα)-2,3-methylenedioxy-12-[3-(cyclohexylaminosulfonylamino)propanesulfonyl]-5,6,8a,9,10,11,12,12a,13,13a-decahydro-8H-isoquino[2,1-g][1,6]naphthyridine;

(8aα,12aα,13aα)-2,3-dimethoxy-12-[3-(N,N-dimethylaminosulfonylamino)propanesulfonyl]-5,6,8a,9,10,11,12,12a,13,13a-decahydro-8H-isoquino[2,1-g][1,6]naphthyridine;

(8aα,12aα,13aα)-1,4-dimethoxy-12-[3-(N,N-dimethylaminosulfonylamino)propanesulfonyl]-5,6,8a,9,10,11,12,12a,13,13a-decahydro-8H-isoquino[2,1-g][1,6]naphthyridine;

(8aα,12aα,13aα)-2,3-(ethylene-1,2-dioxy)-12-[3-(N,N-dimethylaminosulfonylamino)propanesulfonyl]-5,6,8a,9,10,11,12,12a,13,13a-decahydro-8H-isoquino[2,1-g][1,6]naphthyridine;

(8aα,12aα,13aα)-1-methyl-12-[3-(N,N-dimethylaminosulfonylamino)propanesulfonyl]-5,6,8a,9,10,11,12,12a,13,13a-decahydro-8H-isoquino[2,1-g][1,6]naphthyridine;

(8aα,12aα,13aα)-2-methyl-12-[3-(N,N-dimethylaminosulfonylamino)propanesulfonyl]-5,6,8a,9,10,11,12,12a,13,13a-decahydro-8H-isoquino[2,1-g][1,6]naphthyridine;

(8aα,12aα,13aα)-3-methyl-12-[3-(N,N-dimethylaminosulfonylamino)propanesulfonyl]-5,6,8a,9,10,11,12,12a,13,13a-decahydro-8H-isoquino[2,1-g][1,6]naphthyridine;

(8aα,12aα,13aα)-2,3-dimethyl-12-[3-(N,N-dimethylaminosulfonylamino)propanesulfonyl]-5,6,8a,9,10,11,12,12a,13,13a-decahydro-8H-isoquino-[2,1-g][1,6]naphthyridine;

(8aα,12aα,13aα)-3-ethyl-12-[3-(N,N-dimethylaminosulfonylamino)propanesulfonyl]-5,6,8a,9,10,11,12,12a,13,13a-decahydro-8H-isoquino[2,1-g][1,6]naphthyridine;

(8aα,12aα,13aα)-3-isobutyl-12-[3-(N,N-dimethylaminosulfonylamino)propanesulfonyl]-5,6,8a,9,10,11,12,12a,13,13a-decahydro-8H-isoquino[2,1-g][1,6]naphthyridine;

(8aα,12aα,13aα)-3-n-hexyl-12-[3-(N,N-dimethylaminosulfonylamino)propanesulfonyl]-5,6,8a,9,10,11,12,12a,13,13a-decahydro-8H-isoquino[2,1-g][1,6]naphthyridine;

(8aα,12aα,13aα)-1-methoxy-12-[3-(N,N-dimethylaminosulfonylamino)propanesulfonyl]-5,6,8a,9,10,11,12,12a,13,13a-decahydro-8H-isoquino[2,1-g][1,6]naphthyridine;

(8aα,12aα,13aα)-2-methoxy-12-[3-(N,N-dimethylaminosulfonylamino)propanesulfonyl]-5,6,8a,9,10,11,12,12a,13,13a-decahydro-8H-isoquino[2,1-g][1,6]naphthyridine;

(8aα,12aα,13aα)-4-methoxy-12-[3-(N,N-dimethylaminosulfonylamino)propanesulfonyl]-5,6,8a,9,10,11,12,12a,13,13a-decahydro-8H-isoquino[2,1-g][1,6]naphthyridine;

(8aα,12aα,13aα)-3-methoxy-2-methyl-12-[3-(N,N-dimethylaminosulfonylamino)propanesulfonyl]-5,6,8a,9,10,11,12,12a,13,13a-decahydro-8H-isoquino[2,1-g][1,6]naphthyridine;

(8aα,12aα,13aα)-3-ethoxy-12-[3-(N,N-dimethylaminosulfonylamino)propanesulfonyl]-5,6,8a,9,10,11,12,12a,13,13a-decahydro-8H-isoquino[2,1-g][1,6]naphthyridine;

(8aα,12aα,13aα)-3-isopropoxy-12-[3-(N,N-dimethylaminosulfonylamino)propanesulfonyl]-5,6,8a,9,10,11,12,12a,13,13a-decahydro-8H-isoquino[2,1-g][1,6]naphthyridine;

(8aα,12aα,13aα)-3-isopropoxy-12-[3-(N,N-dimethylaminosulfonylamino)propanesulfonyl]-5,6,8a,9,10,11,12,12a,13,13a-decahydro-8H-isoquino[2,1-g][1,6]naphthyridine;

(8aα,12aα,13aα)-3-n-hexyloxy-12-[3-(N,N-dimethylaminosulfonylamino)propanesulfonyl]-5,6,8a,9,10,11,12,12a,13,13a-decahydro-8H-isoquino[2,1-g][1,6]naphthyridine;

(8aα,12aα,13aα)-3-hydroxy-12-[3-(N,N-dimethylaminosulfonylamino)propanesulfonyl]-5,6,8a,9,10,11,12,12a,13,13a-decahydro-8H-isoquino[2,1-g][1,6]naphthyridine;

(8aα,12aα,13aα)-2,3-dihydroxy-12-[3-(N,N-dimethylaminosulfonylamino)propanesulfonyl]-5,6,8a,9,10,11,12,12a,13,13a-decahydro-8H-isoquino[2,1-g][1,6]naphthyridine;

(8aα,12aα,13aα)-1,2-dimethoxy-12-[3-(N,N-dimethylaminosulfonylamino)propanesulfonyl]-5,6,8a,9,10,11,12,12a,13,13a-decahydro-8H-isoquino[2,1-g][1,6]naphthyridine;

(8aα,12aα,13aα)-1,4-dimethoxy-12-[3-(N,N-dimethylaminosulfonylamino)propanesulfonyl]-5,6,8a,9,10,11,12,12a,13,13a-decahydro-8H-isoquino[2,1-g][1,6]naphthyridine;

(8aα,12aα,13aα)-3,4-dimethoxy-12-[3-(N,N-dimethylaminosulfonylamino)propanesulfonyl]-5,6,8a,9,10,11,12,12a,13,13a-decahydro-8H-isoquino[2,1-g][1,6]naphthyridine;

(8aα,12aα,13aα)-2,3-diethoxy-12-[3-(N,N-dimethylaminosulfonylamino)propanesulfonyl]-5,6,8a,9,10,11,12,12a,13,13a-decahydro-8H-isoquino[2,1-g][1,6]naphthyridine;

(8aα,12aα,13aα)-2,3-di-n-butoxy-12-[3-(N,N-dimethylaminosulfonylamino)propanesulfonyl]-5,6,8a,9,10,11,12,12a,13,13a-decahydro-8H-isoquino[2,1-g][1,6]naphthyridine;

(8aα,12aα,13aα)-1,2-methylenedioxy-12-(3-(N,N-dimethylaminosulfonylamino)propanesulfonyl]-5,6,8a,9,10,11,12,12a,13,13a-decahydro-8H-isoquino[2,1-g][1,6]naphthyridine;

(8aα,12aα,13aα)-2-chloro-12-[3-(N,N-dimethylaminosulfonylamino)propanesulfonyl]-5,6,8a,9,10,11,12,12a,13,13a-decahydro-8H-isoquino[2,1-g][1,6]naphthyridine;

(8aα,12aα,13aα)-3-chloro-12-[3-(N,N-dimethylaminosulfonylamino)propanesulfonyl]-5,6,8a,9,10,11,12,12a,13,13a-decahydro-8H-isoquino[2,1-g][1,6]naphthyridine;

(8aα,12aα,13aα)-4-chloro-12-[3-(N,N-dimethylaminosulfonylamino)propanesulfonyl]-5,6,8a,9,10,11,12,12a,13,13a-decahydro-8H-isoquino[2,1-g][1,6]naphthyridine;

(8aα,12aα,13aα)-3-bromo-12-[3-(N,N-dimethylaminosulfonylamino)propanesulfonyl]-5,6,8a,9,10,11,12,12a,13,13a-decahydro-8H-isoquino[2,1-g][1,6]naphthyridine;

(8aα,12aα,13aα)-3-fluoro-12-[3-(N,N-dimethylaminosulfonylamino)propanesulfonyl]-5,6,8a,9,10,11,12,12a,13,13a-decahydro-8H-isoquino[2,1-g][1,6]naphthyridine; and

(8aα,12aα,13aα)-2-fluoro-12-[3-(N,N-dimethylaminosulfonylamino)propanesulfonyl]-5,6,8a,9,10,11,12,12a,13,13a-decahydro-8H-isoquino[2,1-g][1,6]naphthyridine.

## EXAMPLE 4

### Preparation of (±)-(8aα,11aα,12aα)-3-methoxy-11-[3-(aminosulfonylamino)propanesulfonyl]-5,6,8,8a,9,10,11,11a,12,12a-decahydrobenzo[a]pyrrolo[2,3-e]quinolizine and Related Compounds of Formula (1) where $R^2$is $-NH_2$

A. Preparation of Formula (1) where A is propylene, $R^1$ is $-NH_2$, X is 3-methoxy, Y is hydrogen and n is 1

A mixture of 1.43 g of (±)-(8aα,11aα,12aα)-3-methoxy-11-(3-aminopropanesulfonyl)-5,6,8,8a,9,10,11,11a,12,12a-decahydrobenzo [a]pyrrolo[2,3-e]quinolizine (XIV where n is 1), prepared as shown in Preparation 9, 1 ml of triethylamine and 1.7 g of tert-butylaminosulfonyl chloride in 25 ml of methylene chloride was stirred at 25°C for 18 hours. The methylene chloride layer was washed with aqueous ammonium hydroxide, the organic layer separated and the solvent removed under reduced pressure. The residue was purified by flash chromatography on silica gel, eluting with ethyl acetate. The residue was stirred overnight in 6 ml of trifluoroacetic acid, and then the excess acid removed under reduced pressure. The residue was purified by flash chromatography on silica gel, eluting with 10% methanol/methylene chloride, and the product crystallised from ethyl acetate/hexane, to give (8aα,11aα,12aα)-3-methoxy-11-[3-(aminosulfonylamino)propanesulfonyl]-5,6,8a,9,10,11,11a,12,12a-decahydrobenzo[a]-pyrrolo[2,3-e]quinolizine, a compound of formula (1) where n is 1, mp 157-158°C.

B. Preparation of Formula (1) where A is propylene, $R^1$is $-NH_2$, X is 3-methoxy, Y is hydrogen and n is 2

Similarly, replacing (±)-(8aα,11aα,12aα)-3-methoxy-11-(3-aminopropanesulfonyl)-5,6,8,8a,9,10,11,11a,12,12a-decahydrobenzo [a]pyrrolo[2,3-e]quinolizine with (8aR,12aS,13aS)-3-methoxy-12-(3-aminopropanesulfonyl)-5,6,8a,9,10,11,12,12a,13,13a-decahydro-8H-isoquino[2,1-g][1,6]naphthyridine, prepared, for example, as shown in Preparation 11, and following the procedure of Example 4A above, the following compound of formula (1) where n is 2 was prepared as a foam, and converted to its maleate salt:

(8aR,12aS,13aS)-3-methoxy-12-[3-(aminosulfonylamino)propanesulfonyl]-5,6,8a,9,10,11,12,12a,13,13a-decahydro-8H-isoquino[2,1-g][1,6]naphthyridine, $[\alpha]_D$ = +21.4° (c = 0.7, $CH_3OH$).

C. Preparation of Formula (1) where $R^1$is $-NH_2$, varying A, X, Y and n

Similarly, replacing (±)-(8aα,11aα,12aα)-3-methoxy-11-(3-aminopropanesulfonyl)-5,6,8,8a,9,10,11, 11a,12,12a-decahydrobenzo[a]pyrrolo[2,3-e]quinolizine with compounds of formula (XIV) where n is 1 or 2, as racemic mixtures or single enantiomers, prepared, for example, as shown in Preparation 11D and 11E, and optionally replacing tert-butylaminosulfonyl chloride with an appropriate compound of formula $ZSO_2NHR^2$, where Z is as defined above and $R^2$ is tert-butyl, or $ZSO_2NR^2R^3$, where Z and $R^3$ are as defined above and $R^2$ is tert-butyl, and following the procedure in Example 4A above, the following compounds of formula (1) where n is 1 or 2 and $R^1$ is $-NH_2$ or $-NHR^3$ are prepared in racemic or optically active form:

(8aα,11aα,12aα)-3-methoxy-11-[3-(N-methylaminosulfonylamino)propanesulfonyl]-5,6,8,8a,9,10,11,11a,12,12a-decahydrobenzo[a]pyrrolo[2,3-e]quinolizine;

(8aα,11aα,12aα)-3-methoxy-11-[3-(N-ethylaminosulfonylamino)propanesulfonyl]-5,6,8,8a,9,10,11,11a,12,12a-decahydrobenzo[a]pyrrolo[2,3-e]quinolizine;

(8aα,11aα,12aα)-11-[3-(aminosulfonylamino)propanesulfonyl)-5,6,8,8a,9,10,11,11a,12,12a-decahydrobenzo[a]pyrrolo[2,3-e]quinolizine;

(8aα,11aα,12aα)-1-methoxy-11-[3-(aminosulfonylamino)propanesulfonyl]-5,6,8,8a,9,10,11,11a,12,12a-decahydrobenzo[a]pyrrolo[2,3-e]quinolizine;

(8aα,11aα,12aα)-2-methoxy-11-[3-(aminosulfonylamino)propanesulfonyl]-5,6,8,8a,9,10,11,11a,12,12a-decahydrobenzo[a]pyrrolo[2,3-e]quinolizine;

(8aα,11aα,12aα)-4-methoxy-11-[3-(aminosulfonylamino)propanesulfonyl]-5,6,8,8a,9,10,11,11a,12,12a-decahydrobenzo[a]pyrrolo[2,3-e]quinolizine;

(8aα,11aα,12aα)-3-ethoxy-11-[3-(aminosulfonylamino)propanesulfonyl]-5,6,8,8a,9,10,11,11a,12,12a-decahydrobenzo[a]pyrrolo[2,3-e]quinolizine;

(8aα,11aα,12aα)-3-isobutoxy-11-[3-(aminosulfonylamino)propanesulfonyl]-5,6,8,8a,9,10,11,11a,12,12a-decahydrobenzo[a]pyrrolo[2,3-e]quinolizine;

(8aα,11aα,12aα)-3-n-hexyloxy-11-[3-(aminosulfonylamino)propanesulfonyl]-5,6,8,8a,9,10,11,11a,12,12a-decahydrobenzo[a]pyrrolo[2,3-e]quinolizine;

(8aα,11aα,12aα)-3-hydroxy-11-[3-(aminosulfonylamino)propanesulfonyl]-5,6,8,8a,9,10,11,11a,12,12a-decahydrobenzo[a]pyrrolo[2,3-e]quinolizine;

(8aα,11aα,12aα)-2,3-dihydroxy-11-[3-(aminosulfonylamino)propanesulfonyl]-5,6,8,8a,9,10,11,11a,12,12a-decahydrobenzo[a]pyrrolo[2,3-e]quinolizine;

(8aα,11aα,12aα)-2,3-dimethoxy-11-[3-(aminosulfonylamino)propanesulfonyl]-5,6,8,8a,9,10,11,11a,12,12a-decahydrobenzo[a]pyrrolo[2,3-e]quinolizine;

(8aα,11aα,12aα)-1,4-dimethoxy-11-[3-(aminosulfonylamino)propanesulfonyl]-5,6,8,8a,9,10,11,11a,12,12a-decahydrobenzo[a]pyrrolo[2,3-e]quinolizine;

(8aα,11aα,12aα)-3,4-dimethoxy-11-[3-(aminosulfonylamino)propanesulfonyl]-5,6,8,8a,9,10,11,11a,12,12a-decahydrobenzo[a]pyrrolo[2,3-e]quinolizine;

(8aα,11aα,12aα)-2,3-diethoxy-11-[3-(aminosulfonylamino)propanesulfonyl]-5,6,8,8a,9,10,11,11a,12,12a-decahydrobenzo[a]pyrrolo[2,3-e]quinolizine;

(8aα,11aα,12aα)-2,3-di-n-butoxy-11-[3-(aminosulfonylamino)propanesulfonyl]-5,6,8,8a,9,10,11,11a,12,12a-decahydrobenzo[a]pyrrolo[2,3-e]quinolizine;

(8aα,11aα,12aα)-2,3-dihexyloxy-11-[3-(aminosulfonylamino)propanesulfonyl]-5,6,8,8a,9,10,11,11a,12,12a-decahydrobenzo[a]pyrrolo[2,3-e]quinolizine;

(8aα,11aα,12aα)-2,3-methylenedioxy-11-[3-aminosulfonylamino)propanesulfonyl]-5,6,8,8a,9,10,11,11a,12,12a-decahydrobenzo[a]pyrrolo[2,3-e]quinolizine;

(8aα,11aα,12aα)-2,3-methylenedioxy-11-[3-(N-methylaminosulfonylamino)propanesulfonyl]-5,6,8,8a,9,10,11,11a,12,12a-decahydrobenzo[a]pyrrolo[2,3-e]quinolizine;

(8aα,11aα,12aα)-2,3-methylenedioxy-11-[3-(N-ethylaminosulfonylamino)propanesulfonyl]-5,6,8,8a,9,10,

11,11a,12,12a-decahydrobenzo[a]pyrrolo[2,3-e]quinolizine;

(8aα,11aα,12aα)-1,2-methylenedioxy-11[3-(aminosulfonylamino)propanesulfonyl]-5,6,8,8a,9,10,11,11a,12,12a-decahydrobenzo[a]pyrrolo[2,3-e]quinolizine;

(8aα,11aα,12aα)-3,4-methylenedioxy-11-[3-aminosulfonylamino)propanesulfonyl]-5,6,8,8a,9,10,11,11a,12,12a-decahydrobenzo[a]pyrrolo[2,3-e]quinolizine;

(8aα,11aα,12aα)-2,3-(ethylene-1,2-dioxy)-11[3-(aminosulfonylamino)propanesulfonyl]-5,6,8,8a,9,10,11,11a,12,12a-decahydrobenzo[a]pyrrole[2,3-e]quinolizine;

(8aα,11aα,12aα)-1-methyl-11-[3-(aminosulfonylamino)propanesulfonyl]-5,6,8,8a,9,10,11,11a,12,12a-decahydrobenzo[a]pyrrolo[2,3-e]quinolizine;

(8aα,11aα,12aα)-2-methyl-11-[3-(aminosulfonylamino)propanesulfonyl]-5,6,8,8a,9,10,11,11a,12,12a-decahydrobenzo[a]pyrrolo[2,3-e]quinolizine;

(8aα,11aα,12aα)-3-methyl-11-[3-(aminosulfonylamino)propanesulfonyl]-5,6,8,8a,9,10,11,11a,12,12a-decahydrobenzo[a]pyrrolo[2,3-e]quinolizine;

(8aα,11aα,12aα)-2,3-dimethyl-11-[3-(aminosulfonylamino)propanesulfonyl]-5,6,8,8a,9,10,11,11a,12,12a-decahydrobenzo[a]pyrrolo[2,3-e]quinolizine;

(8aα,11aα,12aα)-3-ethyl-11-[3-(aminosulfonylamino)propanesulfonyl]-5,6,8,8a,9,10,11,11a,12,12a-decahydrobenzo[a]pyrrolo[2,3-e]quinolizine;

(8aα,11aα,12aα)-3-isobutyl-11-[3-(aminosulfonylamino)propanesulfonyl]-5,6,8,8a,9,10,11,11a,12,12a-decahydrobenzo[a]pyrrolo[2,3-e]quinolizine;

(8aα,11aα,12aα)-3-n-hexyl-11-[3-(aminosulfonylamino)propanesulfonyl]-5,6,8,8a,9,10,11,11a,12,12a-decahydrobenzo[a]pyrrolo[2,3-e]quinolizine;

(8aα,11aα,12aα)-1-chloro-11-[3-(aminosulfonylamino)propanesulfonyl]-5,6,8,8a,9,10,11,11a,12,12a-decahydrobenzo[a]pyrrolo[2,3-e]quinolizine;

(8aα,11aα,12aα)-2-chloro-11-[3-(aminosulfonylamino)propanesulfonyl]-5,6,8,8a,9,10,11,11a,12,12a-decahydrobenzo[a]pyrrolo[2,3-e]quinolizine;

(8aα,11aα,12aα)-3-chloro-11-[3-(aminosulfonylamino)propanesulfonyl]-5,6,8,8a,9,10,11,11a,12,12a-decahydrobenzo[a]pyrrolo[2,3-e]quinolizine;

(8aα,11aα,12aα)-3-bromo-11-[3-(aminosulfonylamino)propanesulfonyl]-5,6,8,8a,9,10,11,11a,12,12a-decahydrobenzo[a]pyrrolo[2,3-e]quinolizine;

(8aα,11aα,12aα)-2-fluoro-11-[3-(aminosulfonylamino)propanesulfonyl]-5,6,8,8a,9,10,11,11a,12,12a-decahydrobenzo[a]pyrrolo[2,3-e]quinolizine;

(8aα,11aα,12aα)-3-fluoro-11-[3-(aminosulfonylamino)propanesulfonyl]-5,6,8,8a,9,10,11,11a,12,12a-decahydrobenzo[a]pyrrolo[2,3-e]quinolizine;

(8aα,12aα,13aα)-3-methoxy-12-[3-(N-methylaminosulfonylamino)propanesulfonyl]-5,6,8a,9,10,11,12,12a,13,13a-decahydro-8H-isoquino[2,1-g][1,6]naphthyridine;

(8aα,12aα,13aα)-3-methoxy-12-[3-(N-ethylaminosulfonylamino)propanesulfonyl]-5,6,8a,9,10,11,12,12a,13,13a-decahydro-8H-isoquino[2,1-g][1,6]naphthyridine;

(8aα,12aα,13aα)-12-[3-(aminosulfonylamino)propanesulfonyl)-5,6,8a,9,10,11,12,12a,13,13a-decahydro-8H-isoquino[2,1-g][1,6]naphthyridine;

(8aα,12aα,13aα)-2,3-methylenedioxy-12-[3-(aminosulfonylamino)propanesulfonyl]-5,6,8a,9,10,11,12,12a,13,13a-decahydro-8H-isoquino[2,1-g][1,6]naphthyridine;

(8aα,12aα,13aα)-2,3-methylenedioxy-12-[3-(N-methylaminosulfonylamino)propanesulfonyl]-5,6,8a,9,10,11,12,12a,13,13a-decahydro-8H-isoquino[2,1-g][1,6]naphthyridine;

(8aα,12aα,13aα)-2,3-methylenedioxy-12-[3-(N-ethylaminosulfonylamino)propanesulfonyl]-5,6,8a,9,10,11,12,12a,13,13a-decahydro-8H-isoquino[2,1-g][1,6]naphthyridine;

(8aα,12aα,13aα)-2,3-dimethoxy-12-[3-(aminosulfonylamino)propanesulfonyl]-5,6,8a,9,10,11,12,12a,13,13a-decahydro-8H-isoquino[2,1-g][1,6]naphthyridine;

(8aα,12aα,13aα)-1,4-dimethoxy-12-[3-(aminosulfonylamino)propanesulfonyl]-5,6,8a,9,10,11,12,12a,13,13a-decahydro-8H-isoquino[2,1-g][1,6]naphthyridine;

(8aα,12aα,13aα)-2,3-(ethylene-1,2-dioxy)-12-[3-(aminosulfonylamino)propanesulfonyl]-5,6,8a,9,10,11,12,12a,13,13a-decahydro-8H-isoquino[2,1-g][1,6]naphthyridine;

(8aα,12aα,13aα)-1-methyl-12-[3-(aminosulfonylamino)propanesulfonyl]-5,6,8a,9,10,11,12,12a,13,13a-decahydro-8H-isoquino[2,1-g][1,6]naphthyridine;

(8aα,12aα,13aα)-2-methyl-12-[3-(aminosulfonylamino)propanesulfonyl]-5,6,8a,9,10,11,12,12a,13,13a-decahydro-8H-isoquino[2,1-g][1,6]naphthyridine;

(8aα,12aα,13aα)-3-methyl-12-[3-(aminosulfonylamino)propanesulfonyl]-5,6,8a,9,10,11,12,12a,13,13a-decahydro-8H-isoquino[2,1-g][1,6]naphthyridine;

(8aα,12aα,13aα)-2,3-dimethyl-12-[3-(aminosulfonylamino)propanesulfonyl]-5,6,8a,9,10,11,12,12a,13,13a-decahydro-8H-isoquino-[2,1-g][1,6]naphthyridine;

(8aα,12aα,13aα)-3-ethyl-12-[3-(aminosulfonylamino)propanesulfonyl]-5,6,8a,9,10,11,12,12a,13,13a-decahydro-8H-isoquino[2,1-g][1,6]naphthyridine;

(8aα,12aα,13aα)-3-isobutyl-12-[3-(aminosulfonylamino)propanesulfonyl]-5,6,8a,9,10,11,12,12a,13,13a-decahydro-8H-isoquino[2,1-g][1,6]naphthyridine;

(8aα,12aα,13aα)-3-n-hexyl-12-[3-(aminosulfonylamino)propanesulfonyl]-5,6,8a,9,10,11,12,12a,13,13a-decahydro-8H-isoquino[2,1-g][1,6]naphthyridine;

(8aα,12aα,13aα)-1-methoxy-12-[3-(aminosulfonylamino)propanesulfonyl]-5,6,8a,9,10,11,12,12a,13,13a-decahydro-8H-isoquino[2,1-g][1,6]naphthyridine;

(8aα,12aα,13aα)-2-methoxy-12-[3-(aminosulfonylamino)propanesulfonyl]-5,6,8a,9,10,11,12,12a,13,13a-decahydro-8H-isoquino[2,1-g][1,6]naphthyridine;

(8aα,12aα,13aα)-4-methoxy-12-[3-(aminosulfonylamino)propanesulfonyl]-5,6,8a,9,10,11,12,12a,13,13a-decahydro-8H-isoquino[2,1-g][1,6]naphthyridine;

(8aα,12aα,13aα)-3-methoxy-2-methyl-12-[3-(aminosulfonylamino)propanesulfonyl]-5,6,8a,9,10,11,12,12a,13,13a-decahydro-8H-isoquino[2,1-g][1,6]naphthyridine;

(8aα,12aα,13aα)-3-ethoxy-12-[3-(aminosulfonylamino)propanesulfonyl]-5,6,8a,9,10,11,12,12a,13,13a-decahydro-8H-isoquino[2,1-g][1,6]naphthyridine;

(8aα,12aα,13aα)-3-isopropoxy-12-[3-(aminosulfonylamino)propanesulfonyl]-5,6,8a,9,10,11,12,12a,13,13a-decahydro-8H-isoquino[2,1-g][1,6]naphthyridine;

(8aα,12aα,13aα)-3-isopropoxy-12-[3-(aminosulfonylamino)propanesulfonyl]-5,6,8a,9,10,11,12,12a,13,13a-decahydro-8H-isoquino[2,1-g][1,6]naphthyridine;

(8aα,12aα,13aα)-3-n-hexyloxy-12-[3-(aminosulfonylamino)propanesulfonyl]-5,6,8a,9,10,11,12,12a,13,13a-decahydro-8H-isoquino[2,1-g][1,6]naphthyridine;

(8aα,12aα,13aα)-3-hydroxy-12-[3-(aminosulfonylamino)propanesulfonyl]-5,6,8a,9,10,11,12,12a,13,13a-decahydro-8H-isoquino[2,1-g][1,6]naphthyridine;

(8aα,12aα,13aα)-2,3-dihydroxy-12-[3-(aminosulfonylamino)propanesulfonyl]-5,6,8a,9,10,11,12,12a,13,13a-decahydro-8H-isoquino[2,1-g][1,6]naphthyridine;

(8aα,12aα,13aα)-1,2-dimethoxy-12-[3-(aminosulfonylamino)propanesulfonyl]-5,6,8a,9,10,11,12,12a,13,13a-decahydro-8H-isoquino[2,1-g][1,6]naphthyridine;

(8aα,12aα,13aα)-1,4-dimethoxy-12-[3-(aminosulfonylamino)propanesulfonyl]-5,6,8a,9,10,11,12,12a,13,13a-decahydro-8H-isoquino[2,1-g][1,6]naphthyridine;

(8aα,12aα,13aα)-3,4-dimethoxy-12-[3-(aminosulfonylamino)propanesulfonyl]-5,6,8a,9,10,11,12,12a,13,13a-decahydro-8H-isoquino[2,1-g][1,6]naphthyridine;

(8aα,12aα,13aα)-2,3-diethoxy-12-[3-(aminosulfonylamino)propanesulfonyl]-5,6,8a,9,10,11,12,12a,13,13a-decahydro-8H-isoquino[2,1-g][1,6]naphthyridine;

(8aα,12aα,13aα)-2,3-di-n-butoxy-12-[3-(aminosulfonylamino)propanesulfonyl]-5,6,8a,9,10,11,12,12a,13,13a-decahydro-8H-isoquino[2,1-g][1,6]naphthyridine;

(8aα,12aα,13aα)-1,2-methylenedioxy-12-(3-(aminosulfonylamino)propanesulfonyl]-5,6,8a,9,10,11,12,12a,13,13a-decahydro-8H-isoquino[2,1-g][1,6]naphthyridine;

(8aα,12aα,13aα)-2-chloro-12-[3-(aminosulfonylamino)propanesulfonyl]-5,6,8a,9,10,11,12,12a,13,13a-decahydro-8H-isoquino[2,1-g][1,6]naphthyridine;

(8aα,12aα,13aα)-3-chloro-12-[3-(aminosulfonylamino)propanesulfonyl]-5,6,8a,9,10,11,12,12a,13,13a-decahydro-8H-isoquino[2,1-g][1,6]naphthyridine;

(8aα,12aα,13aα)-4-chloro-12-[3-(aminosulfonylamino)propanesulfonyl]-5,6,8a,9,10,11,12,12a,13,13a-decahydro-8H-isoquino[2,1-g][1,6]naphthyridine;

(8aα,12aα,13aα)-3-bromo-12-[3-(aminosulfonylamino)propanesulfonyl]-5,6,8a,9,10,11,12,12a,13,13a-decahydro-8H-isoquino[2,1-g][1,6]naphthyridine;

(8aα,12aα,13aα)-3-fluoro-12-[3-(aminosulfonylamino)propanesulfonyl]-5,6,8a,9,10,11,12,12a,13,13a-decahydro-8H-isoquino[2,1-g][1,6]naphthyridine; and

(8aα,12aα,13aα)-2-fluoro-12-[3-(aminosulfonylamino)propanesulfonyl]-5,6,8a,9,10,11,12,12a,13,13a-decahydro-8H-isoquino[2,1-g][1,6]naphthyridine.

EXAMPLE 5

Conversion of (8aR,12aS,13aS)-3-methoxy-12-[3-(dimethylaminosulfonylamino)propanesulfonyl]sulfonyl-5,6,8a,9,10,11,12,12a.13,13a-decahydro-8H-isoquino[2,1g][1,6]naphthyridine to its hydrochloride salt

Excess 3% hydrogen chloride in methanol is added to a solution of (8aR,12aS,13aS)-3-methoxy-12-[3-(dimethylaminosulfonylamino)propanesulfonyl]-5,6,8a,9,10,11,12,12a,13,13a-decahydro-8H-isoquino[2,1-g][1,6]-naphthyridine in 20 ml methanol. Diethyl ether is added until precipitation is complete. The product is filtered, washed with ether, air dried and recrystallized from ethanol/diethyl ether to yield (8aR,12aS,13aS)-3-methoxy-12-[3-(dimethylaminosulfonyl-amino)propanesulfonyl]-5,6,8a,9,10,11,12,12a,13,13adecahydro-8H-isoquino[2,1-g][1,6]naphthyridine hydrochloride.

In a similar manner, all compounds of formula (1) in free base form may be converted to the acid addition salts by treatment with the appropriate acid, for example, hydrobromic acid, sulfuric acid, nitric acid, phosphoric acid, acetic acid, propionic acid, glycolic acid, pyruvic acid, oxalic acid, malonic acid, succinic acid, malic acid, maleic acid, fumaric acid, tartaric acid, citric acid, benzoic acid, cinnamic acid, mandelic acid, methanesulfonic acid, ethanesulfonic acid, p-toluenesulfonic acid, and the like.

EXAMPLE 6

Conversion of a salt of (8aR,12aS,13aS)-3-methoxy-12-[3-(dimethylaminosulfonylamino)propanesulfonyl]sulfonyl-5,6,8a,9,10,11,12,12a,13,13a-decahydro-8H-isoquino[2,1g][1,6]naphthyridine to free base.

(8aR,12aS,13aS)-3-methoxy-12-[3-(dimethylaminosulfonylamino)propanesulfonyl]-5,6,8a,9,10,11,12,12a,13,13a-decahydro-8H-isoquino[2,1-g][1,6]naphthyridine hydrochloride suspended in 50 ml of ethyl acetate is stirred with excess dilute aqueous potassium carbonate solution until the salt is completely dissolved. The organic layer is then separated, washed twice with water, dried over magnesium sulfate and evaporated to yield (8aR,12aS,13aS)3-methoxy-12-[3-(dimethylaminosulfonylamino)propanesulfonyl]-5,6,8a,9,10,11,12,12a,13,13a-decahydro-8H-isoquino[2,1-g][1,6]naphthyridine.

In a similar manner the acid addition salts of all compounds of formula (1) may be converted to the corresponding compounds in free base form.

EXAMPLE 7

Direct interchange of acid addition salts of (8aR,12aS,13aS)-3-methoxy-12-[3-(dimethylaminosulfonylamino)propanesulfonyl]sulfonyl-5,6,8a,9,10,11,12,12a,13,13a-decahydro-8H-isoquino[2,1g][1,6]naphthyridine

(8aR,12aS,13aS)-3-methoxy-12-[3-(dimethylaminosulfonylamino)propanesulfonyl]-5,6,8a,9,10,11,12,12a,13,13a-decahydro-8H-isoquino[2,1-g][1,6]naphthyridine acetate (1.0 g) is dissolved in 50 ml 5N aqueous hydrochloric acid, and the solution evaporated to dryness. The product is suspended in ethyl acetate and filtered, air dried and recrystallized from methanol/acetone to yield (8aR,12aS,13aS)-3-methoxy-12-[3-(dimethylaminosulfonylamino)propanesulfonyl]-5,6,8a,9,10,11,12,12a,13,13a-decahydro-8H-isoquino[2,1-g][1,6]naphthyridine hydrochloride.

In a similar manner, substituting for hydrochloric acid other acids, such as sulfuric acid, nitric acid, phosphoric acid and the like, other acid addition salts of all compounds of formula (1) are prepared.

In Examples 8 through 13 the active ingredient is (8aR,12aS,13aS)-3-methoxy-12-[3-(dimethylaminosulfonylamino)propanesulfonyl]-5,6,8a,9,10,11,12,12a,13,13a-decahydro-8H-isoquino[2,1-g][1,6]naphthyridine hydrochloride, and a daily dose of 20 mg is assumed.

Other compounds of formula (1) or a pharmaceutically acceptable salts thereof at the same or different dosage may be substituted therein.

EXAMPLE 8

Composition for Oral Administration

| The composition contains: | % wt./wt. |
|---|---|
| Active ingredient | 20% |
| Lactose | 79.5% |
| Magnesium stearate | 0.5% |

The two ingredients are mixed and dispensed into capsules containing 100 mg each; one capsule would approximate a total daily dosage.

EXAMPLE 9

Composition for Oral Administration

| The composition contains: | % wt./wt. |
|---|---|
| Active ingredient | 20.0% |
| Magnesium stearate | 0.5% |
| Crosscarmellose sodium | 2.0% |
| Lactose | 76.5% |
| PVP (polyvinylpyrrolidine) | 1.0% |

The above ingredients are combined and granulated using methanol as solvent. The formulation is then dried and formed into tablets (containing 20 mg of active compound) with an appropriate tableting machine.

EXAMPLE 10

Parenteral Formulation (IV)

| The composition contains: | % wt./wt. |
|---|---|
| Active ingredient | 0.25 g |
| Sodium Chloride | qs to make isotonic |
| Water for injection to | 100 ml |

The active ingredient is dissolved in a portion of the water for injection. A sufficient quantity of sodium chloride is then added with stirring to make the solution isotonic. The solution is made up to weight with the remainder of the water for injection, filtered through a 0.2 micron membrane filter and packaged under sterile conditions.

EXAMPLE 11

Suppository Formulation

| The composition contains: | % wt./wt. |
|---|---|
| Active ingredient | 1.0% |
| Polyethylene glycol 1000 | 74.5% |
| Polyethylene glycol 4000 | 24.5% |

The ingredients are melted together and mixed on a steam bath, and poured into molds containing 2.5 g total weight.

EXAMPLE 12

Topical Formulation

| Ingredients | grams |
|---|---|
| Active compound | 0.2-2 |
| Span 60 | 2 |
| Tween 60 | 2 |
| Mineral oil | 5 |
| Petrolatum | 10 |
| Methyl paraben | 0.15 |
| Propyl paraben | 0.05 |
| BHA (butylated hydroxy anisole) | 0.01 |
| Water | q.s. 100 |

All of the above ingredients, except water, are combined and heated to 60°C with stirring. A sufficient quantity of water at 60°C is then added with vigorous stirring to emulsify the ingredients, and water then added q.s. 100 g.

EXAMPLE 13

Composition for Topical Administration to the Eye

| The composition contains: | % wt/vol |
|---|---|
| Active ingredient | 0.10 |
| Benzalkonium chloride | 0.02 |
| EDTA | 0.01 |
| Phenylethanol | 0.25 |
| Boric acid | 1.62 to adjust pH |
| and water qs | 100 ml |

The first four ingredients are dissolved in less than the required total volume of water, and the pH adjusted to 7.4. The volume is then brought to 100 ml with additional water.

EXAMPLE 14

Assay for pre- and post-synaptic $\alpha_2$-adrenoceptor blockade

Protocol:

Ileum preparations were taken from female Dunkin Hartley guinea pigs in the weight range of 250-600 grams, and set up in 30 ml isolated organ baths containing physiological Tyrode solution of the following composition ($mmol.L^{-1}$) :

NaCl - 136.89, KCl - 2.68, $MgCl_2$. $6H_2O$ - 1.05, $NaH_2PO_4$. $2H_2O$ - 0.42, Glucose - 5.55, $NaHCO_3$ - 11.9, $CaCl_2$. $6H_2O$ - 1.8

The mixture was gassed with 100% oxygen and maintained at 37°C. An initial tension of 1 gram was applied. The preparations were field stimulated at 0.1 Hz (1 msec pulse duration, supramaximal voltage) via a stainless steel electrode passing through the lumen. The resulting contractions were recorded isometrically on a chart recorder. After an equilibrium period of 1 hour an initial cumulative dose-response curve to the agonist UK14304 was obtained. The preparations were then washed thoroughly to remove all of the agonist and left

to equilibrate for a further 40 minutes, after which a second cumulative dose-response curve to the agonist was obtained and measured as the control. The preparations were then treated with the $\alpha_2$-adrenoceptor antagonists of formula (1) for 40 minutes, and then concentration-response curves to the agonist determined. Antagonism of the responses to UK14304 was quantified by the method of Arunlakshana and Schild, Br. J. Pharmacol. Chemother., Vol. 14, 48-58 (1959), and $pA_2$ values were obtained. The compounds of the present invention showed activity when tested by this procedure.

Alternatively, the procedure according to Caroon, J.M. et al., J. Med. Chem., 1982, Vol. 25, 666, as set forth in U.S. Patent 4,791,108, may be used for determining pre- and post-synaptic $\alpha_2$-adrenoceptor blockade in compounds of formula (1). The compounds of the present invention show activity when tested by this procedure.

EXAMPLE 15

Determination of Platelet Aggregation Inhibition

Protocol:

Blood platelets are collected in the standard manner, and incubated in an Aggregation Module Incubator-Cuvette in the presence of either the inhibitor to be tested, or without said inhibitor as a control. The aggregation of the platelets is observed after the addition of an inducer, and the samples are evaluated for the presence of a lag period and the slope of the aggregation curve, as well as the maximum height of the aggregation curve in comparison to the control. $IC_{50}$ values i.e. the concentration of inhibitor required for 50% inhibition can be calculated from the inflection point on the appropriate dose response curve. The compounds of the present invention show activity when tested by this procedure.

EXAMPLE 16

Determination of Effect on Intraocular Pressure

Protocol:

The compound to be tested is dissolved in saline, and applied topically to the eye. The intraocular pressure is measured immediately before application, and at specified time intervals thereafter, by means of a probe which measures the force necessary to flatten a small area of corneal surface, according to the method described by Moses, R. A., Tr. Am. Acad. Opth. and Otol., Jan-Feb 1962: 88-95. The compounds of the present invention show activity when tested by this procedure.

EXAMPLE 17

Determination of Effect on Rat Sexual Behavior

"Sexual Behavior in Developing Male Rats", P. Sodersten, D. A Dammassa and E. R. Smith, Hormones and Behavior, Vol. 8, pp 320-334 (1977).

Protocol

Sexually-naive male rats, weighing 200-250 g, were housed two to a cage in a normal light-cycle room (lights on 5.00 a.m., lights off 7.00 p.m.). The animals were grouped according to their weight after a 10 day acclimatization period, and tested on either the 12th or 13th day. The compound to be tested was administered 30 minutes before evaluating for sexual activity.

Stimulus female Sprague-Dawley rats, housed in a reverse light-cycle room (lights off 10.00 a.m., lights on 8.00 p.m.), were brought into sexual receptivity by injection with 20 mg of estradiol benzoate in 0.1 ml of sesame seed oil 48 hours prior to the test, and with 1 mg of progesterone in 0.1 ml of sesame seed oil 4-6 hours prior to the test.

Each male rat treated with the test compound was placed in an observation cage and allowed to acclimatize for 10 minutes. A stimulus female was then introduced into the cage and the behavior of the male recorded on an Esterline Angus event recorder. The behavior recorded was mounts, intromissions and ejaculations. Intromission latency (time from the start of the test to the first intromission), ejaculation latency (time from the first

intromission to ejaculation), and post-ejaculatory interval (time from ejaculation to the next following intromission) were also recorded. Tests were terminated if the intromission latency was longer than 15 minutes, the ejaculation latency was longer than 30 minutes, or the post-ejaculatory interval was in excess of 15 minutes.

The compounds of the present invention showed activity when tested by this procedure. This enhanced activity was measured by an increase in the behavior score.

EXAMPLE 18

Irritable-Bowel Syndrome Assay

Protocol:

The test used is a modification of the method of Macht and Barba-Gose (Macht, D.T. and Barba-Gose, J. (1931): J. Amer. Pharm. Ass. Vol.20, 558), which traces the transit of a charcoal meal through the intestine as an index of transit time. In the present model, intestinal transit in conscious mice (15-20g) was accelerated with an oral dose of barium chloride (300 mg/kg) administered at the same time as the charcoal meal. The animals were sacrificed 10 min. later and the distance travelled by the charcoal measured.

The antagonist compound was given as a 15 min. oral pretreatment and its effects on barium-stimulated intestinal transit of the charcoal meal was calculated. The compounds of the present invention show activity when tested by this procedure.

EXAMPLE 19

Hypoglycaemic Assay

A compound of formula (1) was administered to groups of male beagle dogs (10 µg/kg intravenously or 30 µg/kg orally) and found to elevate plasma insulin and lower plasma glucose; these effects indicate potential utility as an antidiabetic agent. The compounds of the present invention show activity when tested by this procedure.

EXAMPLE 20

Antihypertensive Assay

Compounds of formula (1) have antihypertensive activity in that the compound will lower blood pressure in conscious spontaneously hypertensive rats when it is administered intravenously. The mean blood pressure is monitored by an indwelling catheter in the tail artery. The compounds of the present invention show activity when tested by this procedure.

EXAMPLE 21

Selective Vasodilation Assay

Tail Skin Temperature and Core Temperature

Male Sprague-Dawley rats weighing 200-300 grams were housed in pairs at a temperature of 18-20°C for 2-6 days before carrying out the assay. One hour before the first measurement the rats were placed in well-ventilated transparent holding cages (length 18 cm, width 12 cm, height 10 cm) and restrained by passing the tail through a 2 cm diameter aperture at the rear of the cage and taping it to a rod which protruded from the rear wall immediately above the opening. A plastic or rubber-covered thermocouple was inserted 5 cm past the anal sphincter and taped to the tail. A flat disc thermocouple (diameter 7 mm) was affixed to the dorsal surface of the tail using two layers of tape. Readings of ambient temperature (Ta), core temperature (Tc), and tail skin temperature (Ts) were taken at 5 minute intervals for 75 minutes. Immediately after the fourth reading the rats were dosed with the drug, by oral intubation or by subcutaneous injection. For each treatment the measurements were compared with those of the appropriate control group. The compounds of formula (1) evoked an increase in tail skin temperature, and thus compounds of the present invention showed activity when tested by this procedure.

EXAMPLE 22

Peripheral Selectivity Assay

Naunyn-Schniedeberg's Arch. Pharmacol., Vol. 307, p 45-50 (Koss and Christensen 1979); Br. J. Pharmacol., Vol. 78, p 507-515 (Beridge et al., 1983); Br. J. Pharmacol., Vol. 83, p 707-712 (Gadie et al., 1984)

Protocol

A. A standard challenge dose of the $\alpha_2$-adrenoceptor agonist BHT 920 (3-10 mcg/kg, iv) was administered to pithed rats at 10 minute intervals. A compound of formula (1) was injected intravenously in ascending doses, each dose being administered 5 minutes before each agonist challenge dose. The dose of the compound of formula (1) that reduced the pressor response to the agonist by 50% ($ED_{50}$) was calculated.

B. In anaesthetized rats clonidine (0.3 mg/kg, sc) was injected to give a prolonged (about 1 hour duration) mydriasis. A compound of formula (1) was injected intravenously in ascending doses at 10 minute intervals, and the eye pupil diameter measured 5 minutes after each dose. The dose of (1) that reduced the response to clonidine by 50% ($ED_{50}$) was calculated.

It was found that the $ED_{50}$ values for antagonism of the pressor response (denoting peripheral activity) were substantially less than the $ED_{50}$ values calculated for antagonism of mydriasis (denoting central nervous system activity). Thus the compounds of formula (1) showed greater selectivity for peripheral effects.

Toxicology

No serious toxicological effects were observed in the above described biological assays.

**Claims**

1. A compound represented by the formula:

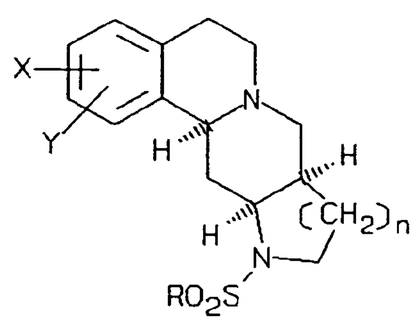

wherein:

X and Y are independently hydrogen; hydroxy; lower alkyl of 1-6 carbon atoms; lower alkoxy of 1-6 carbon atoms; or halo; or X and Y when adjacent and taken together are methylenedioxy or ethylene-1,2-dioxy;

R is lower alkyl of 1-6 carbon atoms; cycloalkyl of 3-8 carbon atoms; phenyl or phenyl lower alkyl wherein any phenyl group may be optionally substituted by one or two substituents chosen from the group consisting of halo, lower alkyl of 1-4 carbon atoms and lower alkoxy of 1-4 carbon atoms; or $-ANHSO_2R^1$; wherein A is lower alkylene of 1-6 carbon atoms; and $R^1$ is lower alkyl of 1-6 carbon atoms or $-NR^2R^3$;

wherein;

$R^2$ and $R^3$ are independently hydrogen or lower alkyl of 1-6 carbon atoms, or $R^2$ and $R^3$ taken together are cycloalkyl of 3-8 carbon atoms; and

n is 1 or 2;

or a pharmaceutically acceptable salt thereof; with the proviso that when n is 2, R cannot be lower alkyl, cycloalkyl, phenyl or phenyl lower alkyl.

2. The compound of claim 1 wherein n is 1 and R is lower alkyl or $-ANHSO_2R^1$, or a pharmaceutically acceptable salt thereof.

3. The compound of claim 2 wherein X and Y are independently hydrogen or lower alkoxy having 1-4 carbon atoms, or X and Y taken together is methylenedioxy, or a pharmaceutically acceptable salt thereof.

4. The racemic compound of claim 3, namely (±)-(8aα,11aα,12aα)-3-methoxy-11-methanesulfonyl-5 ,6,8,8a,9,10,11,11a,12,12a-decahydrobenzo[a]pyrrolo[2,3-e]quinolizine; or (±)-(8aα,11aα,12aα)-2,3-methylenedioxy-11-methanesulfonyl-5 ,6,8,8a,9,10,11,11a,12,12a-decahydrobenzo[a]pyrrolo[2,3-e]quinolizine; or (±)-(8aα,11aα,12aα)-3-methoxy-11-[3-(methanesulfonylamino)propanesulfonyl]-5,6,8,8a,9,10,11,11a,12,12a-decahydrobenzo[a]pyrrolo[2,3-e]quinolizine; or (±)-(8aα,11aα,12aα)-2,3-methylenedioxy-11-[3-(methanesulfonylamino)propanesulfonyl]-5,6,8,8a,9,10,11,11a,12,12a-decahydrobenzo[a]pyrrolo(2,3-e]quinolizine; or a pharmaceutically acceptable salt of the compound.

5. The compound of claim 3 wherein A is alkylene of 2-4 carbon atoms and $R^1$ is lower alkyl or $NR^2R^3$, or a pharmaceutically acceptable salt thereof.

6. The racemic compound of claim 5, namely (±)-(8aα,11aα,12aα)-3-methoxy-11-[3-(aminosulfonylamino)propanesulfonyl]-5,6,8,8a,9,10,11,11a,12,12a-decahydrobenzo[a]pyrrolo[2,3-e]quinolizine; or (±)-(8aα,11aα,12aα)-3-methoxy-11-[3-(dimethylaminosulfonylamino)propanesulfonyl]-5,6,8,8a,-9,10,11,11a, 12,12a-decahydrobenzo[a]pyrrolo[2,3-e]-quinolizine; or a pharmaceutically acceptable salt of the compound.

7. The compound of claim 1 wherein n is 2, or a pharmaceutically acceptable salt thereof.

8. The compound of claim 7 wherein R is $-ANHSO_2R^1$ or a pharmaceutically acceptable salt thereof.

9. The compound of claim 8 wherein A is alkylene of 2-4 carbon atoms and $R^1$ is lower alkyl or $NR^2R^3$, or a pharmaceutically acceptable salt thereof.

10. The compound of claim 9 wherein X and Y are independently hydrogen or lower alkoxy having 1-4 carbon atoms, or X and Y taken together is methylenedioxy, or a pharmaceutically acceptable salt thereof.

11. The racemic compound of claim 10 wherein X is 3-methoxy, Y is hydrogen, A is propylene and $R^1$ is methyl, namely (±)-(8aα,12aα,13aα)-3-methoxy-12-[3-(methanesulfonylamino)propanesulfonyl]-5,6,8a,9,10,11,12,12a,13,13a-decahydro-8H-isoquino[2,1-g][1,6]naphthyridine, or a pharmaceutically acceptable salt thereof.

12. The (+)-isomer of a compound of claim 11, namely (8aR,12aS,13aS)-3-methoxy-12-[3-(methanesulfonylamino)propanesulfonyl]-5,6,8a,9,10,11,12,12a,13,13a-decahydro-8H-isoquino[2,1-g][1,6]naphthyridine, or a pharmaceutically acceptable salt thereof.

13. The racemic compound of claim 10 wherein X is 3-methoxy, Y is hydrogen, A is propylene and $R^2$ and $R^3$ are both methyl, namely (±)-(8aα,12aα,13aα)-3-methoxy-12-[3-(dimethylaminosulfonylamino)propanesulfonyl]-5,6,8a,9,10,11,12,12a,13,13a-decahydro-8H-isoquino[2,1-g][1,6]naphthyridine, or a pharmaceutically acceptable salt thereof.

14. The (+)-isomer of a compound of claim 13, namely (8aR,12aS,13aS)-3-methoxy-12-[3-(dimethylaminosulfonylamino)propanesulfonyl]-5,6,8a,9,10,11,12,12a,13,13a-decahydro-8H-isoquino[2,1-g][1,6]naphthyridine, or a pharmaceutically acceptable salt thereof.

15. An intermediate represented by the formula:

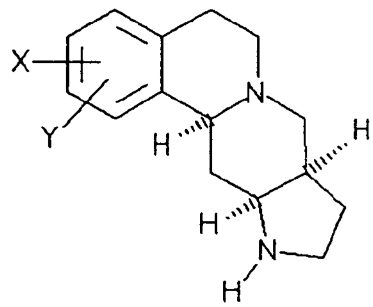

wherein:

X and Y are independently hydrogen, hydroxy, lower alkyl of 1-6 carbon atoms, lower alkoxy of 1-6 carbon atoms or halo, or X and Y when adjacent and taken together are methylenedioxy or ethylene-1,2-dioxy.

**16.** The compound of claim 15 wherein X and Y are independently hydrogen or lower alkoxy having 1-4 carbon atoms, or X and Y taken together is methylenedioxy, or a pharmaceutically acceptable salt thereof.

**17.** A pharmaceutical composition comprising a pharmaceutically acceptable non-toxic carrier and a therapeutically effective amount of a compound of any one of claims 1 to 14.

**18.** A compound of any one of claims 1 to 14 for pharmaceutical use.

**19.** Use of a compound of any one of claims 1 to 14 in the manufacture of a medicament for treating a mammal having a disease-state that is alleviated by treatment with an $\alpha_2$-adrenoceptor antagonist.

**20.** Use of a compound represented by the formula:

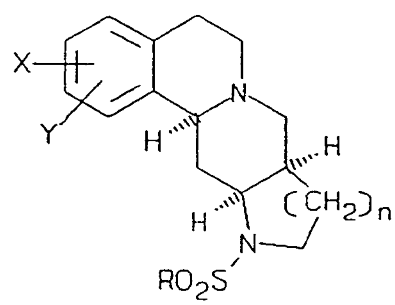

wherein:

X and Y are independently hydrogen; hydroxy; lower alkyl of 1-6 carbon atoms; lower alkoxy of 1-6 carbon atoms; or halo; or X and Y when adjacent and taken together are methylenedioxy or ethylene-1,2-dioxy;

R is lower alkyl of 1-6 carbon atoms; cycloalkyl of 3-8 carbon atoms; phenyl or phenyl lower alkyl wherein any phenyl group may be optionally substituted by one or two substituents chosen from the group consisting of halo, lower alkyl of 1-4 carbon atoms and lower alkoxy of 1-4 carbon atoms; or $-ANHSO_2R^1$; wherein A is lower alkylene of 1-6 carbon atoms; and $R^1$ is lower alkyl of 1-6 carbon atoms or $-NR^2R^3$;

wherein;

$R^2$ and $R^3$ are independently hydrogen or lower alkyl of 1-6 carbon atoms, or $R^2$ and $R^3$ taken together are cycloalkyl of 3-8 carbon atoms; and

n is 1 or 2;

or a pharmaceutically acceptable salt thereof; in the manufacture of a medicament for treating a mammas having peripheral vascular disease, particularly diabetes and its sequelae such as diabetic retinopathy, nephropathy, neuropathy and associated circulatory disturbances.

**21.** A process for the preparation of a compound represented by the formula:

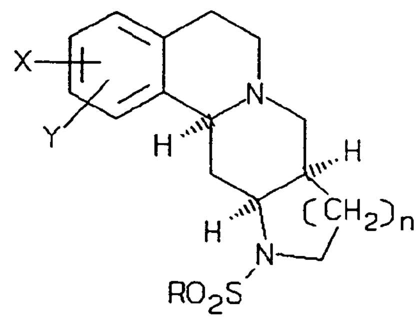

wherein:

X and Y are independently hydrogen; hydroxy; lower alkyl of 1-6 carbon atoms; lower alkoxy of 1-6 carbon atoms; or halo; or X and Y when adjacent and taken together are methylenedioxy or ethylene-1,2-dioxy;

R is lower alkyl of 1-6 carbon atoms; cycloalkyl of 3-8 carbon atoms; phenyl or phenyl lower alkyl wherein any phenyl group may be optionally substituted by one or two substituents chosen from the group consisting of halo, lower alkyl of 1-4 carbon atoms and lower alkoxy of 1-4 carbon atoms; or $-ANHSO_2R^1$; wherein A is lower alkylene of 1-6 carbon atoms; and $R^1$ is lower alkyl of 1-6 carbon atoms or $-NR^2R^3$;

wherein;

$R^2$ and $R^3$ are independently hydrogen or lower alkyl of 1-6 carbon atoms, or $R^2$ and $R^3$ taken together are cycloalkyl of 3-8 carbon atoms; and

n is 1 or 2;

or a pharmaceutically acceptable salt thereof; with the proviso that when n is 2, R cannot be lower alkyl, cycloalkyl, phenyl or phenyl lower alkyl; said process comprising

(a) sulfonylating a compound of the formula

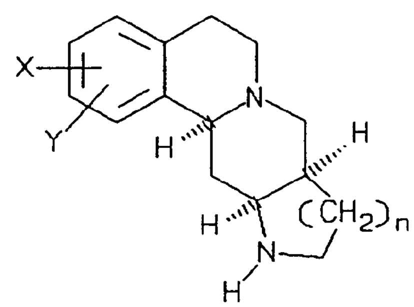

(VII) or (XI)

in which:

X, Y and n are as defined above, with a compound of the formula $ZSO_2R$, where R is lower alkyl, cycloalkyl, or optionally substituted phenyl and Z is chlorine or bromine; or

(b) reacting a compound of the formula:

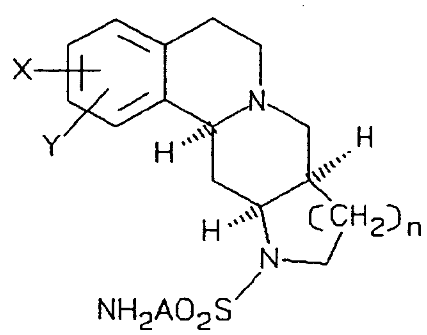

(XIV)

where A, X, Y and n are as defined above, with a compound of the formula $ZSO_2R^1$, where $R^1$ and Z are as defined above; or

(c) reacting a compound of the formula:

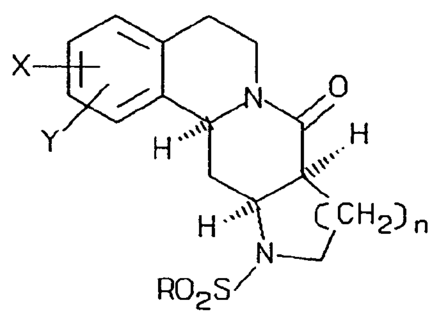

where n, R, X and Y are as defined above, with a suitable reducing agent, preferably sodium borohydride in the presence of boron trifluoride etherate; or

(d) reacting a compound of the formula:

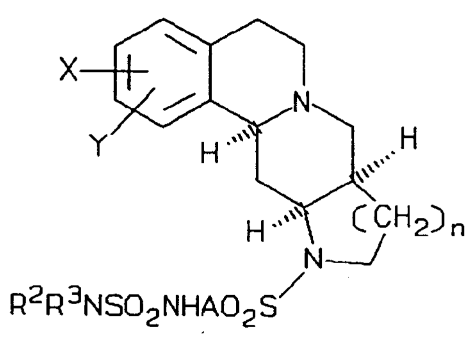

where $R^2$ is tertiary butyl and n, A, $R^3$, X and Y are as defined above, with trifluoroacetic acid; or

(e) reacting the free base of the compound of formula (1) with an acid to give a pharmaceutically acceptable acid addition salt; or

(f) reacting an acid addition salt of the compound of formula (1) with a base to give the corresponding free base; or

(g) converting an acid addition salt of the compound of formula (1) to another pharmaceutically acceptable acid addition salt of formula (1).

**22.** A process for the preparation of a compound represented by the formula:

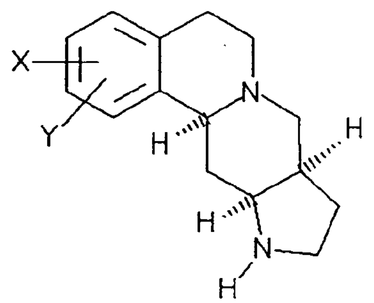

where X and Y are as defined above; said process comprising:

reducing a compound of the formula (X)

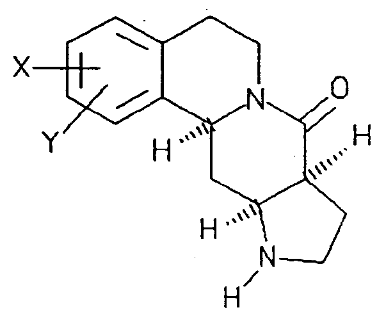

(X)

in which X and Y are as defined above,

with a suitable reducing agent, preferably lithium aluminum hydride.

**Claims for the following Contracting States: ES, GR**

1. A process for the preparation of a compound represented by the formula:

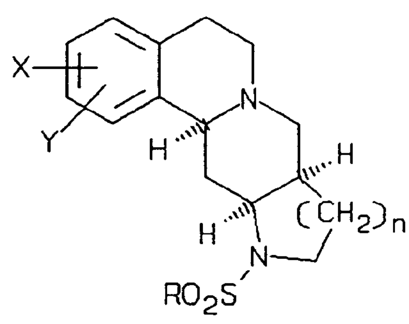

wherein:

X and Y are independently hydrogen; hydroxy; lower alkyl of 1-6 carbon atoms; lower alkoxy of 1-6 carbon atoms; or halo; or X and Y when adjacent and taken together are methylenedioxy or ethylene-1,2-dioxy;

R is lower alkyl of 1-6 carbon atoms; cycloalkyl of 3-8 carbon atoms; phenyl or phenyl lower alkyl wherein any phenyl group may be optionally substituted by one or two substituents chosen from the group consisting of halo, lower alkyl of 1-4 carbon atoms and lower alkoxy of 1-4 carbon atoms; or $-ANHSO_2R^1$; wherein A is lower alkylene of 1-6 carbon atoms; and $R^1$ is lower alkyl of 1-6 carbon atoms or $-NR^2R^3$;

wherein;

$R^2$ and $R^3$ are independently hydrogen or lower alkyl of 1-6 carbon atoms, or $R^2$ and $R^3$ taken

together are cycloalkyl of 3-8 carbon atoms; and

n is 1 or 2;

or a pharmaceutically acceptable salt thereof; with the proviso that when n is 2, R cannot be lower alkyl, cycloalkyl, phenyl or phenyl lower alkyl; said process comprising

(a) sulfonylating a compound of the formula

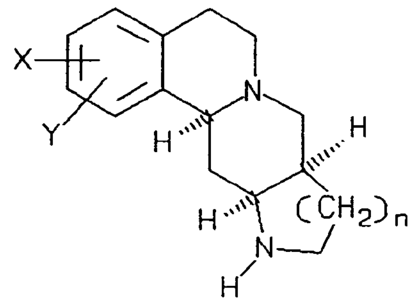

(VII) or (XI)

in which:

X, Y and n are as defined above, with a compound of the formula $ZSO_2R$, where R is lower alkyl, cycloalkyl, or optionally substituted phenyl and Z is chlorine or bromine; or

(b) reacting a compound of the formula:

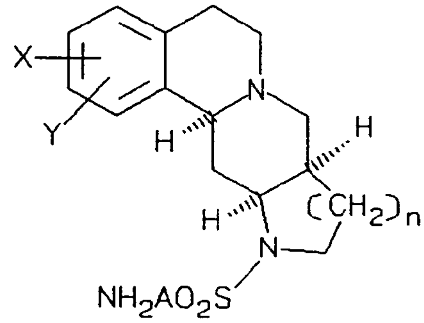

(XIV)

where A, X, Y and n are as defined above, with a compound of the formula $ZSO_2R^1$, where $R^1$ and Z are as defined above; or

(c) reacting a compound of the formula:

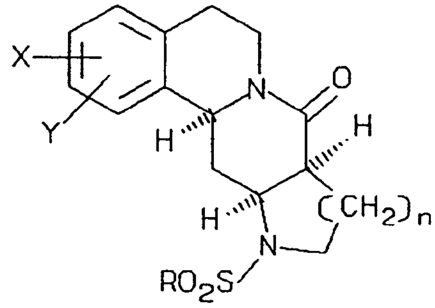

where n, R, X and Y are as defined above, with a suitable reducing agent, preferably sodium borohydride in the presence of boron trifluoride etherate; or

(d) reacting a compound of the formula:

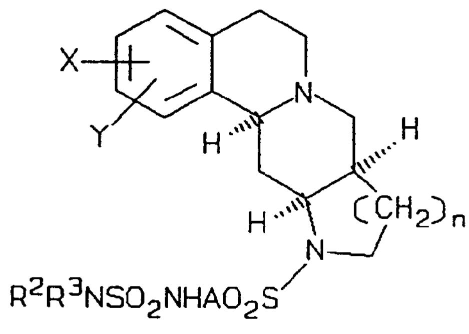

where $R^2$ is tertiary butyl and n, A, $R^3$, X and Y are as defined above, with trifluoroacetic acid; or
(e) reacting the free base of the compound of formula (1) with an acid to give a pharmaceutically acceptable acid addition salt; or
(f) reacting an acid addition salt of the compound of formula (1) with a base to give the corresponding free base; or
(g) converting an acid addition salt of the compound of formula (1) to another pharmaceutically acceptable acid addition salt of formula (1).

2. A method according to claim 1 wherein n is 1 and R is lower alkyl or $-ANHSO_2R^1$.

3. The method of claim 2 wherein X and Y are independently hydrogen or lower alkoxy having 1-4 carbon atoms, or X and Y taken together is methylenedioxy.

4. The method of claim 3 wherein the product is a racemic compound, namely
(±)-(8aα,11aα,12aα)-3-methoxy-11-methanesulfonyl-5,6,8,8a,9,10,11,11a,12,12a-decahydrobenzo[a]pyrrolo[2,3-e]quinolizine; or
(±)-(8aα,11aα,12aα)-2,3-methylenedioxy-11-methanesulfonyl-5,6,8,8a,9,10,11,11a,12,12a-decahydrobenzo[a]pyrrolo[2,3-e]quinolizine; or
(±)-(8aα,11aα,12aα)-3-methoxy-11-(3-(methanesulfonylamino)propanesulfonyl]-5,6,8,8a,9,10,11,11a,12,12a-decahydrobenzo[a]pyrrolo[2,3-e]quinolizine; or
(±)-(8aα,11aα,12aα)-2,3-methylenedioxy-11-(3-(methanesulfonylamino)propanesulfonyl]-5,6,8,8a,9,10,11,11a,12,12a-decahydrobenzo[a]pyrrolo[2,3-e]quinolizine; or a pharmaceutically acceptable salt of the compound.

5. The method of claim 3 wherein A is alkylene of 2-4 carbon atoms and $R^1$ is lower alkyl or $NR^2R^3$.

6. The method of claim 5, wherein the product is a racemic compound, namely
(±)-(8aα,11aα,12aα)-3-methoxy-11-(3-(aminosulfonylamino)propanesulfonyl]-5,6,8,8a,9,10,11,11a,12,12a-decahydrobenzo[a]pyrrolo[2,3-e]quinolizine; or
(±)-(8aα,11aα,12aα)-3-methoxy-11-(3-(dimethylaminosulfonylamino)propanesulfonyl]-5,6,8,8a,-9,10,11,11a,12,12a-decahydrobenzo[a]pyrrolo[2,3-e]-quinolizine; or a pharmaceutically acceptable salt of the compound.

7. The method of claim 1 wherein n is 2.

8. The method of claim 7 wherein R is $-ANHSO_2R^1$.

9. The method of claim 8 wherein A is alkylene of 2-4 carbon atoms and $R^1$ is lower alkyl or $NR^2R^3$.

10. The method of claim 9 wherein X and Y are independently hydrogen or lower alkoxy having 1-4 carbon atoms, or X and Y taken together is methylenedioxy.

11. The method of claim 10 wherein the product is a racemic compound wherein X is 3-methoxy, Y is hydrogen, A is propylene and $R^1$ is methyl, namely (±)-(8aα,12aα,13aα)-3-methoxy-12-[3-(methanesulfonylamino)propanesulfonyl]-5,6,8a,9,10,11,12,12a,13,13a-decahydro-8H-isoquino[2,1-g][1,6]naphthyridine, or a pharmaceutically acceptable salt thereof.

12. The method of claim 11 wherein the product is the (+)-isomer of a compound as obtainable by the method of claim 11, namely (8aR,12aS,13aS)-3-methoxy-12-[3-(methanesulfonylamino)propanesulfonyl]-5,6,8a,9,10,11,12,12a,13,13a-decahydro-8H-isoquino[2,1-g][1,6]naphthyridine, or a pharmaceutically acceptable salt thereof.

13. The method of claim 10 wherein the product is a racemic compound wherein X is 3-methoxy, Y is hydrogen, A is propylene and $R^2$ and $R^3$ are both methyl, namely (±)-(8aα,12aα,13aα)-3-methoxy-12-(3-(dimethylaminosulfonylamino)propanesulfonyl]-5,6,8a,9,10,11,12,12a,13,13a-decahydro-8H-isoquino[2,1-g][1,6]naphthyridine , or a pharmaceutically acceptable salt thereof.

14. The method of claim 13 wherein the product is the (+)isomer of a compound as obtainable by the method of claim 13, namely (8aR,12aS,13aS)-3-methoxy-12-[3-(dimethylaminosulfonylamino)propanesulfonyl)-5,6,8a,9,10,11,12,12a,13,13a-decahydro-8H-isoquino[2,1-g][1,6]naphthyridine, or a pharmaceutically acceptable salt thereof.

15. A process for the preparation of a compound represented by the formula:

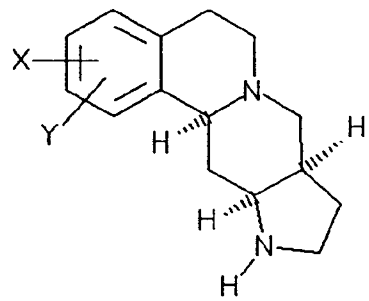

wherein:

X and Y are independently hydrogen, hydroxy, lower alkyl of 1-6 carbon atoms, lower alkoxy of 1-6 carbon atoms or halo, or X and Y when adjacent and taken together are methylenedioxy or ethylene-1,2-dioxy said process comprising:

reducing a compound of the formula (X)

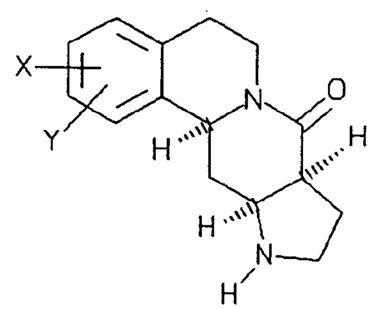

(X)

in which X and Y are as defined above, with a suitable reducing agent, preferably lithium aluminum hydride.

16. The method of claim 15 wherein X and Y are independently hydrogen or lower alkoxy having 1-4 carbon atoms, or X and Y taken together is methylenedioxy; or a pharmaceutically acceptable salt thereof.

17. A method for the production of a pharmaceutical composition comprising mixing a pharmaceutically acceptable non-toxic carrier and a therapeutically effective amount of a compound as producible by the method of any one of claims 1 to 14.

18. Use of a compound as obtainable by the method of any one of claims 1 to 14 in the manufacture of a medicament for treating a mammal having a disease state that is alleviated by treatment with an $\alpha_2$-adreno-

ceptor antagonist.

**19.** Use of a compound represented by the formula:

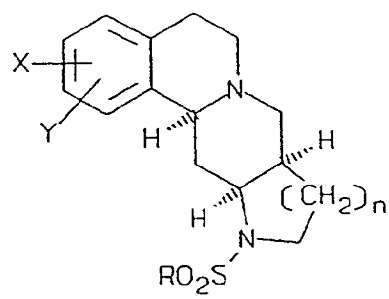

wherein:

X and Y are independently hydrogen; hydroxy; lower alkyl of 1-6 carbon atoms; lower alkoxy of 1-6 carbon atoms; or halo; or X and Y when adjacent and taken together are methylenedioxy or ethylene-1,2-dioxy;

R is lower alkyl of 1-6 carbon atoms; cycloalkyl of 3-8 carbon atoms; phenyl or phenyl lower alkyl wherein any phenyl group may be optionally substituted by one or two substituents chosen from the group consisting of halo, lower alkyl of 1-4 carbon atoms and lower alkoxy of 1-4 carbon atoms; or $-ANHSO_2R^1$; wherein A is lower alkylene of 1-6 carbon atoms; and $R^1$ is lower alkyl of 1-6 carbon atoms or $-NR^2R^3$;

wherein;

$R^2$ and $R^3$ are independently hydrogen or lower alkyl of 1-6 carbon atoms, or $R^2$ and $R^3$ taken together are cycloalkyl of 3-8 carbon atoms; and

n is 1 or 2;

or a pharmaceutically acceptable salt thereof; in the manufacture of a medicament for treating a mammal having peripheral vascular disease, particularly diabetes and its sequelae such as diabetic retinopathy, nephropathy, neuropathy and associated circulatory disturbances.

European Patent Office

# EUROPEAN SEARCH REPORT

Application Number

EP 92 30 6553

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| D,A | EP-A-0 288 196 (SYNTEX)<br>* claims 1,20,23 *<br>--- | 1,15,19 | C07D471/14<br>C07D491/22<br>A61K31/47<br>//(C07D471/14, 221:00,221:00, 209:00)<br>(C07D471/14, 221:00,221:00, 221:00)<br>(C07D491/22, 317:00,221:00, 221:00,209:00) |
| A | JOURNAL OF MEDICINAL CHEMISTRY.<br>vol. 34, no. 2, February 1991, WASHINGTON US<br>pages 705 - 717<br>R. D. CLARK ET AL. 'Structure-affinity relationships of 12-sulfonyl derivatives of 5,8,8a,9,10,11,12,12a,13,13a-decahydro-6H-isoquino(2,1-g)(1,6)naphthyridines at alpha-adrenoceptors'<br>* abstract; page 706, formula 17; page 708, table II *<br>----- | 1,15,19 | |
| | | | TECHNICAL FIELDS SEARCHED (Int. Cl.5 ) |
| | | | C07D<br>A61K |

The present search report has been drawn up for all claims

| Place of search | Date of completion of the search | Examiner |
|---|---|---|
| THE HAGUE | 20 OCTOBER 1992 | VOYIAZOGLOU D. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)